# EUROPEAN PATENT APPLICATION

(11) **EP 1 479 687 A1**
(43) Date of publication of application: **24.11.2004**
(21) Application number: 03707046.3
(22) Date of filing: 25.02.2003
(51) Int. Cl.: C07H 17/08, C07H 15/04, C07H 15/06, A61K 31/7052, A61P 31/04

(54) **NOVEL 15-MEMBERED CYCLIC AZALIDE, NOVEL 16-MEMBERED CYCLIC DIAZALIDE DERIVATIVE, AND PROCESS FOR PRODUCING THESE**

(30) Priority: 26.02.2002 JP 2002049825
(71) Applicant: Meiji Seika Kaisha, Ltd., Tokyo 104-8002 (JP)
(72) Inventor: Miura, T. Pharm. Res. Cent. Meiji Seika Kaisha,Ltd, Yokohama-shi, Kanagawa 222-8567 (JP); Kurihara, K., Ph. Res. Cent. Meiji Seika Kai. Ltd., Yokohama-shi, Kanagawa 222-8567 (JP); Yoshida, T., Phar. Res. Cent. Meiji seika Kai. Ltd, Yokohama-shi, Kanagawa 222-8567 (JP); Ajito, K., Phar. Res. Cent. Meiji Seika Kaisha Ltd, Yokohama-shi, Kanagawa 222-8567 (JP)
(74) Representative: Sternagel, Fleischer, Godemeyer & Partner
(86) International application number: PCT/JP2003/002035
(87) International publication number: WO 2003/072589

(57) **Abstract**

A compound represented by the general formula (I) or a salt thereof which has excellent antibacterial activity (R₁ represents hydrogen atom or an alkylcarbonyl group, R₂ represents hydrogen atom, oxygen atom, hydroxyl group, or an alkylcarbonyloxy group, for example, when R₂ is hydrogen atom, R₃ represents group (a) (each of R₅ and R₆ represents hydrogen atom or an alkyl group), R₄ represents hydrogen atom or group (c) (each of R₈ and R₉ represents hydrogen atom or an alkylcarbonyl group), and Me represents methyl group).

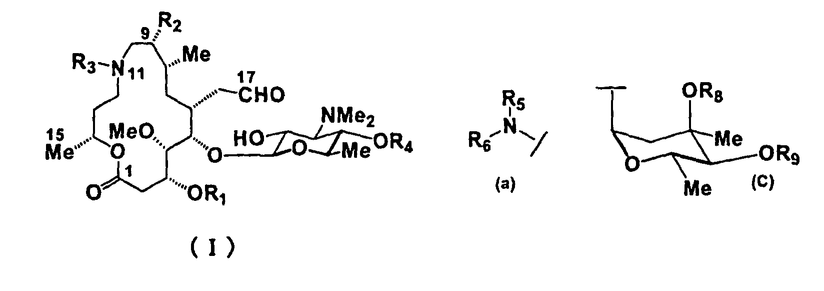

## Description

### Technical Field

The present invention relates mainly to a novel 15-membered azalide and a novel 16-membered diazalide derivative which are effective against Gram-positive and Gram-negative bacteria. The present invention also relates to a synthetic intermediate thereof and a method for preparation thereof.

### Background Art

Macrolide antibiotics generally have low toxicity and are orally available. Therefore, in the field of therapeutic treatments of bacterial infectious diseases, macrolides are one of classes of clinically important antibacterial agents . The macrolide antibiotics are classified roughly into a class of 14-membered macrolides and a class of 16-membered macrolides from a viewpoint of the number of ring-membered atoms of a lactone ring moiety as an aglycone. As a chemically modified antibacterial agent, azithromycin is known which is a commercially available 15-membered macrolide and has a nitrogen atom introduced in the lactone ring (U.S. Patent No.4474768 and U.S. Patent No.4517359). For preparation of azithromycin, the nitrogen atom is introduced by converting the 9-position ketone of erythromycin as a 14-membered macrolide to an oxime, then reducing an imino ether compound which is obtained by Beckmann rearrangement (J. Chem.. Soc. Perkin Trans. I, 1881, 1986, J. Antibiot., 41, 1029, 1988). Macrolide derivatives containing a nitrogen atom as a ring-constituting atom of the lactone ring as mentioned above are presently known as a class of azalides.

Some reports relating to azalide skeletons are known other than the azithromycin. However, as far as position of a nitrogen atom in the lactone ring, only two kinds of structures are known Specifically, only those azalides are known in which a nitrogen atom is located in 9th or 10th position counterclockwise from a carbonyl group of a lactone tentatively regarded in the 1-position.

As azalides derived from 16-membered macrolide, 8a-aza-8a-homotylosin derivatives and 9a-aza-9a-homotylosin derivatives are reported which are synthesized from a 9-keto-oxime derivative of a tylosin by applying a ring expansion reaction through Beckmann rearrangement in the same manner as azithromycin (European Patent No. 410433, WO 00/77016). These derivatives are 17-membered azalides, in which a nitrogen atom located in 9th or 10th position from a carbonyl group of a lactone tentatively regarded in the 1-position, in the same manner as the aforementioned azalides derived from the 14-membered macrolide.

The aforementioned azithromycin exhibits characteristic pharmacodynamics compared with the other macrolide antibiotics, and has a feature to be effective against some Gram-negative bacteria.

Among the 16-membered macrolides, those clinically used so far are a class of leucomycin antibiotics including their derivatives. Studies for a purpose of improving their effectiveness have been made so far by The Kitasato Institute, Toyo Jozo Co., Ltd. (former name) or many entities including our firm, and as a result, rokitamycin (J. Antibiot., 34, 1001, 1981, J. Antibiot., 34, 1011, 1981), and miokamycin (J. Antibiot., 29, 536, 1976, J. Antibiot., 34, 436, 1981) have been launched in the market.

It has been known that pharmacodynamics of the 16-membered macrolide is not fully satisfactory compared with that of the 14-membered macrolide (J. Ferment. Technol., 57, 519, 1979), and it is one of important objects to improve this fault (J. Antimicrobial. Chemotherapy, 28, 787, 1991). Further, the leucomycin 16-membered macrolides available so far are effective mainly against Gram-positive bacteria, and some improvements are achievable in antibacterial activities against Gram-negative bacteria. Accordingly, developments of macrolides have been expected which are effective also against Gram-negative bacteria.

A review of past technologies from a viewpoint of organic synthesis, relating to construction of macrocyclic compounds containing nitrogen atoms, revealed some reports, as for cyclization reaction using a primary amine of dialdehyde under a reducing amination condition, which relate to synthesis of nitrogen-containing aliphatic hetero ring having a small number of constituting elements that form a ring structure, such as 5-membered pyrrolidine derivatives (Tetrahedron, 42, 259, 1986, Synthesis, 789, 2000 and the like), or 6-membered piperidine derivatives (Tetrahedron, 46, 231, 1990, SYNLETT, 361, 1996, Synthesis, 789, 2000 and the like). Almost no cyclization reaction by reductive amination using a primary amine or a dialkylhydrazine of a dialdehyde intermediate compound has been known as for synthesis of nitrogen-containing aliphatic hetero ring having 7-membered ring or more.

Recently, the inventors of the present invention found that a novel 16-membered macrolide derivative, which was obtained by applying various chemical modification on 12- or 13-position of a lactone ring of a leucomycin-class 16-membered macrolide used as a starting material, had improved antibacterial activities against Gram-positive erythromycin-resistant bacteria, and the antibacterial activities were not reduced under physiological conditions. They also found that a certain class of derivatives in which the neutral sugar was removed had potent antibacterial activities against Gram-negative bacteria (WO 02/64607). The inventors of the present invention later found a reaction for a synthesis of 15-membered and 16-membered nitrogen-containing hetero rings using a dialdehyde intermediate compound derived from a leucomycin-class 16-membered macrolide under a reductive amination condition.

Compared with a class of tylosin 16-membered macrolides, leucomycin-class 16-membered macrolides generally have less concern about safety as a medicament. In addition, no report has been made about an azalide prepared by using a leucomycin-class 16-membered macrolide as a starting material.

As for the diazalides, a report was made of a compound derived from erythromycin, in which two nitrogen atoms were introduced into a ring by replacing the oxygen atoms in the lactone moiety as the aglycone of the azalide with nitrogen atoms (WO94/15617). However, no report has been made as for a diazalide having two adjacent nitrogen atoms in a ring which is derivatized from erythromycin or a leucomycin-class macrolide.

Further, no report is made about a method for construction of an azalide fundamental skeleton, using a leucomycin-class 16-membered macrolide as a starting material, by first carrying out a ring-opening of the aglycone and then recyclization utilizing the conjugated double bond between the 10- and 13-positions as a key position of the reaction.

### Disclosure of the Invention

The 16-membered macrolides have high sensitivity to various reactions such as hydrolysis reaction under a strong acidic or basic condition, alkylation reaction using a strong base, and Grignard reaction, and such reactions may sometimes cause degradation of a fundamental skeleton or formation of byproducts. Whilst, as for azithromycin, artisans experienced discoveries of useful substances using erythromycin as a starting material by novel skeletal conversions. Accordingly, a method for unique skeletal conversion using a 16-membered macrolide as a starting material has been desired.

Therefore, the inventors of the present invention was interested in synthesis of novel azalides and novel diazalides having a nitrogen atom in their lactone rings which are derived from leucomycin-class 16-membered macrolides. They are also interested in antibacterial activities of the novel azalides and diazalides and in their effects on pharmacodynamics.

The present invention relates mainly to useful and novel 15-membered azalide derivatives and novel 16-membered diazalide derivatives which are derived from leucomycin-class 16-membered macrolides, pharmaceutically acceptable salts thereof, synthetic intermediates thereof, and methods for preparation thereof.

The inventors of the present invention conducted various studies to achieve the foregoing object. Using leucomycin-class macrolides as starting materials and focusing on conjugated double bonds between the 10- and 13-positions of their lactone ring, they carried out oxidative cleavage for derivatization to novel dialdehyde intermediates and the like unreported so far. In addition, they found a method for constructing azalide and diazalide fundamental skeletons by successive cyclization reaction using amines. Further, by applying these techniques, they created novel 15-membered azalide and novel 16-membered diazalide derivatives introduced with various side chains on the nitrogen atom in the lactone ring.

As a result, the inventors found that a certain class of derivatives of the compounds of the present invention had increased antibacterial activities compared with leucomycin analogues as starting materials, and the antibacterial activities were not reduced under physiological conditions, thereby achieved the present invention. In addition, they succeeded in synthesizing macro-membered, i.e., 15- and 16-membered, nitrogen-containing aliphatic hetero rings, which have been almost unknown so far, from dialdehyde intermediates by using primary amines or dialkylhydrazines.

The compounds of the present invention have one or two nitrogen atoms at 11th position or at 11th and 12th positions counterclockwise from the carbonyl group tentatively regarded as 1-position, which azalide have not been reported so far. Further, diazalides of a class those having nitrogen atoms at the adjacent 11- and 12-positions have not been reported.

The present invention provides, as a novel compound, a compound represented by the following general formula (I) or a pharmaceutically acceptable salt thereof: 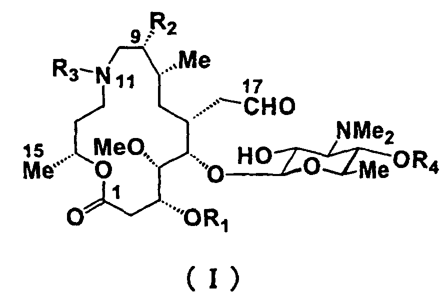 [wherein
R₁ represents hydrogen atom or a C2-C4 linear alkylcarbonyl group,
R₂ represents hydrogen atom, oxygen atom, hydroxyl group, or a C2-C5 alkylcarbonyloxy group,
wherein, when R₂ represents hydrogen atom,
R₃ represents the following group (a): 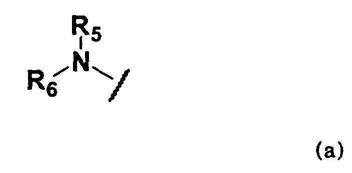 (wherein R₅ and R₆ may be the same or different and each represents hydrogen atom, or a C1-C10 alkyl group which may be substituted with Ar group (Ar represents an aryl group or a heterocyclic group)),
when R₂ represents either hydroxyl group or a C2-C5 alkylcarbonyloxy group,
R₃ represents hydrogen atom, a C1-C10 alkyl group, a C2-C10 alkylcarbonyl group, a C2-C10 alkenyl group, a C3-C10 alkenylcarbonyl group, a C2-C10 alkynyl group, Ar-A-group (wherein Ar has the same meaning as that mentioned above, and A represents a C1-C10 alkyl group, a C2-C10 alkylcarbonyl group, a C2-C10 alkenyl group, a C3-C10 alkenylcarbonyl group, or a C2-C10 alkynyl group), or Ar-(CH₂)ₚ-B-(CH₂)_{q}- group (wherein Ar has the same meaning as that mentioned above, p represents an integer of from 0 to 7, q represents an integer of from 2 to 9, and p+q represents an integer of from 2 to 9, B is a group selected from oxygen atom, sulfur atom or -NR₇-, wherein R₇ represents hydrogen atom, or a C1-C8 alkyl group which may be substituted with Ar group), or
when R₂ represents oxygen atom,
R₃ is W and combines together with R₂ to represent the following group (b): 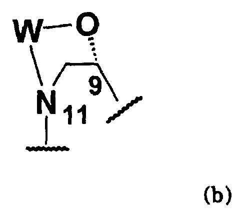 (wherein W represents carbonyl group or thiocarbonyl group), and
R₄ represents hydrogen atom or the following group (c): 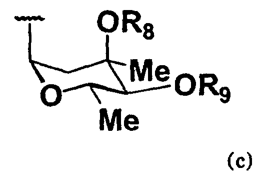 (wherein R₈ and R₉ may be the same or different and each represents hydrogen atom, or a C2-C5 linear or branched alkylcarbonyl group)].

From the second aspect, the present invention provides a compound represented by the following general formula (II) or a pharmaceutically acceptable salt thereof as a novel compound: 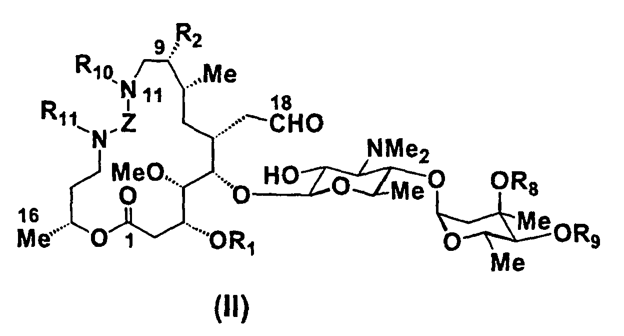 [wherein
Ri represents hydrogen atom or a C2-C4 linear alkylcarbonyl group,
R₂ represents hydroxyl group or a C2-C5 alkylcarbonyloxy group,
R₈ and R₉ may be the same or different and each represents hydrogen atom, or a C2-C5 linear or branched alkylcarbonyl group,
R₁₀ and R₁₁ may be the same or different and each represents hydrogen atom, a C1-C10 alkyl group, a C2-C10 alkenyl group, a C2-C10 alkynyl group, or Ar-A- group (Ar has the same meaning as that mentioned above, and A represents a C1-C10 alkyl group, a C2-C10 alkenyl group or a C2-C10 alkynyl group), or R₁₀ and R₁₁ may optionally form a 6 to 7-membered aliphatic hetero ring together with the nitrogen atom to which they bind and with Z (wherein the aliphatic hetero ring may have a double bond), and Z is a single bond, -(CH₂)ₘ-C₆H₄-(CH₂)ₘ- group (wherein m represents an integer of 0 or 1, and a substituting position on the aromatic ring is not particularly limited) or a -(CH₂)ₙ- group (n represents an integer of from 1 to 3)].

From the third aspect, the present invention provides a compound represented by the following general formula (III) or a pharmaceutically acceptable salt thereof which is a novel compound useful as a synthetic intermediate: 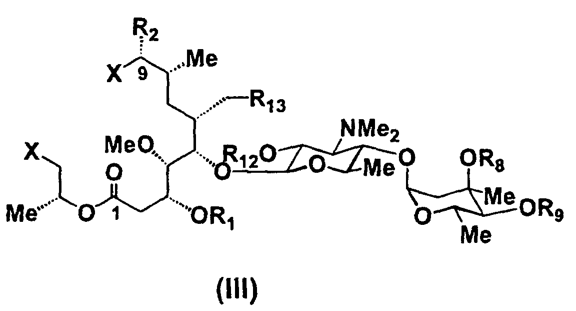 [wherein
R₁ represents hydrogen atom or a C2-C4 linear alkylcarbonyl group,
R₂ represents hydroxyl group, a C2-C5 alkylcarbonyloxy group, or the following group (d): 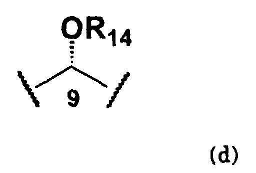 (wherein R₁₄ represents a silyl-type protecting group or an acetal-type protecting group),
R₈ and R₉ may be the same or different and each represents hydrogen atom, or a C2-C5 linear or branched alkylcarbonyl group,
R₁₂ represents hydrogen atom, a C2-C5 linear alkylcarbonyl group, ethoxyethyl group, a silyl-type protecting group, benzyloxycarbonyl group, 4-methoxybenzyloxycarbonyl group, or 4-nitrobenzyloxycarbonyl group,
R₁₃ represents -CH(YR₁₅)₂ group (wherein Y is oxygen atom or sulfur atom, and R₁₅ represents a C1-C5 alkyl group) or the following group (e): 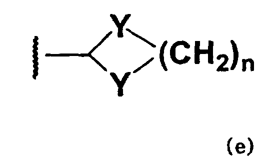 (wherein Y has the same meaning as that mentioned above, and n represents an integer of 2 or 3), and
X represents formyl group, hydroxymethyl group, R₁₆SO₂OCH₂- group (R₁₆ represents methyl group, trifluoromethyl group, or phenyl group, benzyl group, or naphthyl group in which one of the hydrogen atoms on the aromatic ring may be substituted with methyl group, nitro group or azide group), halomethyl group, or R₁₃ (R₁₃ has the same meaning as that mentioned above)].

The present invention further provides a method for preparing a 15 to 21-membered macrolactone, which uses the compound of the formula (III) as a synthetic intermediate and comprises the step of cyclization of said compound.

The present invention also provides a medicament which comprises a substance selected from the group consisting of the compound represented by the aforementioned general formula (I) or (II) and a pharmaceutically acceptable salt thereof as an active ingredient, and according to a preferred embodiment, provided is the aforementioned medicament in the form of a pharmaceutical composition which comprises a pharmaceutically acceptable carrier together with the aforementioned substance which is an active ingredient. These medicaments are useful for therapeutic treatment of bacterial infectious diseases.

Further, the present invention provides a use of a substance selected from the group consisting of the compound represented by the aforementioned general formula (I) or (II) and a pharmaceutically acceptable salt thereof for the manufacture of the aforementioned medicaments, and a method for therapeutic treatment of a bacterial infectious disease, which comprises the step of administering a therapeutically effective amount of a substance selected from the group consisting of the compound represented by the aforementioned general formula (I) or (II) and a pharmaceutically acceptable salt thereof to a mammal including a human.

### Best Mode for Carrying out the Invention

The present invention will be explained in detail.

In the specification, "Me" means a methyl group.

In the specification, the alkyl group and an alkyl moiety of a substituent containing the alkyl moiety (e.g., an alkoxy group, an alkylcarbonyl group and the like) may be, unless otherwise specifically mentioned, any of a linear, branched, cyclic alkyl group or a combination thereof such as methyl, ethyl, propyl, butyl, pentyl, hexyl, isopropyl, isobutyl, tert-butyl, isopentyl, neopentyl, tert-pentyl, cyclopropyl, cyclobutyl, and cyclopentyl, and preferably a linear or branched alkyl.

The alkenyl group, the alkynyl group, and an alkenyl or alkynyl moiety of a substituent containing said moiety may be, unless otherwise specifically mentioned, a linear, branched, cyclic group, or a combination thereof such as vinyl, allyl, 1-propenyl, isopropenyl, 1-butenyl, 2-butenyl, propargyl, 1-butynyl, 1-pentynyl, and 2-butynyl, and preferably a linear or branched group. The number of double bonds or triple bonds contained in the alkenyl or alkynyl moiety is not particularly limited, and a double bond contained in the alkenyl moiety may be in either of Z- or E-configuration.

In the specification, the aryl group means, unless otherwise specifically mentioned, a 6- to 14-membered (monocyclic to tricyclic, preferably monocyclic to dicyclic) aromatic ring which contains no heteroatom, such as phenyl group, 1-naphthyl group, 2-naphthyl group, 1-anthryl group, and 2-anthryl group. The 6- to 14-membered aryl group herein referred to has 6 to 14 carbon atoms in its ring system. The aforementioned 6- to 14-membered aryl group may optionally be substituted with 1 to 5 substituents selected from a halogen atom, hydroxyl group, nitro group, cyano group, trifluoromethyl group, a C1-C6 alkyl group, a C2-C7 acyl group, a C1-C6 alkyloxycarbonyl group, a C1-C6 acyloxy group, dimethylamino group, diethylamino group, a C1-C6 alkoxy group, a 6- to 10-membered aryl group (examples include phenyl group, 1-naphthyl group, 2-naphthyl group and the like, and a binding position is not particularly limited), a 5- to 10-membered heterocyclic group (examples include 2-imidazolyl group, 4-imidazolyl group, 3-pyridyl group, 4-pyridyl group, 3-quinolinyl group, 4-quinolinyl group, 4-isoquinolinyl group, benzimidazolyl group, pyrrolidinyl group, piperidinyl group, 3-morpholinyl group and the like, and a binding position is not particularly limited).

In the specification, the heterocyclic group means, unless otherwise specifically mentioned, an aromatic or aliphatic 5- to 14-membered (monocyclic to tricyclic, preferably monocyclic to dicyclic) hetero ring which contains 1 to 3 heteroatoms selected from oxygen atom, sulfur atom, and nitrogen atom such as 2-imidazolyl group, 4-imidazolyl group, 3-pyridyl group, 4-pyridyl group, 3-quinolinyl group, 4-quinolinyl group, 4-isoquinolinyl group, 2-benzimidazolyl group, pyrrolidinyl group, 3-piperidinyl group, and 3-morpholinyl group (a binding position is not particularly limited). The 5- to 14-membered heterocyclic group herein referred to has 5 to 14 atoms in its ring system. The aforementioned aromatic 5 to 14-membered heterocyclic group may optionally be substituted with 1 to 6 substituents independently selected from a halogen atom, hydroxyl group, nitro group, cyano group, trifluoromethyl group, a C1-C6 alkyl group, a C2-C7 alkylcarbonyl group, a C1-C6 alkyloxycarbonyl group, a C1-C6 acyloxy group, dimethylamino group, diethylamino group, a C1-C6 alkoxy group, a 6 to 10-membered aryl group (examples include phenyl group, 1-naphthyl group, 2-naphthyl group and the like, and a binding position is not particularly limited), a 5- to 10-membered heterocyclic group (examples include 2-imidazolyl group, 4-imidazolyl group, 3-pyridyl group, 4-pyridyl group, 3-quinolinyl group, 4-quinolinyl group, 4-isoquinolinyl group, benzimidazolyl group, pyrrolidinyl group, piperidinyl group, 3-morpholinyl group and the like, and a binding position is not particularly limited).

In the specification, the acyl group and an acyl moiety of a substituent which contains the acyl moiety (e.g., an acyloxy group such as acetoxy group) means, unless otherwise specifically mentioned, a C2-C5 linear or branched alkylcarbonyl group such as formyl group, acetyl group, propionyl group, butyryl group, isobutyryl group, valeryl group, and isovaleryl group.

In the specification, the halogen atom means a fluorine, chlorine, bromine or iodine atom.

In the specification, the silyl-type protecting group means trimethylsilyl group, triethylsilyl group, triisopropylsilyl group, triphenylsilyl group, tribenzylsilyl group, dimethylisopropylsilyl group, tert-butyldimethylsilyl group, tert-butyldiphenylsilyl group and the like.

In the specification, the acetal-type protecting group means, unless otherwise specifically mentioned, a non-cyclic acetal such as dimethyl acetal, diethyl acetal, and diisopropyl acetal, or a cyclic acetal such as ethylene acetal, and propylene acetal. Provided that the acetal-protecting group represented by R₁₄ in the group (d) in the formula (III) means methoxymethyl group, ethoxyethyl group, tetrahydropyranyl group, tetrahydrofuranyl and the like.

In the specification, the halomethyl group means a group in which one of the hydrogen atoms of methyl group is substituted with a halogen atom.

The method for preparation of a 15- to 21-membered lactone comprises, which uses the compound represented by the general formula (III) as a synthetic intermediate and comprises the step of cyclization of said compound, the step of constructing a macrolactone ring from the compound represented by the general formula (III) such as cyclization by reductive aminoalkylation reaction or substitution reaction using a primary alkylamine, a diamine substituted with an alkyl group, a hydrazine substituted with an alkyl group or the like, cyclization by bis-Wittig reaction using trimethylene bis(triphenylphosphonium bromide), tetramethylene bis(triphenylphosphonium bromide) or the like, and cyclization by aldol reaction using an aldehyde having α-hydrogen.

In the formulas (I), (II) and (III), each of hydrogen atom and a C2-C4 linear alkylcarbonyl group represented by R₁ is preferred.

In the formula (I), each of hydrogen atom, oxygen atom, hydroxyl group, and a C2-C5 alkylcarbonyloxy group represented by R₂ is preferred, and hydroxyl group or a C2-C5 alkylcarbonyloxy group is more preferred.

In the formula (II), each of hydroxyl group or a C2-C5 alkylcarbonyloxy group represented by R₂ is preferred.

In the formula (III), each of hydroxyl group, a C2-C5 alkylcarbonyloxy group, and the group (d) represented by R₂ is preferred, a C2-C5 alkylcarbonyloxy group is more preferred.

In the formula (I), when R₂ is a hydrogen atom, the group (a) represented by R₃ is preferably the group (a) wherein R₅ and R₆ are the same or different and each represents a C1-C10 alkyl group which may be substituted with an Ar group (Ar represents an aryl group or a heterocyclic group), more preferably the group (a) wherein R₅ and R₆ are the same or different and each represents a C1-C10 alkyl group which may be substituted with phenyl.

In the formula (I), when R₂ is hydroxyl group or a C2-C5 alkylcarbonyloxy group, R₃ is preferably hydrogen atom, a C1-C10 alkyl group, a C2-C10 alkylcarbonyl group, or Ar-A- group (wherein Ar has the same meaning as that defined above, and A represents a C1-C10 alkyl group, a C2-C10 alkylcarbonyl group, a C2-C10 alkenyl group, or a C2-C10 alkynyl group), more preferably a C1-C10 alkyl group which may be substituted with an Ar group, a C2-C10 alkylcarbonyl group which may be substituted with an Ar group, a C2-C10 alkenyl group which may be substituted with an Ar group, or a C2-C10 alkynyl group which may be substituted with anAr group. Specific examples include methyl group, benzyl group, 2-phenylethyl group, 3-phenylpropyl group, 4-phenylbutyl group, 5-phenylpentyl group, 3-phenylpropionyl group, 3-phenyl-2-propenyl group, 3-phenyl-2-propynyl group, 3-(2-benzyloxyphenyl)propyl group, 3-(pyridin-4-yl)propyl group, N-methyl-N-(3-phenylpropyl)amino group, 3-(quinolin-4-yl)propyl group or 3-(6-methoxyquinolin-4-yl)propyl group.

In the formula (I), when R₂ represents oxygen atom, the group W represented by R₃ is preferably the following group (b-1).

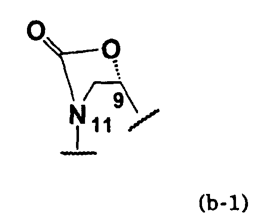

In the formula (I), the group (c) represented by R₄ is preferably that wherein R₈ and R₉ are the same or different and each represents hydrogen atom or a C2-C5 linear or branched alkylcarbonyl group.

In the formulas (II) and (III), R₈ and R₉ may be the same or different and each preferably represents hydrogen atom or a C2-C5 linear or branched alkylcarbonyl group.

In the formula (II), R₁₀ and R₁₁ may be the same or different and each preferably represents hydrogen atom, a C1-C10 alkyl group, or Ar-A- group (Ar has the same meaning as that defined above, and A represents a C1-C10 alkyl group, a C2-C10 alkenyl group, or a C2-C10 alkynyl group), more preferably, R₁₀ and R₁₁ may be the same or different and each represents a C1-C10 alkyl group which may be substituted with a phenyl group, and most preferably each represents methyl group.

In the formula (II), Z is preferably a single bond.

In the formula (III), R₁₂ is preferably a C2-C5 linear alkylcarbonyl group.

In the formula (III), R₁₃ is preferably -CH(YR₁₅)₂ group wherein Y is oxygen atom (wherein R₁₅ represents a C1-C5 alkyl group).

In the formula (III), X is preferably formyl group.

Among the compounds of the formula (I) according to the present invention, a preferred class of compounds include the compounds wherein
R₁ is hydrogen atom or a C2-C4 linear alkylcarbonyl group, R₂ is hydrogen atom, oxygen atom, hydroxyl group, or a C2-C5 alkylcarbonyloxy group,
R₂ is hydrogen atom, and
R₃ is the following group (a-1): 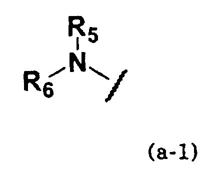 (wherein R₅ is a C1-C10 alkyl group, R₆ represents a C1-C10 alkyl group which may be substituted with an Ar group (Ar has the same meaning as that defined above)), or R₂ is either hydroxyl group or a C2-C5 alkylcarbonyloxy group, and R₃ is hydrogen atom, a C1-C10 alkyl group, a C2-C10 alkylcarbonyl group, or Ar-A- group (wherein Ar has the same meaning as that defined above, and A represents a C1-C10 alkyl group, a C2-C10 alkylcarbonyl group, a C2-C10 alkenyl group, or a C2-C10 alkynyl group), or R₂ is oxygen atom, and
R₃ combines with R₂ to represent the following group (b-1): 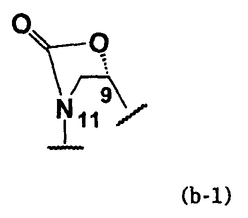 and R₄ is hydrogen atom or the following group (c-1): 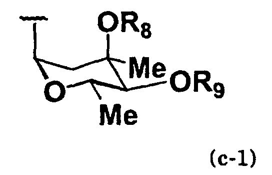 (wherein R₈ is hydrogen atom or a C2-C5 linear or branched alkylcarbonyl group, and R₉ represents a C2-C5 linear or branched alkylcarbonyl group).

Among the compounds of the formula (I) according to the present invention, a more preferred class of compounds include the compounds wherein
R₁ is a C2-C4 linear alkylcarbonyl group,
R₂ is hydroxyl group or a C2-C5 alkylcarbonyloxy group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and R₄ is the group (c) wherein R₈ and R₉ may be the same or different and each represents a C2-C5 linear or branched alkylcarbonyl group.

Among the compounds of the formula (I) according to the present invention, another class of more preferred compounds include the compounds wherein
R₁ is a C2-C4 linear alkylcarbonyl group,
R₂ is hydroxyl group or a C2-C5 alkylcarbonyloxy group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and R₄ is the group (c) wherein R₈ is hydrogen atom, and R₉ is a C2-C5 linear or branched alkylcarbonyl group.

Among the compounds of the formula (I) according to the present invention, a further class of more preferred compounds include the compounds wherein
Ri is hydrogen atom,
R₂ is hydroxyl group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and R₄ is the group (c) wherein R₈ and R₉ may be the same or different and each represents a C2-C5 linear or branched alkylcarbonyl group.

Among the compounds of the formula (I) according to the present invention, a still further class of more preferred compounds include the compounds wherein
R₁ is a C2-C4 linear alkylcarbonyl group,
R₂ is hydroxyl group or a C2-C5 alkylcarbonyloxy group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and
R₄ is hydrogen atom.

Among the compounds of the formula (II) according to the present invention, a class of preferred compounds include the compounds wherein
R₁₀ and R₁₁ may be the same or different and each represents hydrogen atom, a C1-C10 alkyl group, or Ar-A- group (Ar has the same meaning as that defined above, and A represents a C1-C10 alkyl group, a C2-C10 alkenyl group, or a C2-C10 alkynyl group), and
Z is a single bond.

Among the compounds of the formula (II) according to the present invention, a class of more preferred compounds include the compounds wherein
both R₁₀ and R₁₁ are methyl groups.

Among the compounds of the formula (II) according to the present invention, a further class of preferred compounds include the compounds wherein R₁₀ is methyl group and R₁₁ is 4-phenylbutyl group, or the compounds wherein R₁₀ is 4-phenylbutyl group and R₁₁ is methyl group.

Among the compounds of the formula (III) according to the present invention, preferred class of compounds include the compounds wherein X is formyl group.

As for the preparations of the 15- to 21-membered macrolactone according to the present invention, a preferred embodiment includes, among the methods characterized by using the compound of the formula (III) as a synthetic intermediate and comprising the step of cyclization of said compound, a method for preparing the aforementioned compound of the formula (I) or (II).

The compounds represented by the general formulas (I) to (III) according to the present invention can be prepared through the 5 steps shown in the following scheme 1. Alternatively, the compounds can also be prepared through the 3 steps shown in the following scheme 2. Unless otherwise specifically mentioned, R₁ to R₃, R₈ to R₁₂, and R₁₅ in the following schemes have the same meanings as those defined above. As for these methods for preparation, each of the steps, divided as the steps 1 to 5 in scheme 1, will be separately explained in detail.

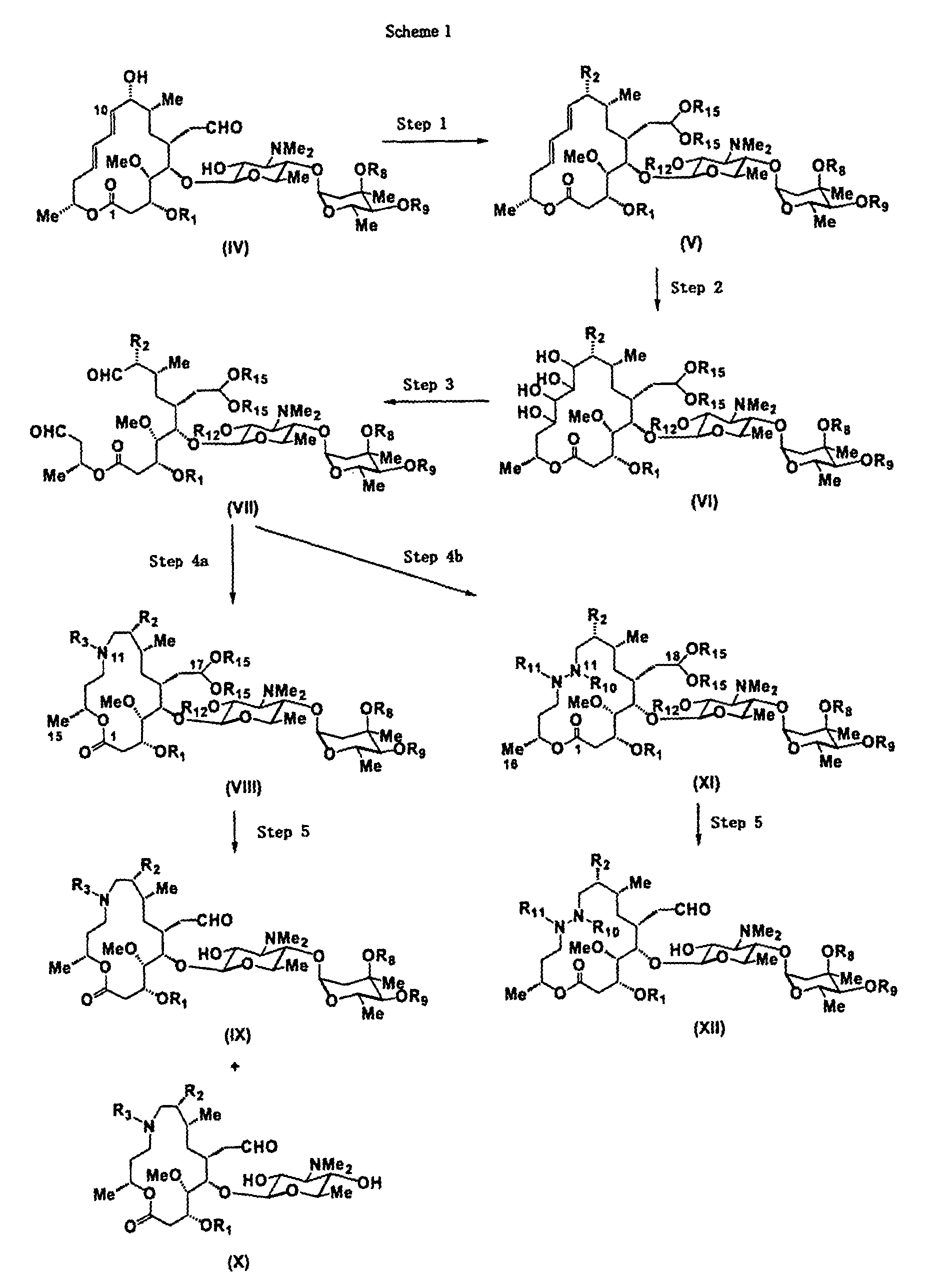

The method for preparation of the compound represented by the formula (V) by using the compound represented by the formula (IV) as the starting material, which is depicted as Step 1, will be explained.

The modification of the 9-position hydroxyl group of the starting material represented by the formula (IV) with an acyl-side chain can be selectively proceeded by the reaction with an acid halide in a solvent of methylene chloride in the presence of pyridine. The solvent in the reaction may be an aprotic solvent such as chloroform, benzene, toluene, and xylene, as well as methylene chloride, and the base is preferably an organic base such as pyridine, and may be used in an amount of 1 to 10 equivalents. As the acylating reagent, an acid halide may be used in the amount of 1 to 5 equivalents. The reaction proceeds in the range of 0°C to 50°C, and the reaction time is 0.5 hour to 24 hours.

Then, modification of the 9-position hydroxyl group of the starting material represented by the formula (IV) with a silyl-protecting group may be performed, for example, by reacting with a required amount of tert-butyldimethylsilyl chloride (TBDMSCl) in a solvent of dry dimethylformamide (DMF) in the presence of imidazole. As the base in the above silylation reaction, an organic base such as pyridine, lutidine, and triethylamine can be used, as well as imidazole, and preferably imidazole may be used in an amount of 2 to 6 equivalents. As the silylating reagent, a reagent used for common TBDMS formation such as TBDMSOClO₃, TBDMSOSO₂CF₃, and TBDMSCN, as well as TBDMSCl, may be used in an amount of 1 to 3 equivalents. As the reaction solvent, acetonitrile, methylene chloride, tetrahydrofuran (THF) and the like can be used as well as DMF. The reaction proceeds in the range of 0°C to 50°C in a good yield, and the reaction time is 1 hour to 24 hours.

When the 9-position hydroxyl group of the starting material represented by the formula (IV) is introduced with a silyl-protecting group other than TBDMS group, the same procedure may be carried out by using, for example, trimethylsilyl chloride, triethylsilyl chloride, triisopropylsilyl chloride, triphenylsilyl chloride, tribenzylsilyl chloride, dimethylisopropylsilyl chloride, tert-butyldiphenylsilyl chloride or the like instead of TBDMSCl.

The successive modification of the 18-position formyl group with a acetal-protecting group can be proceeded in a good yield by carrying out the reaction, for example, in a mixed solvent of methyl orthoformate and methanol in the presence of an organic acid. As the acid, an organic acid such as p-toluenesulfonic acid, and camphorsulfonic acid can be used, and preferably, pyridinium p-toluenesulfonate (PPTS) may be used in an amount of 1 to 3 equivalents. As the solvent, a mixed solution of methyl orthoformate also as a reagent and methanol may be used in a 10-time (V/W) to 60-time (V/W) amount. The reaction proceeds in a good yield in a range of 20°C to 80°C, and the reaction time is 1 hour to 6 days.

In the compound represented by the general formula (V), when an acetal-protecting group, other than dimethylacetal in which R₁₅ represents methyl group, is introduced, the same procedure may be carried out by using, for example, a mixed solvent of ethyl orthoformate and ethanol, isopropyl orthoformate and isopropanol, ethylene glycol and benzene, or propylene glycol and benzene instead of the mixed solvent of methyl orthoformate and methanol.

Then, modification of the 2'-position hydroxyl group in the mycaminose moiety with an acetyl group can be quantitatively proceeded by a reaction using acetic anhydride in an acetonitrile as a solvent. The solvent in the reaction may also be an aprotic solvent such as methylene chloride and chloroform, and as the acetylating agent, acetic anhydride may preferably be used in an amount of 1 to 5 equivalents. The reaction proceeds in a good yield in the range of 20°C to 60°C, and the reaction time is 1 hour to 48 hours.

Subsequently, the step 2 for preparing the compounds represented by the formula (VI) will be explained . The step may be performed by subjecting the compound represented by the formula (V) to a reaction, for example, by using N-methylmorpholine-N-oxide as a co-oxidizing agent in the presence of a catalytic amount of a metal oxidizing agent, in a mixed solvent of acetone and water. The metal oxidizing agent to be used may be potassium osmate(VI) dihydrate, and preferably, 0.05 to 1 equivalent of osmium tetraoxide may be used. As the co-oxidizing agent, a chlorate such as sodium chlorate, silver chlorate, and barium chlorate, hydrogen peroxide, tert-butyl hydroperoxide or the like can also be used in the presence or absence of trimethylamine-N-oxide, potassium hexacyanoferrate(III), or a quarternary ammonium salt such as tetraethylammonium acetate, and preferably 1 to 5 equivalents of N-methylmorpholine-N-oxide may be used. As the solvent, acetonitrile, THF, methylene chloride, tert-butyl alcohol, diethyl ether, a mixed solvent of tert-butyl alcohol and water, a mixed solvent of THF and water or the like may be used as well as a mixed solvent of acetone and water. The reaction proceeds in the range of 0°C to 50°C, and the reaction time is 5 hours to 3 days.

Step 3 for preparing the compound represented by the formula (VII) will be explained. The step may be performed by subjecting the compound represented by the formula (VI) to a reaction, for example, by using lead tetraacetate as an oxidizing agent in benzene in the presence of an inorganic base.

As the oxidizing agent to be used in the reaction, a peracid such as sodium metaperiodate, or active manganese dioxide, pyridinium chlorochromate or the like may be used, and preferably lead tetraacetate may be used in an amount of 1 to 5 equivalents. As the solvent to be used in the reaction with lead tetraacetate, an aprotic solvent such as benzene, toluene, xylene and methylene chloride is preferred. As the solvent to be used in the reaction with a peracid such as metaperiodate, water, or a mixed solvent of ether and water, methylene chloride and water or the like may be used. As the base in the reaction, an inorganic base such as sodium hydrogencarbonate, sodium carbonate, potassium hydrogencarbonate, and potassium carbonate may be used in an amount of 1 to 15 equivalents. The reaction proceeds in the range of 0°C to 50°C, and the reaction time is 5 minutes to 24 hours. The compound represented by the formula (VII) prepared by the above reaction can be used in the following step 4 without purification.

A method for preparing the compound represented by the general formula (III) wherein X is the group other than a formyl group will be explained.

The compound wherein X represents hydroxymethyl group is prepared by reduction of the compound represented by the formula (VII), for example, by using sodium borohydride in an ethanol solvent.

Examples of the reducing agent to be used in the above reaction include lithium borohydride, lithium aluminum hydride, sodium triacetoxyborohydride, sodium cyanoborohydride, zinc borohydride or the like. Preferably, sodium borohydride may be used in an amount of 0.5 to 2 equivalents. As the solvent to be used in the reaction, examples include a lower alcohol such as methanol and isopropanol, acetonitrile, THF, methylene chloride, 1,2-dichloroethane or the like, as well as ethanol.

In order to prepare the compound represented by the general formula (III) wherein X is R₁₆SO₂OCH₂- group, the compound wherein X represents a hydroxymethyl group may be allowed to react, for example, with an acid halide in a methylene chloride as a solvent in the presence of triethylamine. The solvent in the reaction may be an aprotic solvent such as chloroform, benzene, toluene, and xylene, as well as methylene chloride, and as the base, an organic base such as pyridine and diisopropylethylamine may be used, as well as triethylamine, in an amount of 1 to 10 equivalents. The acid halide may be used in an amount of 2 to 5 equivalents. The reaction proceeds in the range of 0°C to 50°C, and the reaction time is 0.5 hour to 24 hours.

In order to prepare the compound represented by the general formula (III) wherein X is halomethyl group, the compound wherein X represents hydroxymethyl group may be subjected to a reaction, for example, by adding 2 to 10 equivalents of carbon tetrabromide in a methylene chloride as a solvent in the presence of 2 to 5 equivalents of triphenylphosphine. The reaction proceeds in the range of 0°C to 50°C, and the reaction time is 0.5 hour to 24 hours. Instead of carbon tetrabromide, 2 to 5 equivalents of N-bromosuccinimide or N-chlorosuccinimide can also be used. Alternatively, instead of carbon tetrabromide, carbon tetrachloride can also be used, and in this reaction, carbon tetrachloride is used as a solvent which also serves as a reagent. The reaction using carbon tetrachloride proceeds in the range of 10°C to 80°C, and the reaction time is 0.5 hour to 24 hours.

In order to form an acetal of the formyl group of the compound represented by the general formula (VII), the compound may be allowed to react in the same manner as in the aforementioned modification of the 18-position formyl group in the compound represented by the formula (IV) with an acetal-protecting group.

Steps 4a and 4b for preparation of the compounds represented by the formulas (VIII) and (XI) will be explained. The compounds are prepared, for example, by carrying out cyclization reaction of the compound represented by the formula (VII) by reductive aminoalkylation using sodium cyanoborohydride as a reducing agent with addition of a primary amine (Step 4a) such as R₃NH₂ or a hydrazine (Step 4b) such as R₁₀NHNHR₁₁ in the presence of acetic acid in ethanol. Instead of the aforementioned primary amine and hydrazine, a 6- to 7-membered aliphatic diamine such as piperazine, homopiperazine, or hexahydropyridazine, or a diamine such as R₁₀NH-(CH₂)ₘ-C₆H₄-(CH₂)ₘ-NHR₁₁ or R₁₀NH-(CH₂)n-NHR₁₁ can also be used.

The reducing agent in the reaction may be sodium triacetoxyborohydride, sodium borohydride, sodium cyanoborohydride-titanium tetraisopropoxide, sodium cyanoborohydride-magnesium perchlorate, a borane/pyridine complex, zinc borohydride/zinc chloride, dibutylchlorostannane-hexamethyl phosphoramide, potassium tetracarbonylhydride ferrate or the like, and preferably, sodium cyanoborohydride may be used in an amount of 1 to 10 equivalents. The amine or diamine such as R₃NH₂ or R₁₀NHNHR₁₁ may form a salt with an inorganic acid such as hydrochloric acid or an organic acid such as acetic acid, and may be used in an amount of 0.8 to 2.0 equivalents. Then, as the acid to be added, hydrochloric acid or the like may be used, and preferably, acetic acid may be used in an amount of 1 to 30 equivalents. As the solvent, a lower alcohol such as methanol and isopropanol, acetonitrile, THF, methylene chloride, 1,2-dichloroethane or the like, as well as ethanol, may be used, and the solvent may be used in a 30-time (V/W) to 300-time (V/W) amount based on the compound represented by the formula (VII). The reaction proceeds in the range of 0°C to 50°C, and the reaction time is 1 hour to 48 hours.

Finally, Step 5 for preparation of the novel compounds represented by the formulas (IX), (X) and (XII) will be explained. Deprotection of the 2'-position acetyl group in the mycaminose moiety of the compound represented by the formula (VIII) or (XI) can be proceeded in methanol or a mixed solvent of methanol and water. The reaction proceeds in the range of 20°C to 80°C, and the reaction time is 12 hours to 5 days. In this reaction, depending on a type of the substituent R³ of the compound represented by the formula (VIII) or reaction conditions, deacetylation of the 9-position hydroxyl group can be made simultaneously with deacetylation of the 2'-position.

Then, the 17-position acetal-protecting group in the formula (VIII) or the 18-position acetal-protecting group in the formula (XI) is eliminated by the reaction with difluoroacetic acid in a mixed solvent of acetonitrile and water to obtain the compound represented by the formula (IX) or (XII). When R₈ is hydrogen atom in the formula (VIII), the compound can be converted to the compound represented by the formula (IX) and/or the formula (X). Further, by prolongation of the reaction time or elevation of the concentration of the acid to be used, the compound can be converted solely to the compound represented by the formula (X). The mixed solution of the equal amount of acetonitrile and water may be used in a 10-time (V/W) to 300-time (V/W) amount based on the compound represented by the formula (VIII) or (XI). As the acid, monofluoroacetic acid, trifluoroacetic acid, acetic acid, hydrochloric acid or the like can be used, and preferably, difluoroacetic acid may be used in an amount of 1 to 100 equivalents. The reaction proceeds in good yields in the range of 0°C to 50°C, and the reaction time is 0.5 hour to 4 days.

In order to prepare novel cyclic carbamate compounds represented by the formula (IX) wherein R₂ and R₃ combine together to represent the following group: 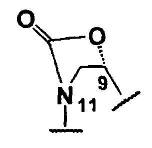 the compound represented by the formula (VIII) wherein R₂ is acetyloxy group, R₃ is benzyl group or 4-methoxybenzyl group, and R₁₂ represents acetyl group is converted into the compound wherein both of the 2'-position and the 9-position are hydroxyl groups by carrying out the aforementioned deacetylation reaction in the same manner. Then, the 11-position benzyl group or 4-methoxybenzyl group is eliminated by catalytic hydrogen reduction to obtain the compound wherein R₂ is hydroxyl group, and each of R₃ and R₁₂ represents hydrogen atom. The catalyst to be used in the reaction may be palladium-black, palladium hydroxide or the like, and preferably, palladium-carbon may be used in an amount of 5 to 100% (W/W) based on the starting material. As the solvent, a lower alcohol such as methanol, ethanol and isopropanol, dioxane, water, acetonitrile, THF, ethyl acetate or the like may be used as a sole solvent, or these solvents may be used in combination as a mixed solvent. The reaction proceeds in the range of 0°C to 50°C, and the reaction time is 1 hour to 48 hours.

In order to construct a cyclic carbamate ring between the 9-position and the 11-position, for example, the compound may be allowed to react with triphosgene in methylene chloride in the presence of triethylamine. As the base in the reaction, an organic base such as trimethylamine and diisopropylethylamine can be used, and preferably, triethylamine may be used in an amount of 2 to 6 equivalents. The reaction proceeds in the range of 0°C to 50°C, and the reaction time is 10 minutes to 24 hours.

When the 9-position hydroxyl group of the compound represented by the formula (VIII) is modified with a silyl-protecting group, the compound represented by the formula (IX) is prepared by successively carrying out the deacetylation at the 2'-position and the deacetalization at the 17-position in the same manner as those mentioned above, and then eliminating the silyl-protecting group by using tetrabutylammonium fluoride in a mixed solution of equal volume of acetic acid and THF. The reaction proceeds in the range of 20°C to 100°C, and the reaction time is 12 hours to 4 days.

As for another method for preparation of the novel compounds represented by the formula (IX) and/or the formula (X), each of the steps, divided as the steps 1 to 3 in scheme 2, will be separately explained in detail.

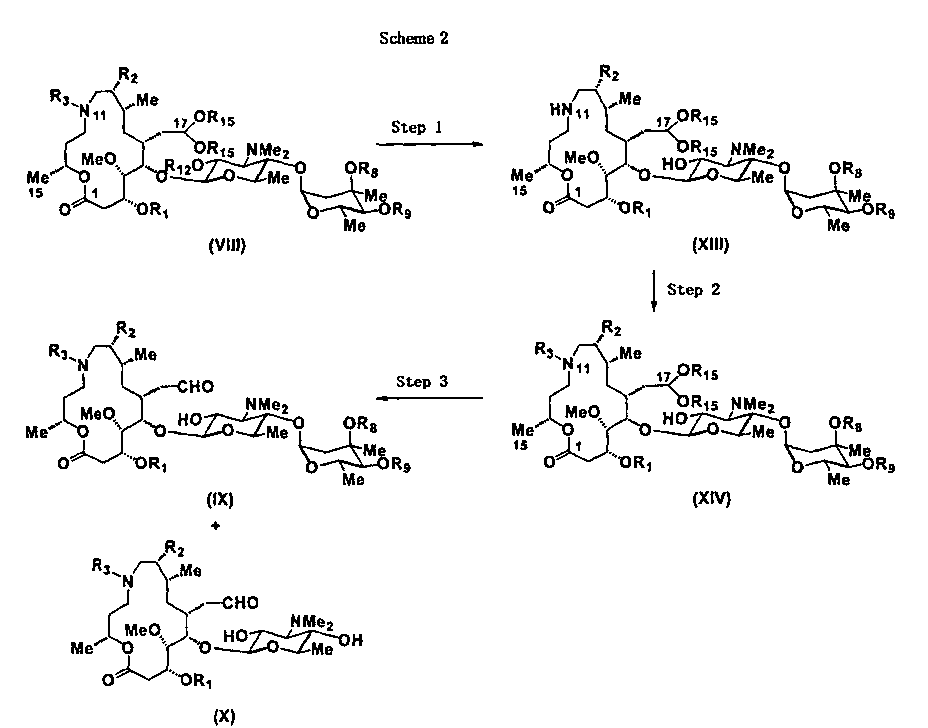

Step 1 for preparing the compound represented by the formula (XIII) will be explained which uses, as the starting material, the compound of the formula (VIII) wherein R₂ represents acetyloxy group, R₃ represents benzyl group or 4-methoxybenzyl group, and R₁₂ represents acetyl group obtained in Step 4a of Scheme 1. In the same manner as those mentioned above, the compound is converted into the compound represented by the formula (XIII) by successively carrying out the deacetylation at the 2'-position, and elimination of the 11-position benzyl group or 4-methoxybenzyl group by catalytic hydrogen reduction. In the deacetylation reaction, depending on a type of the substituent R₃ of the compound represented by the formula (VIII) or reaction conditions, deacetylation of the 9-position hydroxyl group can be made simultaneously with the deacetylation of the 2'-position.

Step 2 for preparing the compound represented by the formula (XIV) will be explained. The compound is prepared by, for example, adding a variety of an aldehyde to the compound represented by the formula (XIII) in methanol in the presence of acetic acid, and carrying out reductive aminoalkylation reaction using sodium cyanoborohydride as a reducing agent.

As the reducing agent to be used in the reaction, sodium triacetoxyborohydride, sodium borohydride, sodium cyanoborohydride-titanium tetraisopropoxide, sodium cyanoborohydride-magnesium perchlorate, a borane/pyridine complex, zinc borohydride/zinc chloride dibutylchlorostannane-hexamethyl phosphoramide, potassium tetracarbonylhydride ferrate or the like may be used, and preferably, sodium cyanoborohydride may be used in an amount of 1 to 10 equivalents. The aldehyde may be used in an amount of 0.8 to 5 equivalents, and the acid to be added may be hydrochloric acid or the like, and preferably acetic acid may be used in an amount of 1 to 30 equivalents. As the solvent, a lower alcohol such as ethanol and isopropanol, acetonitrile, THF, methylene chloride, 1,2-dichloroethane or the like, as well as methanol, may be used, and the solvent may be used in a 1-time (V/W) to 100-time (V/W) amount. The reaction proceeds in the range of 0°C to 50°C, and the reaction time is 0.5 hour to 48 hours.

In order to introduce an acyl group to the 11-position, for example, the compound of the formula (XIII) may be allowed to react with an acid halide in a solvent of methylene chloride in the presence of triethylamine.

As the solvent to be used in the reaction, an aprotic solvent such as chloroform, benzene, toluene and xylene may be used, and preferably, methylene chloride may be used in a 1-time (V/W) to 100-time (V/W) amount. As the base, an organic base such as diisopropylethylamine and pyridine may be used, and preferably, triethylamine may be used in an amount of 1 to 10 equivalents. As the acylating reagent, an acid halide may be used in an amount of 1 to 5 equivalents. The reaction proceeds in the range of 0°C to 50°C, and the reaction time is 0.5 hour to 24 hours.

Finally, Step 3 for preparing the novel compounds represented by the formula (IX) and/or the formula (X) will be explained. In the same manner as those mentioned above, the compound represented by the formula (IX) is prepared by eliminating the acetal-protecting group in the 17-position of the compound represented by the formula (XIV) using an acid such as difluoroacetic acid in a mixed solvent of acetonitrile and water. When R₈ is a hydrogen atom in the formula (XIV), the compound can be converted into the compounds represented by the formula (IX) and/or the formula (X), and furthermore, by prolongation of the reaction time or elevation of the concentration of the acid to be used, the compound can be converted solely into the compound represented by the formula (X).

The preparation method of the compound according to the present invention is not limited to those explained above or those specifically shown in examples. In addition, the compound of the present invention is not limited to those prepared by the methods explained above or the methods specifically shown in examples, and those prepared by any method fall within the scope of the present invention. For example, it should be understood that the compounds obtained by applying known means to synthesize, produce, extract, and purify these compounds, on the basis of the aforementioned general explanations and the specific explanations in the examples, also fall within the scope of the present invention.

The compound of the present invention forms a salt with variety of bases or acids, and this property is utilized for manufacture of pure substances or for a form provided as medicaments. For the manufacture, for example, an acidification will make the compound soluble in a polar solvent such as water, which enables extraction and purification or isolation as a form of salt which exerts desired physicochemical properties. For the use for the medicaments, the compound can be in the form of a pharmaceutically acceptable salt. As an active ingredient of the medicament of the present invention, a substance in any of the aforementioned forms may be used.

Forms of salts which the compounds of the present invention form are not particularly limited. Forms of pharmaceutically acceptable salts of the aforementioned compounds are preferred. For example, as base addition salts, examples include a lithium salt, a sodium salt, a potassium salt, a magnesium salt, a calcium salt, a salt with ammonia or an appropriate nontoxic amine, e.g., a salt with a C1-C6 alkylamine (triethylamine and the like), a salt with a C1-C6 alkanolamine (diethanol amine, triethanol amine and the like), a procaine salt, a salt with a cyclohexylamine (dicyclohexylamine and the like), a salt with a benzylamine (N-methylbenzylamine, N-ethylbenzylamine, N-benzyl- β-phenethylamine, N,N-dibenzylethylenediamine, dibenzylamine and the like), or a salt with a heterocyclic amine (morpholine, N-ethylpyridine and the like). Examples of acid addition salts include, for example, a salt with a hydrohalogenic acid (hydrofluoric acid, hydrochloric acid, hydrobromic acid, hydroiodic acid and the like), a salt with an inorganic acid (sulfate, nitrate, phosphate, perchlorate, carbonate and the like), a salt with a carboxylic acid (acetic acid, trichloroacetic acid, trifluoroacetic acid, hydroxyacetic acid, lactic acid, citric acid, tartaric acid, oxalic acid, benzoic acid, mandelic acid, butyric acid, maleic acid, propionic acid, formic acid, malic acid and the like), a salt with an amino acid (arginine acid, aspartic acid, glutamic acid salt and the like), a salt with an organic acid (methanesulfonic acid, p-toluenesulfonic acid and the like).

As for the forms of solvates which the compounds of the present invention may form, types of solvents are not particularly limited. Examples include, for example, water; an alcohol such as methanol, ethanol and isopropanol; an ether such as tetrahydrofuran and the like.

The configurations in the aforementioned general formulas (I), (II) and (III) according to the present invention represent absolute configurations (the three-dimensional descriptions are accord with those ordinarily used). The compound of the present invention may optionally have one or more asymmetric carbons in the substituents in addition to the asymmetric carbon atoms depicted in the aforementioned general formulas (I), (II) and (III). Both of any stereoisomers (optically active isomers and diastereoisomers) based on the asymmetric carbon atoms existing in the substituents and any mixtures thereof (racemates, diastereomeric mixtures) fall within the scope of the present invention. In addition to the compounds in a free form represented by the aforementioned general formulas (I), (II) and (III) or salts thereof, any hydrates thereof or any solvates thereof also fall within the scope of the present invention.

The medicament which comprises a substance selected from the group consisting of the compound of the present invention and a pharmaceutically acceptable salt thereof, and a hydrate thereof and a solvate thereof as an active ingredient can be administered in either of administration routes, i.e., oral administration or parenteral administration (e.g., intravenous injection, intramuscular injection, subcutaneous administration, intraperitoneal administration, rectal administration, and percutaneous administration) to a human and to an animal other than a human.

Accordingly, the medicament of the present invention can be in a form of an appropriate pharmaceutical composition depending on the route of administration. Specifically, the medicament can be prepared as any of pharmaceutical forms basically including injections such as intravenous injection and intramuscular injection, oral preparations such as capsules, tablets, granules, powders, pills, subtilized granules, and troches, rectal administration agents, oleaginous suppositories, aqueous suppositories and the like.

These various preparations can be manufactured by an ordinary method using excipients, bulking agents, binders, penetrants, disintegrants, surface active agents, lubricants, dispersants, buffering agents, preservatives, solubilizing aids, antiseptics, flavoring and corrigent agents, soothing agents, stabilizers and the like which are ordinarily used.

Examples of the excipient include, for example, lactose, fructose, glucose, corn starch, sorbitol, crystalline cellulose and the like, examples of the disintegrant include, for example, starch, sodium alginate, gelatin, calcium carbonate, calcium citrate, dextrin, magnesium carbonate, synthetic magnesium silicate and the like, examples of the binder include, for example, methyl cellulose or a salt thereof, ethyl cellulose, gum Arabic, gelatin, hydroxypropyl cellulose, polyvinylpyrolidone and the like, examples of the lubricant include talc, magnesium stearate, polyethylene glycol, hardening vegetable oil and the like, and examples of the other additives include syrup, vaseline, glycerol, ethanol, propylene glycol, citric acid, sodium chloride, sodium sulfite, sodium phosphate and the like.

An amount of the aforementioned active ingredient contained in the medicament of the present invention is generally 10 to 95 wt%, preferably 30 to 80 wt% in the total composition, which depends on the form of a preparation.

A dose is appropriately chosen in consideration of a method of administration, the age and sexuality of a patient, a type of a disease, a degree of a symptom and the like, and generally about 1 to 3,000 mg, preferably 10 to 2,000 mg for an adult per day as the weight of the compound of the present invention. This dose can be administered once a day or a few times a day in divided portions.

### Examples

The present invention will be exemplified in more detail with reference to the following examples. The following examples are described only to demonstrate the present invention, and the scope of the present invention is not limited to these examples.

### Example 1

### Preparation method of 9,2',3"-tri-O-acetyl-10,11,12,13-tetrahydro-10,11,12,13-tetrahydroxymidecamycin 18-dimethylacetal (in the formula (VI), the compound wherein R₁ is propionyl group, R₂ is acetoxy group, R₈ is acetyl group, R₉ is propionyl group, R₁₂ is acetyl group and R₁₅ is methyl group)

9,3"-Di-O-acetylmidecamycin 18-dimethylacetal (International Publication WO 02/64607) 64.2 g was added with and dissolved in acetonitrile 610 ml, and added with acetic anhydride 7.8 ml, and the mixture was stirred at 40°C for 24 hours. After the reaction mixture was concentrated under reduced pressure, ethyl acetate 660 ml was added, and the organic layer was successively washed twice with saturated aqueous sodium hydrogencarbonate 300 ml, and saturated brine 300 ml. The organic layer was dried over anhydrous sodium sulfate, and then filtered, and the filtrate was concentrated under reduced pressure to obtain 9,2',3"-tri-O-acetylmidecamycin 18-dimethylacetal (in the formula (V), the compound wherein R₁ is propionyl group, R₂ is acetoxy group, R₈ is acetyl group, R₉ is propionyl group, R₁₂ is acetyl group, and R₁₅ is methyl group) 67.0 g.

This compound 20.0 g was added with and dissolved in acetone 500 ml and water 77 ml, and added with N-methylmorpholine-N-oxide 9.5 ml and 4% aqueous osmium tetraoxide 19.5 ml, and the mixture was stirred at room temperature. After 20 hours, N-methylmorpholine-N-oxide 2.4 ml was added, and the mixture was further stirred for 4.5 hours. After the reaction mixture was concentrated under reduced pressure, ethyl acetate 600 ml was added, and the organic layer was successively washed with water 200 ml, 5% aqueous sodium thiosulfate 300 ml, and saturated brine 300 ml. The organic layer was dried over anhydrous sodium sulfate, and filtered, and then the filtrate was concentrated under reduced pressure. The resulting residue was roughly purified by flush silica gel column chromatography (chloroform/methanol (25:1-15:1)). The resulting roughly-purified product was further purified by flush silica gel column chromatography (chloroform/ethyl acetate/methanol (30:30:1-25:25:1)) to obtain the title compound 8.46 g. Physicochemical properties of the present compound;
(1) Molecular formula: C₄₉H₈₃NO₂₃
(2) Mass spectrum (FAB): m/z 1054 (M+H)⁺
(3) Specific rotation: [ α ]_{D}²¹ -81°(c1.0, CHCl₃)
(4) ¹H NMR spectrum (300MHz, CDCl₃) δ(ppm): 0.96(d, 19-H), 1.07(d, 6"-H), 1.13(t, 3-OCOCH₂CH₃), 1.19(t, 4"-OCOCH₂CH₃), 1.20(d, 6'-H), 1.27(d, 16-H), 1.41 (s, 3"-CH₃), 1.50(b r dd, 14-H), 1.68(dd, 2"-Hax), 1.88(b r dd, 17-H), 2.03(s, 9-OCOCH₃), 2.04(s, 3"-OCOCH₃), 2.17(s, 2'-OCOCH₃), 2.35(m, 8-H), 2.44(s, 3'-N(CH₃)₂), 2.60(t, 3'-H), 2.73(dd,2-H), 3.15(t, 4'-H), 3.18(s, 18-OCH₃), 3.20(d, 2"-Heq), 3.22(s, 18-OCH₃), 3.27(dq, 5'-H), 3.39(br d, 4-H), 3.57(s, 4-OCH₃), 3.63(m, 12-H), 3.83(br d, 5-H), 3.91(dd, 10-H), 4.09(br t, 13-H), 4.38(dd, 18-H), 4.48(dq, 5"-H), 4.57(d, 4"-H), 4.70 (d, 1'-H), 4.82(d, 1"-H), 4.96(dd, 2'-H),5.02(m, 9-H), 5.04(m, 15-H), 5.34(br d, 3-H).

### Example 2

### Preparation method of (-)-(1R)-1-methyl-3-oxopropyl (3R,4S,5S,6R,8R,9R)-9-acetoxy-5-[2-O-acetyl-4-O-(3-O-acetyl-2,6-dideoxy-3-C-methyl-4-O-propionyl-α-L-ribohexopyranosyl)-3,6-dideoxy-3-dimethylamino-β-D-glucopyranosyl]-6-(2,2-dimethoxyethyl)-4-methoxy-8-methyl-10-oxo-3-propionyloxydecanoate (in the formula (VII), the compound wherein R₁ is propionyl group, R₂ is acetoxy group, R₈ is acetyl group, R₉ is propionyl group, R₁₂ is acetyl group, and R₁₅ is methyl group)

The compound of Example 1 (30 mg) was dissolved in benzene 1 ml, added with sodium carbonate 18 mg, and then with lead tetraacetate 29 mg divided into 5 portions over 20 minutes. After the reaction mixture was stirred at room temperature for 1 hour, the supernatant was transferred into a separatory funnel. The residue was added with benzene 5 ml, the supernatant was transferred into the separatory funnel, and then the same operations were repeated 3 times. Water 10 ml and saturated aqueous sodium hydrogencarbonate 15 ml were added to the separatory funnel to wash the organic layer. The organic layer was further washed with saturated brine 15 ml, dried over anhydrous sodium sulfate, and then filtered. The filtrate was concentrated under reduced pressure, and the resulting residue was purified by preparative TLC (hexane/acetone (2:3)) to obtain the title compound 6.5 mg.
Physicochemical properties of the present compound:
(1) Molecular formula: C₄₇H₇₇NO₂₁
(2) Mass spectrum (FAB): m/z 992 (M+H)⁺
(3) ¹H NMR spectrum (300MHz, CDCl₃) δ(ppm): 1.05(d, 8- CH₃), 1.08(d, 6"-H), 1.14(t, 3-OCOCH₂CH₃), 1.20(t, 4"-OCOCH₂CH₃), 1.21(d, 6'-H), 1.33(d, OCH(CH₃) CH₂CHO), 1.42 (s, 3"-CH₃), 1.68(dd, 2"-Hax), 1.85(m, 6-H), 2.03(s, 3"-OCOCH₃), 2.07(s, 2'-OCOCH₃), 2.20(s, 9-OCOCH₃), 2.36, 2.37(2xq, 4"-OCOCH₂CH₃), 2.44(s, 3'-N(CH₃)₂), 2.62(t, 3'-H), 2.64(brd, 2-H), 2.76 (dd, 2-H), 3.17(t, 4'-H), 3.19(s, 6-CH₂CH(OCH₃)₂), 3.23(d, 2"-Heq), 3.26(s, 6-CH₂CH(OCH₃)₂), 3.46(dd, 4-H), 3.52(s, 4-OCH₃), 3.82(br d, 5-H), 4.48(dq, 5"-H), 4.58 (d, 4"-H), 4.66(d, 1'-H), 4.81(d, 1"-H), 4.92(br d, 9-H), 4.96(dd, 2'-H), 5.27(br dd, 3-H), 5.39(ddq, OCH(CH₃)CH₂CHO), 9.59(d, 10-H), 9.75(dd, OCH(CH₃)CH₂CHO).

### Example 3

### Preparation method of (-)-(3R,4S,5S,6R,8R,9R,14R)-9-acetoxy-5-[2-O-acetyl-4-O-(3-O-acetyl-2,6-dideoxy-3-C-methyl-4-O-propionyl-α-L-ribohexopyranosyl)-3,6-dideoxy-3-dimethylamino-β-D-glucopyranosyl]-6-(2,2-dimethoxyethyl)-4-methoxy-8,11-dimethyl-3-propionyloxy-11-aza-pentadecan-14-olide (in the formula (VIII), the compound wherein R₁ is propionyl group, R₂ is acetoxy group, both of R₃ and R₁₅ are methyl groups, R₈ is acetyl group, R₉ is propionyl group, and R₁₂ is acetyl group)

The compound of Example 2 (457 mg) was added with and dissolved in ethanol 46 ml, and added with acetic acid 395 ml, methylamine hydrochloride 32 mg, and sodium cyanoborohydride 75 mg under ice cooling, and the mixture was stirred for 18 hours, then further added with sodium cyanoborohydride 75 mg, and stirred at room temperature for 4 hours. The reaction mixture was diluted with ethyl acetate 180 ml, and washed successively with water 50 ml, saturated aqueous sodium hydrogencarbonate 50 ml, and saturated brine 50 ml, and the organic layer was dried over anhydrous sodium sulfate, and then filtered. The filtrate was concentrated under reduced pressure, and the resulting residue was purified by silica gel column chromatography (chloroform/methanol (60:1-50:1)) to obtain the title compound 51 mg. Physicochemical properties of the present compound:
(1) Molecular formula: C₄₈H₈₂N₂O₁₉
(2) Mass spectrum (FAB): m/z 991 (M+H)⁺
(3) Specific rotation: [ α ]_{D}²² -72°(c1.0, CHCl₃)
(4) ¹H NMR spectrum (300MHz, CDCl₃) δ(ppm): 0.94(d, 18-H), 1.07(d, 6"-H), 1.14(t, 3-OCOCH₂CH₃), 1.19(t, 4"-OCOCH₂CH₃), 1.20(d, 6'-H), 1.24(d, 15-H), 1.41 (s, 3"-CH₃), 1.53(br dd, 16-H), 1.67(dd, 2"-Hax), 1.82(br s, 16-H), 2.02(s, 9-OCOCH₃), 2.03(s, 3"-OCOCH₃), 2.04(s, 2'-OCOCH₃), 2.26(s, 11-NCH₃), 2.43(s, 3'-N(CH₃)₂), 2.58(dd, 2-H), 2.62(t, 3'-H), 2.82(dd, 2-H), 3.14(t, 4'-H), 3.17(s, 17-OCH₃), 3.19(d, 2"-Heq), 3.25(s, 17-OCH₃), 3.35(br d, 4-H), 3.57(s, 4-OCH₃), 3.86(br d, 5-H), 4.48(dq, 5"-H), 4.56(d, 4"-H), 4.68(d, 1'-H), 4.81(d, 1"-H), 4.98(dd, 2'-H), 5.12(m, 14-H), 5.16(m, 3-H).

### Example 4

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-9-hydroxy-4-methoxy-8,11-dimethyl-3-propionyloxy-11-aza-pentadecan-14-olide (in the formula (VIII), the compound wherein R₁ is propionyl group, R₂ is hydroxyl group, both of R₃ and R₁₅ are methyl groups, R₈ is acetyl group, R₉ is propionyl group and R₁₂ is hydrogen atom)

The compound of Example 3 (50 mg) was added with and dissolved in methanol 2 ml, and the mixture was stirred at room temperature for 71.5 hours. The reaction mixture was concentrated under reduced pressure and the resulting residue was purified by preparative TLC (chloroform/methanol/aqueous ammonia (10:1:0.1)) to obtain the title compound 7 mg.
Physicochemical properties of the present compound:
(1) Molecular formula: C₄₄H₇₈N₂O₁₇
(2) Mass spectrum (FAB): m/z 907 (M+H)⁺
(3) Specific rotation: [ α ]_{D}²³ -60°(c0.4, CHCl₃)
(4) ¹H NMR spectrum (300MHz, CDCl₃) δ (ppm): 0.97(d, 18-H), 1.09(d, 6"-H), 1.13(t, 3-OCOCH₂CH₃), 1.20(t, 4"-OCOCH₂CH₃), 1.21(d, 6'-H), 1.27(d, 15-H), 1.42(s, 3"-CH₃), 1.53(m, 8-H), 1.60 (m, 16-H), 1.69(dd,2"-Hax), 1.90(br dd, 16-H), 2.02(s, 3"-OCOCH₃), 2.39(s, 11-NCH₃), 2.55(s, 3'-N(CH₃)₂), 2.62(dd, 2-H), 2.79 (dd, 2-H), 3.16(s, 17-OCH₃), 3.23(d, 2"-Heq), 3.27(s, 17-OCH₃), 3.43(dd, 2'-H), 3.54(br d, 4-H), 3.60(s, 4-OCH₃), 3.87(br d, 5-H), 4.47(d, 1'-H), 4.57(d, 4"-H), 4.85(d, 1"-H), 4.90(m, 14-H), 5.27(br dd, 3-H).

### Example 5

### Preparation method of (-)-(3R,4S,5S,6R,8R,9R,14R)-5-[4-O-(3-O-acetyl-2,6-dideoxy-3-C-methyl-4-O-propionyl- α -L-ribo-hexopyranosyl)-3,6-dideoxy-3-dimethylamino-β-D-glucopyranosyl]-6-formylmethyl-9-hydroxy-4-methoxy-8,11-dimethyl-3-propionyloxy-11-aza-pentadecan-14-olide (in the formula (IX), the compound wherein R₁ is propionyl group, R₂ is hydroxyl group, R₃ is methyl group, R₈ is acetyl group and R₉ is propionyl group)

The compound of Example 4 (15 mg) was added with a mixed solvent of the same amounts of acetonitrile and water 450 ml, and difluoroacetic acid 21 ml, and the mixture was stirred at room temperature for 25 hours. The reaction mixture was diluted with chloroform 25 ml, and washed with saturated aqueous sodium hydrogencarbonate 15 ml. Further, the organic layer was successively washed with saturated aqueous sodium hydrogencarbonate 20 ml, and saturated brine 20 ml, dried over anhydrous sodium sulfate, and then filtered. The filtrate was concentrated under reduced pressure, and the resulting residue was purified by preparative TLC (chloroform/methanol/aqueous ammonia (15:1:0.1)) to obtain the title compound 10 mg. Physicochemical properties of the present compound:
(1) Molecular formula: C₄₂H₇₂N₂O₁₆
(2) Mass spectrum (FAB): m/z 861 (M+H)⁺
(3) Specific rotation: [ α ]_{D}²⁵ -72°(c0.48, CHCl₃)
(4) ¹H NMR spectrum (300MHz, CDCl₃) δ (ppm): 0.99(d, 18-H), 1.10(d, 6"-H), 1.15(d, 6'-H), 1.17(t, 3-OCOCH₂CH₃), 1.20(t, 4"-OCOCH₂CH₃), 1.28(d, 15-H), 1.42(s, 3"-CH₃), 1.56(br dd, 7-H), 1.70(dd, 2"-Hax), 1.88(m, 13-H), 2.01(s, 3"-OCOCH₃), 2.07(br t, 6-H), 2.34(s, 11-NCH₃), 2.56(s, 3'-N(CH₃)₂), 2.80(dd, 2-H), 2.96 (dd, 16-H), 3.22(m, 4'-H), 3.22(m, 5'-H), 3.23(d, 2"-Heq), 3.37(dd, 2'-H), 3.60(s, 4-OCH₃), 3.62(dd, 4-H), 3.87(br d, 5-H), 4.43(d, 1'-H), 4.53(dq, 5"-H), 4.59(d, 4"-H), 4.86(d, 1"-H), 4.88(m, 14-H), 5.40(br dd, 3-H), 9.66(s, 17-H).

### Example 6

### Preparation method of (-)-(3R,4S,5S,6R,8R,9R,14R)-9-acetoxy-5-[2-O-acetyl-4-O-(3-O-acetyl-2,6-dideoxy-3-C-methyl-4-O-propionyl-α-L-ribohexopyranosyl)-3,6-dideoxy-3-dimethylamino-β-D-glucopyranosyl]-11-benzyl-6-(2,2-dimethoxyethyl)-4-methoxy-8-methyl-3-propionyloxy-11-aza-pentadecan-14-olide (in the formula (VIII), the compound wherein R₁ is propionyl group, R₂ is acetoxy group, R₃ is benzyl group, R₈ is acetyl group, R₉ is propionyl group, R₁₂ is acetyl group, and R₁₅ is methyl group)

In the same manner as the method of Example 3, the title compound 6.7 mg was obtained from the compound of Example 2 (60 mg) by using benzylamine instead of methylamine hydrochloride.
Physicochemical properties of the present compound:
(1) Molecular formula: C₅₄H₈₆N₂O₁₉
(2) Mass spectrum (FAB): m/z 1067 (M+H)⁺
(3) Specific rotation: [ α ]_{D}²² -79°(c0.64, CHCl₃)
(4) ¹H NMR spectrum (300MHz, CDCl₃) δ (ppm): 0.90(d, 18-H), 1.08(d, 6"-H), 1.16(t, 3-OCOCH₂CH₃), 1.19(d, 15-H), 1.20(t, 4"-OCOCH₂CH₃),1.25(d, 6'-H), 1.43 (s, 3"-CH₃), 1.59(m, 16-H), 1.69(dd, 2"-Hax), 1.85(m, 16-H), 2.02(s, 9-OCOCH₃), 2.03(s, 3"-OCOCH₃), 2.06(s, 2'-OCOCH₃), 2.45(s, 3'-N(CH₃)₂), 2.84(dd, 2-H), 3.16(s, 17-OCH₃), 3.21(d, 2"-Heq), 3.27(s, 17-OCH₃), 3.48 (br d, 4-H), 3.56(s, 4-OCH₃), 3.59(d, C₆H₅CH₂), 3.71(d, C₆H₅CH₂), 3.91(br d, 5-H), 4.52(dq, 5"-H), 4.58(d, 4"-H), 4.69(d, 1'-H), 4.82(d, 1"-H), 4.90(m, 9-H), 4.90 (m, 14-H), 4.99(dd, 2'-H), 5.19(br dd, 3-H), 7.29(m, C₆H₅). ¹³C NMR spectrum (400MHz, CDCl₃) δ (ppm): 17.0(18-C), 17.3(6"-C),18.0(6'-C), 20.2(15-C), 22.1(3"-CH₃), 30.4(7-C), 31.7(16-C), 32.0(13-C), 33.6(8-C), 34.0(6-C), 36.2(2"-C), 36.8(2-C), 49.2(12-C), 53.8(10-C), 58.6(11-CH₂), 61.3(4-OCH₃), 63.0(5"-C), 68.0(3'-C), 69.9(3-C), 70.4(14-C), 70.8(2'-C), 72.7(5'-C), 75.1(9-C), 75.8(5-C), 77.7(4"-C), 78.1(3"-C), 79.2(4'-C), 97.8(1"-C), 100.6(1'-C), 101.5(17-C), 169.4(1-C).

### Example 7

### Preparation method of (-)-(3R,4S,5S,6R,8R,9R,14R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-11-benzyl-6-(2,2-dimethoxyethyl)-4-methoxy-8-methyl-3-propionyloxy-11-aza-pentadecan-14-olide (in the formula (VIII), the compound wherein R₁ is propionyl group, R₂ is acetoxy group, R₃ is benzyl group, R₈ is acetyl group, R₉ is propionyl group, R₁₂ is hydrogen atom and R₁₅ is methyl group)

The compound of Example 6 (20 mg) was added with and dissolved in a mixed solvent of methanol and water (9:1) 2 ml, and the mixture was stirred at 55°C for 24 hours. The reaction mixture was concentrated under reduced pressure, and the resulting residue was purified by preparative TLC (chloroform/methanol (20:1)) to obtain the title compound 11 mg.
Physicochemical properties of the present compound:
(1) Molecular formula: C₅₂H₈₄N₂O₁₈
(2) Mass spectrum (FAB): m/z 1025 (M+H)⁺
(3) Specific rotation: [ α ]_{D}²¹ -60°(c0.61, CHCl₃)
(4) ¹H NMR spectrum (300MHz, CDCl₃) δ(ppm): 0.90(d, 18-H), 1.10(d, 6"-H), 1.15(t, 3-OCOCH₂CH₃), 1.16(d, 15-H), 1.20(t, 4"-OCOCH₂CH₃),1.24(d, 6'-H), 1.43(s, 3"-CH₃), 1.56(m, 16-H), 1.69(dd, 2"-Hax), 2.03(s, 9-OCOCH₃), 2.03(s, 3"-OCOCH₃), 2.56(s, 3'-N(CH₃)₂), 2.87(dd, 2-H), 3.17(s, 17-OCH₃), 3.27(s, 17-OCH₃), 3.44(dd, 2'-H), 3.54(d, C₆H₅CH₂), 3.57(br d, 4-H), 3.62(s, 4-OCH₃), 3.72(d, C₆H₅CH₂), 3.92(br d, 5-H), 4.51(d, 1'-H), 4.54(dq, 5"-H), 4.60(d, 4"-H), 4.87(d, 1"-H), 4.87(m, 14-H),4.91(m, 9-H), 5.24(br dd, 3-H), 7.24(m, C₆H₅), 7.29(m, C₆H₅).

### Example 8

### Preparation method of (-)-(3R,4S,5S,6R,8R,9R,14R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-11-benzyl-6-formylmethyl-4-methoxy-8-methyl-3-propionyloxy-11-aza-pentadecan-14-olide (in the formula (IX), the compound wherein R₁ is propionyl group, R₂ is acetoxy group, R₃ is benzyl group, R₈ is acetyl group, and R₉ is propionyl group)

In the same manner as the method of Example 5, the title compound 20 mg was obtained from the compound of Example 7 (25 mg).
Physicochemical properties of the present compound:
(1) Molecular formula: C₅₀H₇₈N₂O₁₇
(2) Mass spectrum (FAB): m/z 979 (M+H)⁺
(3) Specific rotation: [ α ]_{D}²¹ -70°(c0.47, CHCl₃)
(4) ¹H NMR spectrum (300MHz, CDCl₃) δ(ppm): 0.90(d, 18-H), 1.10(d, 6"-H), 1.16(d, 6'-H), 1.17(t, 3-OCOCH₂CH₃), 1.19(d, 15-H), 1.20(t, 4"-OCOCH₂CH₃), 1.43(s, 3"-CH₃), 1.48(br dd, 7-H), 1.70(dd, 2"-Hax), 2.02(s, 9-OCOCH₃), 2.03(s, 3"-OCOCH₃), 2.15(m, 6-H), 2.58(s, 3'-N(CH₃)₂),2.85(dd, 2-H), 2.95 (dd, 16-H), 3.24(d, 2"-Heq), 3.25(m, 4'-H), 3.25(m, 5'-H), 3.38(dd, 2'-H), 3.55(d, C₆H₅CH₂), 3.61( s , 4-OCH₃), 3.64(br d, 4-H), 3.73(d, C₆H₅CH₂), 3.94(br d, 5-H), 4.48(d, 1'-H), 4.48(dq, 5"-H), 4.60(d, 4"-H), 4.78(br q, 14-H), 4.89(d, 1"-H), 4.89(m, 9-H), 5.38(br dd, 3-H), 7.25(m, C₆H₅), 7.29(m, C₆H₅), 9.65(s, 17-H).

### Example 9

### Preparation method of (-)-(3R,4S,5S,6R,8R,9R,14R)-9-acetoxy-5-[2-O-acetyl-4-O-(3-O-acetyl-2,6-dideoxy-3-C-methyl-4-O-propionyl- α -L-ribohexopyranosyl)-3,6-dideoxy-3-dimethylamino- β-D-glucopyranosyl]-6-(2,2-dimethoxyethyl)-4-methoxy-8-methyl-11-(2-phenylethyl)-3-propionyloxy-11-azapentadecan-14-olide (in the formula (VIII), the compound wherein R₁ is propionyl group, R₂ is acetoxy group, R₃ is 2-phenylethyl group, R₈ is acetyl group, R₉ is propionyl group, R₁₂ is acetyl group, and R₁₅ is methyl group)

In the same manner as the method of Example 3, the title compound 48 mg was obtained from the compound of Example 2 (600 mg) by using 2-phenylethylamine instead of methylamine hydrochloride.
Physicochemical properties of the present compound:
(1) Molecular formula: C₅₅H₈₈N₂O₁₉
(2) Mass spectrum (FAB): m/z 1081 (M+H)⁺
(3) Specific rotation: [ α ]_{D}²⁵ -86°(c1.0, CHCl₃)
(4) ¹H NMR spectrum (300MHz, CDCl₃) δ (ppm): 0.93(d, 18-H), 1.08(d, 6"-H), 1.15(t, 3-OCOCH₂CH₃), 1.20(t, 4"-OCOCH₂CH₃), 1.21(d, 6'-H), 1.23(d, 15-H), 1.42(s, 3"-CH₃), 1.55(br dd, 16-H), 1.68(dd, 2"-Hax), 1.82(m, 16-H), 2.02(s, 9-OCOCH₃), 2.03(s, 3"-OCOCH₃), 2.05(s, 2'-OCOCH₃), 2.44(s, 3'-N(CH₃)₂), 2.83(dd, 2-H), 3.15(s, 17-OCH₃), 3.20(d, 2"-Heq), 3.26(s, 17-OCH₃), 3.43(br d, 4-H), 3.56(s, 4-OCH₃), 3.90(br d, 5-H), 4.50(dq, 5"-H), 4.57(d, 4"-H), 4.68(d, 1'-H), 4.78(m, 9-H), 4.82(d, 1"-H), 4.94 (ddq, 14-H), 4.99(dd, 2'-H), 5.17(m, 3-H), 7.19(m, C₆H₅), 7.27(m, C₆H₅).

### Example 10

### Preparation method of (-)-(3R,4S,5S,6R,8R,9R,14R)-9-acetoxy-5-[4-O-(3-O-acetyl-2,6-dideoxy-3-C-methyl-4-O-propionyl- α-L-ribo-hexopyranosyl)-3,6-dideoxy-3-dimethylamino- β-D-glucopyranosyl]-6-(2,2-dimethoxyethyl)-4-methoxy-8-methyl-11-(2-phenylethyl)-3-propionyloxy-11-aza-pentadecan-14-olide (in the formula (VIII), the compound wherein R₁ is propionyl group, R₂ is acetoxy group, R₃ is 2-phenylethyl group, R₈ is acetyl group, R₉ is propionyl group, R₁₂ is hydrogen atom, and R₁₅ is methyl group) and (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-11-(2-phenylethyl)-3-propionyloxy-11-aza-pentadecan-14-olide (in the formula (VIII), the compound wherein R₁ is propionyl group, R₂ is hydroxyl group, R₃ is 2-phenylethyl group, R₈ is acetyl group, R₉ is propionyl group, R₁₂ is hydrogen atom, and R₁₅ is methyl group)

In the same manner as the method of Example 4, the 9-acetoxyazalide 12 mg and the 9-hydroxyazalide 6.8 mg of the title compounds were obtained from the compound of Example 9 (23 mg).
Physicochemical properties of the 9-acetoxyazalide:
(1) Molecular formula: C₅₃H₈₆N₂O₁₈
(2) Mass spectrum (FAB): m/z 1039 (M+H)⁺
(3) Specific rotation: [ α ]_{D}²⁴ -65°(c1.0, CHCl₃)
(4) ¹H NMR spectrum (300MHz, CDCl₃) δ (ppm): 0.93(d, 18-H), 1.10(d, 6"-H), 1.15(t, 3-OCOCH₂CH₃), 1.20(t, 4"-OCOCH₂CH₃), 1.22(d, 6'-H), 1.24(d, 15-H), 1.42(s, 3"-CH₃), 1.55(m, 16-H), 1.70(dd, 2"-Hax), 2.03(s, 9-OCOCH₃), 2.03(s, 3"-OCOCH₃), 2.56(s, 3'-N(CH₃)₂), 2.85(dd, 2-H), 3.18(s, 17-OCH₃), 3.20(m, 4'-H), 3.20(m, 5'-H), 3.23(d, 2"-Heq), 3.27(s, 17-OCH₃), 3.44(dd, 2'-H), 3.50(br d, 4-H), 3.62(s, 4-OCH₃), 3.91(br d, 5-H), 4.50(d, 1'-H), 4.54(dq, 5"-H), 4.59(d, 4"-H), 4.81(m, 9-H), 4.86(d, 1"-H), 4.95(m, 14-H), 5.23(m, 3-H), 7.19(m, C₆H₅), 7.27(m, C₆H₅).
   Physicochemical properties of the 9-hydroxyazalide:

(1) Molecular formula: C₅₁H₈₄N₂O₁₇
(2) Mass spectrum (FAB): m/z 997 (M+H)⁺
(3) Specific rotation: [ α ]_{D}²⁴ -67°(c 0.71, CHCl₃)
(4) ¹H NMR spectrum (300MHz, CDCl₃) δ(ppm): 0.95(d, 18-H), 1.10(d, 6"-H), 1.13(t, 3-OCOCH₂CH₃), 1.20(t, 4"-OCOCH₂CH₃), 1.22(d, 6'-H), 1.24(d, 15-H), 1.40(br dd, 7-H), 1.43(s, 3"-CH₃), 1.61(m, 8-H), 1.61(m, 16-H), 1.70(dd, 2"-Hax), 1.89(ddd, 16-H), 2.02(s, 3"-OCOCH₃), 2.40(t, 3'-H), 2.55(s, 3'-N(CH₃)₂), 2.59(dd, 2-H), 2.82(dd, 2-H), 3.14(s, 17-OCH₃), 3.22(m, 4'-H), 3.22(m, 5'-H), 3.23(d, 2"-Heq), 3.27(s, 17-OCH₃), 3.41(dd, 2'-H), 3.55(dd, 4-H), 3.60(s, 4-OCH₃), 3.90(br d, 5-H), 4.48(d, 1'-H), 4.50(dd, 17-H), 4.55(dq, 5"-H), 4.59(d, 4"-H), 4.77(ddq, 14-H), 4.86(d, 1"-H), 5.20(br dd, 3-H), 7.20(m, C₆H₅), 7.30(m, C₆H₅).

### Example 11

### Preparation method of (-)-(3R,4S,5S,6R,8R,9R,14R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-formylmethyl-4-methoxy-8-methyl-11-(2-phenylethyl)-3-propionyloxy-11-aza-pentadecan-14-olide (in the formula (IX), the compound wherein R₁ is propionyl group, R₂ is acetoxy group, R₃ is 2-phenylethyl group, R₈ is acetyl group, and R₉ is propionyl group)

In the same manner as the method of Example 5, the title compound 20 mg was obtained from the 9-acetoxyazalide 22 mg which was obtained in Example 10. Physicochemical properties of the present compound:
(1) Molecular formula: C₅₁H₈₀N₂O₁₇
(2) Mass spectrum (FAB): m/z 993 (M+H)⁺
(3) Specific rotation: [ α ]_{D}²³ -65°(c0.98, CHCl₃)
(4) ¹H NMR spectrum (300MHz, CDCl₃) **δ**(ppm): 0.93(d, 18-H), 1.10(d, 6"-H), 1.15(d, 6'-H), 1.18(t, 3-OCOCH₂CH₃), 1.20(t, 4"-OCOCH₂CH₃), 1.27(d, 15-H), 1.42(s, 3"-CH₃), 1.48(br dd, 7-H), 1.70(dd, 2"-Hax), 2.02(s, 9-OCOCH₃), 2.02(s, 3"-OCOCH₃), 2.20(m, 6-H), 2.57(s, 3'-N(CH₃)₂),2.95 (dd, 16-H), 3.23(m, 4'-H), 3.23(m, 5'-H), 3.23(d, 2"-Heq), 3.27(dd, 2'-H), 3.58(br d, 4-H), 3.60( s , 4-OCH₃), 3.94(br d, 5-H), 4.46(d, 1'-H), 4.52(dq, 5"-H), 4.59(d, 4"-H), 4.79(m, 9-H), 4.87(d, 1"-H), 4.87(m, 14-H), 5.37(m, 3-H), 7.19(m, C₆H₅), 7.27(m, C₆H₅), 9.65(s, 17-H).

### Example 12

### Preparation method of (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino-β-D-glucopyranosyl]-6-formylmethyl-9-hydroxy-4-methoxy-8-methyl-11-(2-phenylethyl)-3-propionyloxy-11-aza-pentadecan-14-olide (in the formula (IX), the compound wherein R₁ is propionyl group, R₂ is hydroxyl group, R₃ is 2-phenylethyl group, R₈ is acetyl group, and R₉ is propionyl group)

In the same manner as the method of Example 5, the title compound 12 mg was obtained from the 9-hydroxyazalide 14 mg which was obtained in Example 10. Physicochemical properties of the present compound:
(1) Molecular formula: C₄₉H₇₈N₂O₁₆
(2) Mass spectrum (FAB): m/z 951 (M+H)⁺
(3) Specific rotation: [ α ]_{D}²² -73°(c0.60, CHCl₃)
(4) ¹H NMR spectrum (300MHz, CDCI₃) δ (ppm): 0.96(d, 18-H), 1.10(d, 6"-H), 1.15(d, 6'-H), 1.17(t, 3-OCOCH₂CH₃), 1.20(t, 4"-OCOCH₂CH₃), 1.27(d, 15-H), 1.42(s, 3"-CH₃), 1.46(br dd, 7-H), 1.71(dd, 2"-Hax), 2.01(s, 3"-OCOCH₃), 2.01(m, 6-H), 2.56(s, 3'-N(CH₃)₂), 2.60(dd, 2-H), 2.81(dd, 2-H), 2.96(dd, 16-H), 3.23(m, 4'-H), 3.23(m, 5'-H), 3.23(d, 2"-Heq), 3.34(dd, 2'-H), 3.60( s , 4-OCH₃), 3.64(br d, 4-H), 3.89(br d, 5-H), 4.44(d, 1'-H), 4.51(dq, 5"-H), 4.59(d, 4"-H), 4.76(ddq, 14-H), 4.86(d, 1"-H), 5.33(br dd, 3-H), 7.20(m, C₆H₅), 7.30(m, C₆H₅), 9.65(s, 17-H).

### Example 13

### Preparation method of (-)-(3R,4S,5S,6R,8R,9R,14R)-9-acetoxy-5-(2-O-acetyl-4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8-methyl-11-(3-phenylpropyl)-3-propionyloxy-11-aza-pentadecan-14-olide (in the formula (VIII), the compound wherein R₁ is propionyl group, R₂ is acetoxy group, R₃ is 3-phenylpropyl group, R₈ is acetyl group, R₉ is propionyl group, R₁₂ is acetyl group, and R₁₅ is methyl group)

In the same manner as the method of Example 3, the title compound 49 mg was obtained from the compound of Example 2 (604 mg) by using 3-phenylpropylamine instead of methylamine hydrochloride.
Physicochemical properties of the present compound:
(1) Molecular formula: C₅₆H₉₀N₂O₁₉
(2) Mass spectrum (FAB): m/z 1095 (M+H)⁺
(3) Specific rotation: [ α ]_{D}²⁶ -86°(c1.0, CHCl₃)
(4) ¹H NMR spectrum (300MHz, CDCl₃) δ (ppm): 0.92(d, 18-H), 1.07(d, 6"-H), 1.14(t, 3-OCOCH₂CH₃), 1.19(t, 4"-OCOCH₂CH₃), 1.20(d, 6'-H), 1.24(d, 15-H), 1.42(s, 3"-CH₃), 1.54(br dd, 16-H), 1.67(dd, 2"-Hax), 1.70(m, Ph(CH₂)₃), 1.85(m, 16-H), 1.99(s, 9-OCOCH₃), 2.02(s, 3"-OCOCH₃), 2.04(s, 2'-OCOCH₃), 2.44(s, 3'-N(CH₃)₂), 2.84(dd, 2-H), 3.14(s, 17-OCH₃), 3.14(m, 4'-H), 3.15(d, 2"-Heq), 3.25(s, 17-OCH₃), 3.46(br d, 4-H), 3.55(s, 4-OCH₃), 3.90(br d, 5-H), 4.49(dq, 5"-H), 4.57(d, 4"-H), 4.68(d,1'-H), 4.79(m, 9-H), 4.81(d, 1"-H), 4.93(m, 14-H), 4.98(dd, 2'-H), 5.16(m, 3-H), 7.17(m, C₆H₅), 7.27(m, C₆H₅)

### Example 14

### Preparation method of (-)-(3R,4S,5S,6R,8R,9R,14R)-9-acetoxy-5-[4-O-(3-O-acetyl-2,6-dideoxy-3-C-inethyl-4-O-propionyl-α-L-ribo-hexopyranosyl)-3,6-dideoxy-3-dimethylamino-β-D-glucopyranosyl]-6-(2,2-dimethoxyethyl)-4-methoxy-8-methyl-11-(3-phenylpropyl)-3-propionyloxy-11-aza-pentadecan-14-olide (in the formula (VIII), the compound wherein R₁ is propionyl group, R₂ is acetoxy group, R₃ is 3-phenylpropyl group, R₈ is acetyl group, R₉ is propionyl group, R₁₂ is hydrogen atom, and R₁₅ is methyl group) and (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl)-6-(2,2-dimethoxyethyl)-9-hydroxy-4-methoxy-8-methyl-11-(3-phenylpropyl)-3-propionyloxy-11-aza-pentadecan-14-olide (in the formula (VIII), the compound wherein R₁ is propionyl group, R₂ is hydroxyl group, R₃ is 3-phenylpropyl group, R₈ is acetyl group, R₉ is propionyl group, R₁₂ is hydrogen atom, and R₁₅ is methyl group)

In the same manner as the method of Example 4, the 9-acetoxyazalide 18 mg and the 9-hydroxyazalide 15 mg were obtained from the compound of Example 13 (49 mg).
Physicochemical properties of the 9-acetoxyazalide:
(1) Molecular formula: C₅₄H₈₈N₂O₁₈
(2) Mass spectrum (FAB): m/z 1053 (M+H)⁺
(3) Specific rotation: [ α ]_{D}²⁴ -58°(c0.90, CHCl₃)
(4) ¹H NMR spectrum (300MHz, CDCl₃) δ(ppm): 0.93(d, 18-H), 1.10(d, 6"-H), 1.15(t, 3-OCOCH₂CH₃), 1.20(t, 4"-OCOCH₂CH₃), 1.22(d, 6'-H), 1.25(d, 15-H), 1.43(s, 3"-CH₃), 1.70(dd, 2"-Hax), 2.01(s, 9-OCOCH₃), 2.03(s, 3"-OCOCH₃), 2.56(s, 3'-N(CH₃)₂), 2.86(dd, 2-H), 3.17(s, 17-OCH₃), 3.23(d, 2"-Heq), 3.27(s, 17-OCH₃), 3.44(dd, 2'-H), 3.54(br d, 4-H), 3.62(s, 4-OCH₃), 3.91(br d, 5-H), 4.50(d, 1'-H), 4.51(dq, 5"-H), 4.59(d,4"-H), 4.82(m, 9-H), 4.86(d, 1"-H), 4.93(m, 14-H), 5.22(m, 3-H), 7.17(m, C₆H₅), 7.27(m, C₆H₅). Physicochemical properties of the 9-hydroxyazalide:
   (1) Molecular formula: C₅₂H₈₆N₂O₁₇
   (2) Mass spectrum (FAB): m/z 1011 (M+H)⁺
   (3) Specific rotation: [α]_{D}²⁶ -60°(c0.76, CHCl₃)
   (4) ¹H NMR spectrum (300MHz, CDCl₃) δ (ppm): 0.96(d, 18-H), 1.10(d, 6"-H), 1.13(t, 3-OCOCH₂CH₃), 1.20(t, 4"-OCOCH₂CH₃), 1.22(d, 6'-H), 1.26(d, 15-H), 1.43(s, 3"-CH₃), 1.44(br dd, 7-H), 1.60(m, 8-H), 1.60(m, 16-H), 1.70(dd, 2"-Hax), 1.86(m, Ph(CH₂)₃), 1.90(m, 16-H), 2.02(s, 3"-OCOCH₃), 2.55(s, 3'-N(CH₃)₂), 2.83(dd, 2-H), 3.13(s, 17-OCH₃), 3.22(m, 4'-H), 3.22(m, 5'-H), 3.23(d, 2"-Heq), 3.27(s, 17-OCH₃), 3.41(dd, 2'-H), 3.58(br d, 4-H), 3.60(s, 4-OCH₃), 3.90(br d, 5-H), 4.49(d, 1'-H), 4.52(dq, 5"-H), 4.59(d, 4"-H), 4.83(ddq, 14-H), 4.86(d, 1"-H), 5.22(br dd, 3-H), 7.19(m, C₆H₅), 7.29(m, C₆H₅).

### Example 15

### Preparation method of (-)-(3R,4S,5S,6R,8R,9R,14R)-9-acetoxy-5-[4-O-(3-O-acetyl-2,6-dideoxy-3-C-methyl-4-0-propionyl- α -L-ribo-hexopyranosyl)-3,6-dideoxy-3-dimethylamino-β-D-glucopyranosyl]-6-formylmethyl-4-methoxy-8-methyl-11-(3-phenylpropyl)-3-propionyloxy-11-aza-pentadecan-14-olide (in the formula (IX), the compound wherein R₁ is propionyl group, R₂ is acetoxy group, R₃ is 3-phenylpropyl group, R₈ is acetyl group, and R₉ is propionyl group)

In the same manner as the method of Example 5, the title compound 12 mg was obtained from the 9-acetoxyazalide 18 mg which was obtained in Example 14. Physicochemical properties of the present compound:
(1) Molecular formula: C₅₂H₈₂N₂O₁₇
(2) Mass spectrum (FAB): m/z 1007 (M+H)⁺
(3) Specific rotation: [ α ]_{D}²⁵ -70°(c0.61, CHCl₃)
(4) ¹H NMR spectrum (300MHz, CDCl₃) δ(ppm): 0.93(d, 18-H), 1.10(d, 6"-H), 1.16(d, 6'-H), 1.18(t, 3-OCOCH₂CH₃), 1.20(t, 4"-OCOCH₂CH₃), 1.26(d, 15-H), 1.43(s, 3"-CH₃), 1.45(br dd, 7-H), 1.71(dd, 2"-Hax), 2.00(s, 9-OCOCH₃), 2.02(s, 3"-OCOCH₃), 2.20(m, 6-H), 2.57(s, 3'-N(CH₃)₂),2.84(dd, 2-H), 2.95(dd, 16-H), 3.23(m, 4'-H), 3.23(m, 5'-H), 3.23(d, 2"-Heq), 3.26(dd, 2'-H), 3.59( s , 4-OCH₃), 3.94(dd, 5-H), 4.46(d, 1'-H), 4.49(dq, 5"-H), 4.60(d, 4"-H), 4.79(m, 14-H), 4.82(m, 9-H), 4.87(d, 1"-H), 5.36(m, 3-H), 7.18(m, C₆H₅), 7.28(m, C₆H₅), 9.64(s, 17-H).

### Example 16

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl)-6-formylmethyl-9-hydroxy-4-methoxy-8-methyl-11-(3-phenylpropyl)-3-propionyloxy-11-aza-pentadecan-14-olide (in the formula (IX), the compound wherein R₁ is propionyl group, R₂ is hydroxyl group, R₃ is 3-phenylpropyl group, R₈ is acetyl group, and R₉ is propionyl group)

In the same manner as the method of Example 5, the title compound 12 mg was obtained from the 9-hydroxyazalide 15 mg which was obtained in Example 14. Physicochemical properties of the present compound:
(1) Molecular formula: C₅₀H₈₀N₂O₁₆
(2) Mass spectrum (FAB): m/z 965 (M+H)⁺
(3) Specific rotation: [ α ]_{D}²⁶ -72°(c0.61, CHCl₃)
(4) ¹H NMR spectrum (300MHz, CDCl₃) δ(ppm): 0.98(d, 18-H), 1.10(d, 6"-H), 1.15(d, 6'-H), 1.17(t, 3-OCOCH₂CH₃), 1.20(t, 4"-OCOCH₂CH₃), 1.27(d, 15-H), 1.43(s, 3"-CH₃), 1.47(m, 8-H), 1.71(dd, 2"-Hax), 2.01(s, 3"-OCOCH₃), 2.56(s, 3'-N(CH₃)₂), 2.63(t, Ph(CH₂)₃), 2.82(dd, 2-H), 2.97(dd, 16-H), 3.23(m, 4'-H), 3.23(m, 5'-H), 3.27(d, 2"-Heq), 3.53(dd, 2'-H), 3.60( s , 4-OCH₃), 3.65(dd, 4-H), 3.89(br d, 5-H), 4.44(d, 1'-H), 4.50(dq, 5"-H), 4.60(d, 4"-H), 4.79(ddq, 14-H), 4.86(d, 1"-H), 5.34(br dd, 3-H), 7.19(m, C₆H₅), 7.29(m, C₆H₅), 9.66(s, 17-H).

### Example 17

### Preparation method of (-)-(3R,4S,5S,6R,8R,9R,14R)-9-acetoxy-5-[2-O-acetyl-4-O-(3-O-acetyl-2,6-dideoxy-3-C-methyl-4-O-propionyl- α-L-ribo-hexopyranosyl)-3,6-dideoxy-3-dimethylamino- β-D-glucopyranosyl]-6-(2,2-dimethoxyethyl)-4-methoxy-8-methyl-11-(4-phenylbutyl)-3-propionyloxy-11-aza-pentadecan-14-olide (in the formula (VIII), the compound wherein R₁ is propionyl group, R₂ is acetoxy group, R₃ is 4-phenylbutyl group, R₈ is acetyl group, R₉ is propionyl group, R₁₂ is acetyl group, and R₁₅ is methyl group)

In the same manner as the method of Example 3, the title compound 32 mg was obtained from the compound of Example 2 (373 mg) by using 4-phenylbutylamine instead of methylamine hydrochloride.
Physicochemical properties of the present compound:
(1) Molecular formula: C₅₇H₉₂N₂O₁₉
(2) Mass spectrum (FAB): m/z 1109 (M+H)⁺
(3) Specific rotation: [ α ]_{D}²³ -69°(c0.53, CHCl₃)
(4) ¹H NMR spectrum (300MHz, CDCl₃) **δ** (ppm): 0.92(d, 18-H), 1.08(d, 6"-H), 1.14(t, 3-OCOCH₂CH₃), 1.20(t, 4"-OCOCH₂CH₃), 1.21(d, 6'-H), 1.25(d, 15-H), 1.42(s, 3"-CH₃), 1.58(m, 16-H), 1.68(dd, 2"-Hax), 1.81(m, 16-H), 1.99(s, 9-OCOCH₃), 2.02(s, 3"-OCOCH₃), 2.05(s, 2'-OCOCH₃), 2.44(s, 3'-N(CH₃)₂), 2.83(dd, 2-H), 3.14(s, 17-OCH₃), 3.20(d, 2"-Heq), 3.26(s, 17-OCH₃), 3.45(br d, 4-H), 3.55(s, 4-OCH₃), 3.89(br d, 5-H), 4.50(dq, 5"-H), 4.57(d, 4"-H), 4.68(d, 1'-H), 4.78(m, 9-H), 4.81(d, 1"-H), 4.94(m, 14-H), 4.98(dd, 2'-H), 5.16(m, 3-H), 7.18(m, C₆H₅), 7.27(m, C₆H₅).

### Example 18

### Preparation method of (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-11-(4-phenylbutyl)-3-propionyloxy-11-aza-pentadecan-14-olide (in the formula (VIII), the compound wherein R₁ is propionyl group, R₂ is hydroxyl group, R₃ is 4-phenylbutyl group, R₈ is acetyl group, R₉ is propionyl group, R₁₂ is hydrogen atom, and R₁₅ is methyl group)

In the same manner as the method of Example 4, the title compound 15 mg was obtained from the compound of Example 17 (26 mg).
Physicochemical properties of the present compound:
(1) Molecular formula: C₅₃H₈₈N₂O₁₇
(2) Mass spectrum (FAB): m/z 1025 (M+H)⁺
(3) Specific rotation: [ α ]_{D}²¹ -68°(c0.60, CHCl₃)
(4) ¹H NMR spectrum (300MHz, CDCl₃) δ(ppm): 0.95(d, 18-H), 1.07(d, 6"-H), 1.11(t, 3-OCOCH₂CH₃), 1.18(t, 4"-OCOCH₂CH₃), 1.19(d, 6'-H), 1.24(d, 15-H), 1.40(s, 3"-CH₃), 1.68(dd, 2"-Hax), 1.87(br dd, 16-H), 2.00(s, 3"-OCOCH₃), 2.53(s, 3'-N(CH₃)₂), 2.79(dd, 2-H), 3.11(s, 17-OCH₃), 3.21(d, 2"-Heq), 3.24(s, 17-OCH₃), 3.38(dd, 2'-H), 3.54(br d, 4-H), 3.58(s, 4-OCH₃), 3.87(br d, 5-H), 4.47(d, 1'-H), 4.51(dq, 5"-H), 4.57(d,4"-H), 4.78(m, 14-H), 4.84(d, 1"-H), 5.20(br dd, 3-H), 7.16(m, C₆H₅), 7.26(m, C₆H₅).

### Example 19

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-formylmethyl-9-hydroxy-4-methoxy-8-methyl-11-(4-phenylbutyl)-3-propionyloxy-11-aza-pentadecan-14-olide (in the formula (IX), the compound wherein R₁ is propionyl group, R₂ is hydroxyl group, R₃ is 4-phenylbutyl group, R₈ is acetyl group, and R₉ is propionyl group)

In the same manner as the method of Example 5, the title compound 15 mg was obtained from the compound of Example 18 (23 mg).
Physicochemical properties of the present compound:
(1) Molecular formula: C₅₁H₈₂N₂O₁₆
(2) Mass spectrum (FAB): m/z 979 (M+H)⁺
(3) Specific rotation: [ α ]_{D}²² -72°(c0.75, CHCl₃)
(4) ¹H NMR spectrum (300MHz, CDCl₃) **δ** (ppm): 0.98(d, 18-H), 1.10(d, 6"-H), 1.15(d, 6'-H), 1.17(t, 3-OCOCH₂CH₃), 1.20(t, 4"-OCOCH₂CH₃), 1.27(d, 15-H), 1.42(s, 3"-CH₃), 1.49(m, Ph(CH₂)₄), 1.64(quint, Ph(CH₂)₄), 1.70(dd, 2"-Hax), 2.01(s, 3"-OCOCH₃), 2.36(dd, 16-H), 2.56(s, 3'-N(CH₃)₂), 2.63(t, Ph(CH₂)₄), 2.81(dd, 2-H), 2.97(dd, 16-H), 3.22(m, 4'-H), 3.22(m, 5'-H), 3.23(d, 2"-Heq), 3.34(dd, 2'-H), 3.60( s , 4-OCH₃), 3.65(dd, 4-H), 3.89(br d, 5-H), 4.44(d, 1'-H), 4.51(dq, 5"-H), 4.59(d, 4"-H), 4.78(br dq, 14-H), 4.86(d, 1"-H), 5.34(br dd, 3-H), 7.19(m, C₆H₅),7.28(m, C₆H₅), 9.65(s, 17-H).

### Example 20

### Preparation method of (-)-(3R,4S,5S,6R,8R,9R,14R)-9-acetoxy-5-[2-O-acetyl-4-O-(3-O-acetyl-2,6-dideoxy-3-C-methyl-4-O-propionyl- α-L-ribo-hexopyranosyl)-3,6-dideoxy-3-dimethylamino-β-D-glucopyranosyl]-6-(2,2-dimethoxyethyl)-4-methoxy-8-methyl-11-(5-phenylpentyl)-3-propionyloxy-11-aza-pentadecan-14-olide (in the formula (VIII), the compound wherein R₁ is propionyl group, R₂ is acetoxy group, R₃ is 5-phenylpentyl group, R₈ is acetyl group, R₉ is propionyl group, R₁₂ is acetyl group, and R₁₅ is methyl group)

In the same manner as the method of Example 3, the title compound 34 mg was obtained from the compound of Example 2 (650 mg) by using 5-phenylpentylamine instead of methylamine hydrochloride.
Physicochemical properties of the present compound:
(1) Molecular formula: C₅₈H₉₄N₂O₁₉
(2) Mass spectrum (FAB): m/z 1122 (M+H)⁺
(3) Specific rotation: [ α ]_{D}²⁶ -87°(c0.50, CHCl₃)
(4) ¹H NMR spectrum (300MHz, CDCl₃) **δ** (ppm): 0.92(d, 18-H), 1.07(d, 6"-H), 1.14(t, 3-OCOCH₂CH₃), 1.19(t, 4"-OCOCH₂CH₃), 1.20(d, 6'-H), 1.26(d, 15-H), 1.42(s, 3"-CH₃), 1.54(br dd, 16-H), 1.74(dd, 2"-Hax), 1.82(m, 16-H), 2.00(s, 9-OCOCH₃), 2.02(s, 3"-OCOCH₃), 2.05(s, 2'-OCOCH₃), 2.44(s, 3'-N(CH₃)₂), 2.60(t, Ph(CH₂)₅), 2.83(dd, 2-H), 3.14(s, 17-OCH₃), 3.16(m, 4'-H), 3.19(d, 2"-Heq), 3.25(m, 5'-H), 3.25(s, 17-OCH₃), 3.45(br d, 4-H), 3.55(s, 4-OCH₃), 3.89(br d, 5-H), 4.49(dq, 5"-H), 4.57(d, 4"-H), 4.68(d, 1'-H), 4.76(m, 9-H), 4.81(d, 1"-H), 4.95(m, 14-H), 4.98(dd, 2'-H), 5.16(m, 3-H), 7.17(m, C₆H₅), 7.27(m, C₆H₅)

### Example 21

### Preparation method of (-)-(3R,4S,5S,6R,8R,9R,14R)-5-[4-O-(3-O-acetyl-2,6-dideoxy-3-C-methyl-4-0-propionyl- α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-11-(5-phenylpentyl)-3-propionyloxy-11-aza-pentadecan-14-olide (in the formula (VIII), the compound wherein R₁ is propionyl group, R₂ is hydroxyl group, R₃ is 5-phenylpentyl group, R₈ is acetyl group, R₉ is propionyl group, R₁₂ is hydrogen atom, and R₁₅ is methyl group)

In the same manner as the method of Example 4, the title compound 27 mg was obtained from the compound of Example 20 (68 mg).
Physicochemical properties of the present compound:
(1) Molecular formula: C₅₄H₉₀N₂O₁₇
(2) Mass spectrum (FAB): m/z 1039 (M+H)⁺
(3) Specific rotation: [ α ]_{D}²⁶ -66°(c1.30, CHCl₃)
(4) ¹H NMR spectrum (300MHz, CDCl₃) δ (ppm): 0.96(d, 18-H), 1.09(d, 6"-H), 1.13(t, 3-OCOCH₂CH₃), 1.20(t, 4"-OCOCH₂CH₃), 1.21(d, 6'-H), 1.27(d, 15-H), 1.42(s, 3"-CH₃), 1.63(m, Ph(CH₂)₅), 1.70(dd, 2"-Hax), 1.89(br dd, 16-H), 2.02(s, 3"-OCOCH₃), 2.55(s, 3'-N(CH₃)₂), 2.61(m, Ph(CH₂)₅), 2.82(dd, 2-H), 3.14(s, 17-OCH₃), 3.21(m, 4'-H), 3.21(m, 5'-H), 3.23(d, 2"-Heq), 3.27(s, 17-OCH₃), 3.41(dd, 2'-H), 3.45(br d, 9-H), 3.57(dd, 4-H), 3.60(s, 4-OCH₃), 3.90(br d, 5-H), 4.48(d, 1'-H), 4.50(dd, 17-H), 4.53(dq, 5"-H), 4.59(d, 4"-H), 4.83(m, 14-H), 4.86(d, 1"-H), 5.22(br dd, 3-H), 7.17(m, C₆H₅), 7.28(m, C₆H₅).

### Example 22

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-formylmethyl-9-hydroxy-4-methoxy-8-methyl-11-(5-phenylpentyl)-3-propionyloxy-11-aza-pentadecan-14-olide (in the formula (IX), the compound wherein R₁ is propionyl group, R₂ is hydroxyl group, R₃ is 5-phenylpentyl group, R₈ is acetyl group, and R₉ is propionyl group)

In the same manner as the method of Example 5, the title compound 21 mg was obtained from the compound of Example 21 (26 mg).
Physicochemical properties of the present compound:
(1) Molecular formula: C₅₂H₈₄N₂O₁₆
(2) Mass spectrum (FAB): m/z 993 (M+H)⁺
(3) Specific rotation: [ α ]_{D}²⁵ -79°(c0.51, CHCl₃)
(4) ¹H NMR spectrum (300MHz, CDCl₃) δ(ppm): 0.98(d, 18-H), 1.10(d, 6"-H), 1.15(d, 6'-H), 1.17(t, 3-OCOCH₂CH₃), 1.20(t, 4"-OCOCH₂CH₃), 1.27(d, 15-H), 1.33(quint, Ph(CH₂)₅), 1.42(s, 3"-CH₃), 1.45(m, 8-H), 1.48(m, 7-H), 1.63(m, Ph(CH₂)₅), 1.70(dd, 2"-Hax), 2.00(s, 3"-OCOCH₃), 2.56(s, 3'-N(CH₃)₂), 2.81(dd, 2-H), 2.96(dd, 16-H), 3.23(m, 4'-H), 3.23(m, 5'-H), 3.23(d, 2"-Heq), 3.35(dd, 2'-H), 3.60( s , 4-OCH₃), 3.65(dd, 4-H), 3.89(br d, 5-H), 4.79(m, 14-H), 4.86(d, 1"-H), 5.34(br dd, 3-H), 7.18(m, C₆H₅), 7.28(m, C₆H₅), 9.66(s, 17-H).

### Example 23

### Preparation method of (-)-(3R,4S,5S,6R,8R,9R,14R)-9-acetoxy-5-[2-O-acetyl-4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8-methyl-3 -propionyloxy-11-(3-(quinolin-4-yl)propyl)-11-aza-pentadecan-14-olide (in the formula (VIII), the compound wherein R₁ is propionyl group, R₂ is acetoxy group, R₃ is 3-(quinolin-4-yl)propyl group, R₈ is acetyl group, R₉ is propionyl group, R₁₂ is acetyl group, and R₁₅ is methyl group)

In the same manner as the method of Example 3, the title compound 136 mg was obtained from the compound of Example 2 (1.25 g) by using 3-(quinolin-4-yl)propylamine instead of methylamine hydrochloride.
Physicochemical properties of the present compound:
(1) Molecular formula: C₅₉H₉₁N₃O₁₉
(2) Mass spectrum (FAB): m/z 1146 (M+H)⁺
(3) Specific rotation: [ α ]_{D}²⁶ -79°(c1.0, CHCl₃)
(4) ¹H NMR spectrum (300MHz, CDCl₃) δ(ppm): 0.93(d, 18-H), 1.06(d, 6"-H), 1.13(t, 3-OCOCH₂CH₃), 1.18(t, 4"-OCOCH₂CH₃), 1.22(d, 6'-H), 1.23(d, 15-H), 1.40(s, 3"-CH₃), 1.54(m, 16-H), 1.67(dd, 2"-Hax), 1.86(m, quinoline-(CH₂)₃), 1.96(s, 9-OCOCH₃), 2.01(s, 3"-OCOCH₃), 2.03(s, 2'-OCOCH₃), 2.43(s, 3'-N(CH₃)₂), 2.84(dd, 2-H), 3.07(t, quinoline-(CH₂)₃), 3.12(s, 17-OCH₃), 3.19(d, 2"-Heq), 3.24(s, 17-OCH₃), 3.48(dd, 4-H), 3.54(s, 4-OCH₃), 3.89(br d, 5-H), 4.47(dq, 5"-H), 4.56(d, 4"-H), 4.67(d, 1'-H), 4.80(d, 1"-H), 4.84(m, 9-H), 4.93(m, 14-H), 4.97(dd, 2'-H), 5.17(m, 3-H), 7.25(d, quinoline), 7.56(ddd, quinoline), 7.70(ddd, quinoline), 8.06(br d, quinoline), 8.09(br d, quinoline), 8.79(d, quinoline).

### Example 24

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-3-propionyloxy-11-(3-(quinolin-4-yl)propyl)-11-aza-pentadecan-14-olide (in the formula (VIII), the compound wherein R₁ is propionyl group, R₂ is hydroxyl group, R₃ is 3-(quinolin-4-yl)propyl group, R₈ is acetyl group, R₉ is propionyl group, R₁₂ is hydrogen atom, and R₁₅ is methyl group)

The compound of Example 23 (135 mg) was added with and dissolved in methanol 5.4 ml, and the solution was stirred at room temperature for 47 hours. The reaction mixture was further stirred at 45°C for 44 hours, and then concentrated under reduced pressure, and the resulting residue was purified by preparative TLC (chloroform/methanol (10:1)) to obtain the title compound 29 mg.
Physicochemical properties of the present compound:
(1) Molecular formula: C₅₅H₈₇N₃O₁₇
(2) Mass spectrum (FAB): m/z 1062 (M+H)⁺
(3) Specific rotation: [ α ]_{D}²⁶ -64°(c1.30, CHCl₃)
(4) ¹H NMR spectrum (300MHz, CDCl₃) δ (ppm): 0.96(d, 18-H), 1.09(d, 6"-H), 1.13(t, 3-OCOCH₂CH₃), 1.19(t, 4"-OCOCH₂CH₃), 1.21(d, 6'-H), 1.25(d, 15-H), 1.42(s, 3"-CH₃), 1.69(dd, 2"-Hax), 1.91(m, 16-H), 1.94(quint, quinoline-(CH₂)₃), 2.01(s, 3"-OCOCH₃), 2.55(s, 3'-N(CH₃)₂), 2.59(dd, 2-H), 2.83(dd, 2-H), 3.09(t, quinoline-(CH₂)₃), 3.13(s, 17-OCH₃), 3.22(d, 2"-Heq), 3.23(m, 4'-H), 3.23(m, 5'-H), 3.26(s, 17-OCH₃), 3.40(dd, 2'-H), 3.59(dd, 4-H), 3.60(s, 4-OCH₃), 3.90(br d, 5-H), 4.48(d, 1'-H), 4.52(dq, 5"-H), 4.59(d, 4"-H), 4.83(m, 14-H), 4.86(d, 1"-H), 5.24(br dd, 3-H), 7.25(d, quinoline), 7.58(ddd, quinoline), 7.71(ddd, quinoline), 8.03(br d, quinoline), 8.12(br d, quinoline), 8.81(d, quinoline).

### Example 25

### Preparation method of (-)-(3R,4S,5S,6R,8R,9R,14R)-5-[4-O-(3-O-acetyl-2,6-dideoxy-3-C-methyl-4-0-propionyl- α-L-ribo-hexopyranosyl)-3,6-dideoxy-3-dimeth ylamino-β-D-glucopyranosyl]-6-formylmethyl-9-hydroxy-4-methoxy-8-methyl-3-propionyloxy-11-(3-(quinolin-4-yl)propyl)-11-aza-pentadecan-14-olide (in the formula (IX), the compound wherein R₁ is propionyl group, R₂ is hydroxyl group, R₃ is 3-(quinolin-4-yl)propyl group, R₈ is acetyl group, and R₉ is propionyl group)

In the same manner as the method of Example 5, the title compound 23 mg was obtained from the compound of Example 24 (28 mg).
Physicochemical properties of the present compound:
(1) Molecular formula: C₅₃H₈₁N₃O₁₆
(2) Mass spectrum (FAB): m/z 1016 (M+H)⁺
(3) Specific rotation: [ α ]_{D}²⁶-71°(c0.58, CHCl₃)
(4) ¹H NMR spectrum (300MHz, CDCl₃) δ(ppm): 0.98(d, 18-H), 1.09(d, 6"-H), 1.15(d, 6'-H), 1.16(t, 3-OCOCH₂CH₃), 1.19(t, 4"-OCOCH₂CH₃), 1.25(d, 15-H), 1.42(s, 3"-CH₃), 1.48(br s, 8-H), 1.70(dd, 2"-Hax), 1.94(m, quinoline-(CH₂)₃), 2.00(s, 3"-OCOCH₃), 2.56(s, 3'-N(CH₃)₂), 2.82(dd, 2-H), 2.98(dd, 16-H), 3.01(dt, quinoline-(CH₂)₃), 3.22(m, 4'-H), 3.22(m, 5'-H), 3.22(d, 2"-Heq), 3.34(dd, 2'-H), 3.60( s , 4-OCH₃), 3.65(dd, 4-H), 3.89(br d, 5-H), 4.45(d, 1'-H), 4.51(dq, 5"-H), 4.59(d, 4"-H), 4.79(m, 14-H), 4.86(d, 1"-H), 5.34(br dd, 3-H), 7.25(d, quinoline), 7.57(ddd, quinoline), 7.71(ddd, quinoline), 8.03(dd, quinoline), 8.12(dd, quinoline), 8.81(d, quinoline), 9.65(s, 17-H).

### Example 26

### Preparation method of (-)-(3R,4S,5S,6R,8R,14R)-5-[2-O-acetyl-4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8-methyl-11-(N-methyl-N-(3-phenylpropyl)amino)-3-propionyloxy-11-aza-pentadecan-1 4-olide (in the formula (VIII), the compound wherein R₁ is propionyl group, R₂ is hydrogen atom, R₃ is N-methyl-N-(3-phenylpropyl)amino group, R₈ is acetyl group, R₉ is propionyl group, R₁₂ is acetyl group, and R₁₅ is methyl group)

In the same manner as the method of Example 3, the title compound 57 mg was obtained from the compound of Example 2 (620 mg) by using 1-methyl-1-(3-phenylpropyl)hydrazine instead of methylamine hydrochloride. Physicochemical properties of the present compound:
(1) Molecular formula; C₅₅H₉₁N₃O₁₇
(2) Mass spectrum (FAB): m/z 1066 (M+H)⁺
(3) Specific rotation: [ α ]_{D}²⁵ -71°(c1.0, CHCl₃)
(4) ¹H NMR spectrum (300MHz, CDCl₃) δ (ppm): 0.90(d, 18-H), 1.07(d, 6"-H), 1.14(t, 3-OCOCH₂CH₃), 1.19(t, 4"-OCOCH₂CH₃), 1.20(d, 6'-H), 1.24(d, 15-H), 1.42(s, 3"-CH₃), 1.46(br dd, 16-H), 1.67(dd, 2"-Hax), 1.81(m, Ph(CH₂)₃), 2.02(s, 3"-OCOCH₃), 2.04(s, 2'-OCOCH₃), 2.25(s, 11-NCH₃), 2.44(s, 3'-N(CH₃)₂), 3.12(s, 17-OCH₃), 3.19(d, 2"-Heq), 3.25(s, 17-OCH₃), 3.27(br d, 4-H), 3.55(s, 4-OCH₃), 3.81(br s, 5-H), 4.44(dd, 17-H), 4.49(dq, 5"-H), 4.57(d, 4"-H), 4.66(d, 1'-H), 4.81(d, 1"-H), 4.88(m,14-H), 4.96(dd, 2'-H), 5.36(br dd, 3-H), 7.19(m, C₆H₅), 7.27(m, C₆H₅)

### Example 27

### Preparation method of (-)-(3R,4S,5S,6R,8R,14R)-5-[4-O-(3-O-acetyl-2,6-dideoxy-3-C-methyl-4-O-propionyl- α -L-ribo-hexopyranosyl)-3,6-dideoxy-3-dimethylamino-β-D-glucopyranosyl]-6-(2,2-dimethoxyethyl)-4-methoxy-8-methyl-11-(N-methyl-N-(3-phenylpropyl)amino)-3-propionyloxy-11-aza-pentadecan-14-olide (in the formula (VIII), the compound wherein R₁ is propionyl group, R₂ is hydrogen atom, R₃ is N-methyl-N-(3-phenylpropyl)amino group, R₈ is acetyl group, R₉ is propionyl group, R₁₂ is hydrogen atom, and R₁₅ is methyl group)

In the same manner as the method of Example 4, the title compound 38 mg was obtained from the compound of Example 26 (57 mg).
Physicochemical properties of the present compound:
(1) Molecular formula: C₅₃H₈₉N₃O₁₆
(2) Mass spectrum (FAB): m/z 1024 (M+H)⁺
(3) Specific rotation: [ α ]_{D}²⁶ -58°(c1.0, CHCl₃)
(4) ¹H NMR spectrum (300MHz, CDCl₃) δ (ppm): 0.90(d, 18-H), 1.09(d, 6"-H), 1.15(t, 3-OCOCH₂CH₃), 1.20(t, 4"-OCOCH₂CH₃), 1.23(d, 6'-H), 1.24(d, 15-H), 1.42(s, 3"-CH₃), 1.59(m, 7-H), 1.69(dd, 2"-Hax), 1.80(quint, Ph(CH₂)₃), 2.03(s, 3"-OCOCH₃), 2.25(s, 11-N CH₃), 2.55(s, 3'-N(CH₃)₂), 3.18(s, 17-OCH₃), 3.22(d, 2"-Heq), 3.27(s, 17-OCH₃), 3.47(dd, 2'-H), 3.58(s, 4-OCH₃), 3.83(br d, 5-H), 4.43(d, 1'-H), 4.56(m, 5"-H), 4.57(d, 4"-H), 4.85(d, 1"-H), 4.94(m, 14-H). 5.40(m, 3-H), 7.19(m, C₆H₅), 7.27(m, C₆H₅).

### Example 28

### Preparation method of (-)-(3R,4S,5S,6R,8R,14R)-5-[4-O-(3-O-acetyl-2,6-dideoxy-3-C-methyl-4-O-propionyl- α-L-ribo-hexopyranosyl)-3,6-dideoxy-3-dimethylamino-β-D-glucopyranosyl]-6-formylmethyl-4-methoxy-8-methyl-11-(N-methyl-N-(3-phenylpropyl)amino)-3-propionyloxy-11-aza-pentadecan-14-olide (in the formula (IX), the compound wherein R₁ is propionyl group, R₂ is hydrogen atom, R₃ is N-methyl-N-(3-phenylpropyl)amino group, R₈ is acetyl group, and R₉ is propionyl group)

In the same manner as the method of Example 5, the title compound 27 mg was obtained from the compound of Example 27 (37 mg).
Physicochemical properties of the present compound:
(1) Molecular formula: C₅₁H₈₃N₃O₁₅
(2) Mass spectrum (FAB): m/z 978 (M+H)⁺
(3) Specific rotation: [ α ]_{D}²⁵ -70°(c0.61, CHCl₃)
(4) ¹H NMR spectrum (300MHz, CDCl₃) δ(ppm): 0.91(d, 18-H), 1.09(d, 6"-H), 1.15(d, 6'-H), 1.18(t, 3-OCOCH₂CH₃), 1.20(t, 4"-OCOCH₂CH₃), 1.25(d, 15-H), 1.31(m, 8-H), 1.42(s, 3"-CH₃), 1.69(dd, 2"-Hax), 1.80(quint, Ph(CH₂)₃), 2.02(s, 3"-OCOCH₃), 2.22(br d, 16-H), 2.26(s, 11-NCH₃),2.55(s, 3'-N(CH₃)₂), 3.02(dd, 17-H), 3.22(m, 4'-H), 3.22(d, 2"-Heq), 3.23(m, 5'-H), 3.39(br d, 4-H), 3.43(dd, 2'-H), 3.57( s , 4-OCH₃), 3.87(br d, 5-H), 4.46(d, 1'-H), 4.54(dq, 5"-H), 4.59(d, 4"-H), 4.85(d, 1"-H), 4.90(m, 14-H), 5.54(br dd, 3-H), 7.19(m, C₆H₅), 7.28(m, C₆H₅), 9.63(s, 17-H).

### Example 29

### Preparation method of (-)-(3R,4S,5S,6R,8R,9R,14R)-9-acetoxy-5-[2-O-acetyl-4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-11-(4-methoxybenzyl)-8-methyl-3-propionyloxy-11-aza-pentadecan-14-olide (in the formula (VIII), the compound wherein R₁ is propionyl group, R₂ is acetoxy group, R₃ is 4-methoxybenzyl group, R₈ is acetyl group, R₉ is propionyl group, R₁₂ is acetyl group, and R₁₅ is methyl group)

In the same manner as the method of Example 3, the title compound 48 mg was obtained from the compound of Example 2 (491 mg) by using 4-methoxybenzylamine instead of methylamine hydrochloride.
Physicochemical properties of the present compound:
(1) Molecular formula: C₅₅H₈₈N₂O₂₀
(2) Mass spectrum (FAB): m/z 1097 (M+H)⁺
(3) Specific rotation: [ α ]_{D}²⁵ -87°(c1.0, CHCl₃)
(4) ¹H NMR spectrum (300MHz, CDCl₃) δ (ppm): 0 .89(d, 18-H), 1.07(d, 6"-H), 1.15(t, 3-OCOCH₂CH₃), 1.17(d, 15-H), 1.19(t, 4"-OCOCH₂CH₃), 1.21(d, 6'-H), 1.42(s, 3"-CH₃), 1.54(br dd, 16-H), 1.68(dd, 2"-Hax), 2.02(s, 9-OCOCH₃), 2.02(s, 3"-OCOCH₃), 2.05(s, 2'-OCOCH₃), 2.44(s, 3'-N(CH₃)₂), 2.83(dd, 2-H), 3.14(s, 17-OCH₃), 3.15(m, 4'-H), 3.15(m, 5'-H), 3.19(d, 2"-Heq), 3.26(s, 17-OCH₃), 3.48(br d, 4-H), 3.50(d, C₆H₄CH₂),3.55(s, 4-OCH₃), 3.64(d, C₆H₄CH₂), 3.79(s, CH₃OC₆H₄), 3.90(br d, 5-H), 4.49(dq, 5"-H), 4.57(d, 4"-H), 4.68(d, 1'-H), 4.81(d, 1"-H), 4.89(m, 9-H), 4.89 (m, 14-H), 4.98(dd, 2'-H), 5.19(m, 3-H), 6.83(d, C₆H₄), 7.19(d, C₆H₄).

### Example 30

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino- β-D-glucopyranosyl]-6-(2,2-dimethoxyethyl)-9-hydroxy-4-methoxy-11-(4-methoxybenzyl)-8-methyl-3-propionyloxy-11-azapentadecan-14-olide (in the formula (VIII), the compound wherein R₁ is propionyl group, R₂ is hydroxyl group, R₃ is 4-methoxybenzyl group, R₈ is acetyl group, R₉ is propionyl group, R₁₂ is hydrogen atom, and R₁₅ is methyl group)

In the same manner as the method of Example 7, the title compound 13 mg was obtained from the compound of Example 29 (27 mg).
Physicochemical properties of the present compound:
(1) Molecular formula: C₅₁H₈₄N₂O₁₈
(2) Mass spectrum (FAB): m/z 1013 (M+H)⁺
(3) Specific rotation: [ α ]_{D}²⁶ -68°(c0.60, CHCl₃)
(4) ¹H NMR spectrum (300MHz, CDCl₃) δ (ppm): 0.93(d, 18-H), 1.10(d, 6"-H), 1.14(t, 3-OCOCH₂CH₃), 1.20(t, 4"-OCOCH₂CH₃), 1.21(d, 6'-H),1.22(d, 15-H), 1.43(s, 3"-CH₃), 1.61(br dd, 16-H), 1.70(dd, 2"-Hax), 2.02(s, 3"-OCOCH₃), 2.56(s, 3'-N(CH₃)₂), 2.60(dd, 2-H), 2.84(dd, 2-H), 3.13(s, 17-OCH₃), 3.22(m, 4'-H), 3.22(m, 5'-H), 3.23(d, 2"-Heq), 3.27(s,17-OCH₃), 3.41(dd, 2'-H), 3.43(d, C₆H₄CH₂), 3.61(s, 4-OCH₃), 3.63(dd, 4-H), 3.73(d, C₆H₄CH₂), 3.81(s, C₆H₄OMe), 3.90(br d, 5-H), 4.49(d, 1'-H), 4.50(t, 17-H), 4.55(dq, 5"-H), 4.59(d, 4"-H), 4.82(m, 14-H), 4.86(d, 1"-H), 5.24(br dd, 3-H), 6.86(d, C₆H₄), 7.20(d, C₆H₄).

### Example 31

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-9-hydrogy-4-methoxy-8-methyl-3-propionyloxy-11-aza-pentadecan-14-olide (in the formula (VIII), the compound wherein R₁ is propionyl group, R₂ is hydroxyl group, R₃ is hydrogen atom, R₈ is acetyl group, R₉ is propionyl group, R₁₂ is hydrogen atom, and R₁₅ is methyl group)

The compound of Example 30 (63 mg) was added with and dissolved in 1,4-dioxane 1.5 ml, and the solution was added with 10% Pd-C catalyst 6.3 mg suspended in ethanol 1 ml. The atmosphere in the reaction vessel was replaced with hydrogen, and the mixture was stirred at room temperature for 135 minutes. The reaction mixture was added further with 10% Pd-C catalyst 12.6 mg suspended in ethanol 0.5 ml, stirred for 165 minutes, then added further with 10% Pd-C catalyst 6.3 mg suspended in ethanol 0.5 ml, and stirred for 1 hour, and the catalyst was removed by filtration. The filtrate was concentrated under reduced pressure, and the resulting residue was purified by preparative TLC (chloroform/methanol/aqueous ammonia (10:1:0.1)) to obtain the title compound 32 mg.
Physicochemical properties of the present compound:
(1) Molecular formula: C₄₃H₇₆N₂O₁₇
(2) Mass spectrum (FAB): m/z 893 (M+H)⁺
(3) Specific rotation: [ α ]_{D}²⁵ -67°(c0.80, CHCl₃)
(4) ¹H NMR spectrum (300MHz, CDCl₃) δ (ppm): 0.91(d, 18-H), 1.08(d, 6"-H), 1.14(t, 3-OCOCH₂CH₃), 1.19(t, 4"-OCOCH₂CH₃), 1.21(d, 6'-H), 1.27(d, 15-H), 1.41(s, 3"-CH₃), 1.69(dd, 2"-Hax), 2.01(s, 3"-OCOCH₃), 2.54(s, 3'-N(CH₃)₂), 2.60(dd, 2-H), 2.72(dd, 2-H), 2.80(br d, 10-H), 2.91(m, 12-H), 3.15(s, 17-OCH₃), 3.22(d, 2"-Heq), 3.25(s, 17-OCH₃), 3.41(dd, 2'-H), 3.62(s, 4-OCH₃), 3.86(br d, 5-H), 4.46(br t, 17-H), 4.46(d, 1'-H), 4.52(dq, 5"-H), 4.58(d, 4"-H), 4.85(d, 1"-H), 4.96(m, 14-H), 5.17(br dd, 3-H).

### Example 32

### Preparation method of (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino- β-D-glucopyranosyl]-9-O,11-carbonyl-6-(2,2-dimethoxyethyl)-4-methoxy-8-methyl-3-propionyloxy-11-aza-pentadecan-14-olide (in the formula (VIII), the compound wherein R₁ is propionyl group, R₂ and R₃ combine together to form -O-(CO)-, R₈ is acetyl group, R₉ is propionyl group, R₁₂ is hydrogen atom, and R₁₅ is methyl group)

The compound of Example 31 (30 mg) was added with and dissolved in dichloromethane 1 ml, and the solution was added with triethylamine 42 ml and a dichloromethane (0.5 ml) solution of triphosgene 11 mg under ice cooling, and stirred for 1.5 hours under ice cooling. The reaction mixture was added with chloroform 20 ml and saturated aqueous sodium hydrogencarbonate 15 ml, and the organic layer was separated. The organic layer was washed with saturated brine 15 ml, and dried over anhydrous sodium sulfate, and then filtered. The filtrate was concentrated under reduced pressure, and the resulting residue was purified by preparative TLC (ethyl acetate/methanol/aqueous ammonia (35:1:0.1)) to obtain the title compound 23 mg. Physicochemical properties of the present compound:
(1) Molecular formula: C₄₄H₇₄N₂O₁₈
(2) Mass spectrum (FAB): m/z 919 (M+H)⁺
(3) Specific rotation: [ α ]_{D}²⁶ -48°(c0.61, CHCl₃)
(4) ¹H NMR spectrum (300MHz, CDCl₃) δ(ppm): 1.02(d, 18-H), 1.08(d, 6"-H), 1.13(t, 3-OCOCH₂CH₃), 1.19(t, 4"-OCOCH₂CH₃), 1.20(d, 6'-H), 1.32(d, 15-H), 1.41(s, 3"-CH₃), 1.48(m, 16-H), 1.68(dd, 2"-Hax), 1.82(m, 13-H), 2.01(s, 3"-OCOCH₃), 2.08(m, 13-H), 2.37(q, 4"-OCOCH₂CH₃), 2.40(t, 3'-H), 2.42(q, 3-OCOCH₂CH₃), 2.54(s, 3'-N(CH₃)₂), 2.62(dd, 2-H), 2.79(dd, 2-H), 3.04(br dd, 12-H), 3.15(t, 4'-H), 3.20(d, 2"-Heq), 3.21(s, 17-OCH₃), 3.25(s, 17-OCH₃), 3.39(dd, 4-H), 3.46(dd, 10-H), 3.54(dd, 2'-H), 3.56(s, 4-OCH₃), 3.81(br d, 5-H), 3.83(m, 12-H), 4.26(br dd, 9-H), 4.46(t, 17-H), 4.47(d, 1'-H), 4.57(m, 4"-H), 4.57(m, 5"-H), 4.83(d, 1"-H), 5.04(br dq, 14-H), 5.35(br dd, 3-H).

### Example 33

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-9-O,11-carbonyl-6-formylmethyl-4-methoxy-8-methyl-3-propionyloxy-11-aza-pentadecan-14-olide (in the formula (IX), the compound wherein R₁ is propionyl group, R₂ are R₃ combine together to form -O-(CO)-, R₈ is acetyl group, and R₉ is propionyl group)

In the same manner as the method of Example 5, the title compound 18 mg was obtained from the compound of Example 32 (22 mg).
Physicochemical properties of the present compound:
(1) Molecular formula: C₄₂H₆₈N₂O₁₇
(2) Mass spectrum (FAB): m/z 873 (M+H)⁺
(3) Specific rotation: [ α ]_{D}²⁶ -55°(c0.51, CHCl₃)
(4) ¹H NMR spectrum (300MHz, CDCl₃) δ (ppm): 1.03(d, 18-H), 1.08(d, 6"-H), 1.14(d, 6'-H), 1.17(t, 3-OCOCH₂CH₃), 1.19(t, 4"-OCOCH₂CH₃), 1.33(d, 15-H), 1.41(s, 3"-CH₃), 1.63(m, 8-H), 1.68(dd, 2"-Hax), 1.81(m, 6-H), 1.85(m, 13-H), 2.01(s, 3"-OCOCH₃), 2.09(m, 13-H), 2.24(dd, 16-H), 2.40(q, 3-OCOCH₂CH₃), 2.40(t, 3'-H), 2.45(q, 4"-OCOCH₂CH₃), 2.55(s, 3'-N(CH₃)₂), 2.67(dd, 2-H), 2.80(dd, 2-H), 3.02(m, 12-H), 3.09(dd, 16-H), 3.14(t, 4'-H), 3.21(d, 2"-Heq), 3.38(dd, 4-H), 3.49(dd, 2'-H), 3.51(m, 10-H), 3.57(s, 4-OCH₃), 3.90(m, 12-H), 3.92(br d, 5-H), 4.21(br dd, 9-H), 4.44(d, 1'-H), 4.55(m, 5"-H), 4.57(m, 4"-H), 4.83(d, 1"-H), 5.03(m, 14-H), 5.43(br dd, 3-H), 9.64(s, 17-H).

### Example 34

### Preparation method of 2'-O-acetyl-9-O-tert-butyldimethylsilyl-10,11,12,13-tetrahydro-10,11,12,13-tetrahydroxyrokitamycin 18-dimethylacetal (in the formula (VI), the compound wherein R₁ is hydrogen atom, R₂ is tert-butyldimethylsilyloxy group, R₈ is propionyl group, R₉ is n-butyryl group, R₁₂ is acetyl group, and R₁₅ is methyl group)

2'-O-Acetyl-9-O-tert-butyldimethylsilylrokitamycin 18-dimethylacetal (International Publication WO 00/73317) 1.08 g was added with and dissolved in acetone 27 ml and water 4.2 ml, and the solution was added with N-methylmorpholine-N-oxide 0.49 ml and 4% aqueous osmium tetraoxide 1 ml, and stirred at room temperature for 24 hours. After the reaction mixture was concentrated under reduced pressure, the concentrate was extracted with ethyl acetate 40 ml, and then the organic layer was washed with saturated brine 30 ml. The organic layer was dried over anhydrous sodium sulfate and filtered, then the filtrate was concentrated under reduced pressure, and the resulting residue was purified by flush silica gel column chromatography (chloroform/methanol (40:1-30:1)) to obtain the title compound 394 mg.
Physicochemical properties of the present compound:
(1) Molecular formula: C₅₂H₉₅NO₂₁Si
(2) Mass spectrum (FAB): m/z 1098 (M+H)⁺
(3) Specific rotation: [ α ]_{D}²² -81°(c1.0, CHCl₃)
(4) ¹H NMR spectrum (300MHz, CDCl₃) δ(ppm): 0.04(s, 9-OSi(CH₃)₂^{t}Bu), 0.07(s, 9-OSi(CH₃)₂^{t}Bu), 0.93(d, 19-H), 0.93(s, 9-OSi(CH₃)₂^{t}Bu),0.98(t, 4"-OCO(CH₂)₂CH₃), 1.07(d, 6"-H), 1.16(t, 3"-OCOCH₂CH₃), 1.22(d,6'-H), 1.29(d, 16-H), 1.42(s, 3"-CH₃), 1.55(b r dd, 14-H), 1.69(dd, 2"-Hax), 1.70(sex, 4"-OCO(CH₂)₂CH₃), 2.02(s, 2'-OCOCH₃), 2.29, 2.32(each q, 3"-OCOCH₂CH₃), 2.38(t, 4"-OCO(CH₂)₂CH₃), 2.43(s, 3'-N(CH₃)₂), 2.59(t, 3'-H), 3.15(t, 4'-H), 3.21(d, 2"-Heq), 3.31(s, 18-OCH₃), 3.35(s, 18-OCH₃), 3.48(s, 4-OCH₃), 3.63(m, 12-H), 3.89(br d, 5-H), 4.49(dq, 5"-H),4.58(d, 4"-H), 4.82(d, 1"-H), 4.97(dd, 2'-H).

### Example 35

### Preparation method of (-)-(3R,4S,5S,6R,8R,9R,14R)-5-[2-O-acetyl-4-O-(4-O-butyryl-2,6-dideoxy-3-C-methyl-3-O-propionyl-α-L-ribo-hexopyranosyl)-3,6-dideoxy-3-dimethylamino-β-D-glucopyranosyl]-9-O-tert-butyldimethylsilyl-6-(2,2-dimethoxyethyl)-3-hydroxy-4-methoxy-8-methyl-11-(4-phenylbutyl)-11-aza-pentadecan-14-olide (in the formula (VIII), the compound wherein R₁ is hydrogen atom, R₂ is tert-butyldimethylsilyloxy group, R₃ is 4-phenylbutyl group, R₈ is propionyl group, R₉ is n-butyryl group, R₁₂ is acetyl group, and R₁₅ is methyl group)

In the same manner as the method of Example 2, from the compound of Example 34 (400 mg) instead of the compound of Example 1, (-)-(1R)-1-methyl-3-oxopropyl (3R,4S,5S,6R,8R,9R)-5-[2-O-acetyl-4-O-(4-O-butyryl -2,6-dideoxy-3-C-methyl-3-0-propionyl- α-L-ribo-hexopyranosyl)-3,6-dideoxy-3-dimethylamino-β-D-glucopyranosyl]-9-O-tert-butyldimethylsilyl-6-(2,2-dimethoxyethyl)-3-hydroxy-4-methoxy-8-methyl-10-oxodecanoate (in the formula (VII), the compound wherein R₁ is hydrogen atom, R₂ is tert-butyldimethylsilyloxy group, R₈ is propionyl group, R₉ is n-butyryl group, R₁₂ is acetyl group, and R₁₅ is methyl group) 370 mg was obtained.

From the above compound 370 mg, the title compound 40 mg was obtained in the same manner as the method of Example 3 by using 4-phenylbutylamine instead of methylamine hydrochloride.
Physicochemical properties of the present compound:
(1) Molecular formula: C₆₀H₁₀₄N₂O₁₇Si
(2) Mass spectrum (FAB): m/z 1153 (M+H)⁺
(3) Specific rotation: [ α ]_{D}²¹ -76°(c1.0, CHCl₃)
(4) ¹H NMR spectrum (300MHz, CDCl₃) δ (ppm): 0.03(s, 9-OSi(CH₃)₂^{t}Bu), 0.87(s, 9-OSi(CH₃)₂^{t}Bu), 0.90(d, 18-H), 0.98(t, 4"-OCO(CH₂)₂CH₃), 1.07(d, 6"-H), 1.15(t, 3"-OCOCH₂CH₃), 1.20(d, 6'-H), 1.22(d, 15-H), 1.42(s, 3"-CH₃), 1.55(m, Ph(CH₂)₄), 1.67(dd, 2"-Hax), 1.70(sex, 4"-OCO(CH₂)₂CH₃), 2.04(s, 2'-OCOCH₃), 2.29, 2.32(each q, 3"-OCOCH₂CH₃), 2.38(t, 4"-OCO(CH₂)₂CH₃), 2.42(s, 3'-N(CH₃)₂), 3.01(br d, 4-H), 3.14(t, 4'-H),3.20(d, 2"-Heq), 3.32(s, 17-OCH₃), 3.49(s, 4-OCH₃), 3.96(br d, 5-H), 4.48(dq, 5"-H), 4.49(d, 1'-H), 4.57(d, 4"-H), 4.81(d, 1"-H), 4.97(dd, 2'-H), 5.12(m, 14-H), 7.17(m, Ph), 7.28(m, Ph).

### Example 36

### Preparation method of (-)-(3R,4S,5S,6R,8R,9R,14R)-9-O-tert-butyldimethylsilyl-5-[4-O-(4-O-butyryl-2,6-dideoxy-3-C-methyl-3-O-propionyl-α-L-ribo-hexopyranosyl)-3,6-dideoxy-3-dimethylamino-β-D-glucopyranosyl]-6-(2,2-dimethoxyethyl)-3-hydroxy-4-methoxy-8-methyl-11-(4-phenylbutyl)-11-aza-pentadecan-14-olide (in the formula (VIII), the compound wherein R₁ is hydrogen atom, R₂ is tert-butyldimethylsilyloxy group, R₃ is 4-phenylbutyl group, R₈ is propionyl group, R₉ is n-butyryl group, R₁₂ is hydrogen atom, and R₁₅ is methyl group)

In the same manner as the method of Example 24, the title compound 35 mg was obtained from the compound of Example 35 (49 mg).
Physicochemical properties of the present compound:
(1) Molecular formula: C₅₈H₁₀₂N₂O₁₆Si
(2) Mass spectrum (FAB): m/z 1111 (M+H)⁺
(3) Specific rotation: [ α ]_{D}²² -70°(c0.85, CHCl₃)
(4) ¹H NMR spectrum (300MHz, CDCl₃) δ(ppm): 0.06(s, 9-OSi(CH₃)₂^{t}Bu), 0.89(s, 9-OSi(CH₃)₂^{t}Bu), 0.90(d, 18-H), 0.99(t, 4"-OCO(CH₂)₂CH₃), 1.09(d, 6"-H), 1.14(t, 3"-OCOCH₂CH₃), 1.22(d, 15-H), 1.22(d, 6'-H), 1.42(s, 3"-CH₃), 1.57(m, Ph(CH₂)₄), 1.70(dd, 2"-Hax), 1.70(sex, 4"-OCO(CH₂)₂CH₃), 2.30, 2.32(each q, 3"-OCOCH₂CH₃), 2.39(t, 4"-OCO(CH₂)₂CH₃), 2.53(s, 3'-N(CH₃)₂), 2.61(t, Ph(CH₂)₄), 3.10(m, 4-H), 3.12(d, 2"-Heq), 3.32(s, 17-OCH₃), 3.33(s, 17-OCH₃), 3.49(dd, 2'-H), 3.61(s, 4-OCH₃), 3.92(m, 5-H), 4.34(d, 1'-H), 4.47(t, 17-H), 4.55(m, 5"-H), 4.59(d, 4"-H ), 4.83(d, 1"-H), 5.19(m, 14-H), 7.17(m, Ph), 7.28(m, Ph).

### Example 37

### Preparation method of (-)-(3R,4S,5S,6R,8R,9R,14R)-9-O-tert-butyldimethylsilyl-5-[4-O-(4-O-butyryl -2,6-dideoxy-3-C-methyl-3-O-propionyl-α-L-ribo-hexopyranosyl)-3,6-dideoxy-3-dimethylamino-β-D-glucopyranosyl]-6-formylmethyl-3-hydroxy-4-methoxy-8-methyl-11-(4-phenylbutyl)-11-aza-pentadecan-14-olide (in the formula (IX), the compound wherein R₁ is hydrogen atom, R₂ is tert-butyldimethylsilyloxy group, R₃ is 4-phenylbutyl group, R₈ is propionyl group, and R₉ is n-butyryl group)

In the same manner as the method of Example 5, the title compound 32 mg was obtained from the compound of Example 36 (34 mg).
Physicochemical properties of the present compound:
(1) Molecular formula: C₅₆H₉₆N₂O₁₅Si
(2) Mass spectrum (FAB): m/z 1065 (M+H)⁺
(3) Specific rotation: [ α ]_{D}²⁰ -61°(c0.60, CHCl₃)
(4) ¹H NMR spectrum (300MHz, CDCl₃) **δ** (ppm): 0.02(s, 9-OSi(CH₃)₂^{t}Bu), 0.03(s, 9-OSi(CH₃)₂^{t}Bu), 0.87(s, 9-OSi(CH₃)₂^{t}Bu), 0.93(d, 18-H),0.99(t, 4"-OCO(CH₂)₂CH₃), 1.09(d, 6"-H), 1.14(t, 3"-OCOCH₂CH₃), 1.19(d,6'-H), 1.22(d, 15-H), 1.42(s, 3"-CH₃), 1.57(m, Ph(CH₂)₄), 1.65(dd, 2"-Hax), 1.70(sex, 4"-OCO(CH₂)₂CH₃), 2.39(t, 4"-OCO(CH₂)₂CH₃), 2.53(s, 3'-N(CH₃)₂), 2.62(t, Ph(CH₂)₄), 2.74(dd, 2-H), 2.97(dd, 16-H), 3.23(d, 2"-Heq), 3.43(dd, 2'-H), 3.59(s, 4-OCH₃), 3.81(br d, 5-H), 4.17(m, 3-H), 4.34(d, 1'-H), 4.53(dq, 5"-H), 4.59(d, 4"-H), 4.84(d, 1"-H), 5.14(m, 14-H), 7.18(m, Ph), 7.28(m, Ph), 9.70(s, 17-H).

### Example 38

### Preparation method of (-)-(3R,4S,5S,6R,8R,9R,14R)-5-[4-O-(4-O-butyryl-2,6-dideoxy-3-C-methyl-3-O-propionyl-α-L-ribo-hexopyranosyl)-3,6-dideoxy-3-dimethylamino-β-D-glucopyranosyl]-6-formylmethyl-3,9-dihydroxy-4-methoxy-8-methyl-11-(4-phenylbutyl)-11-aza-pentadecan-14-olide (in the formula (IX), the compound wherein R₁ is hydrogen atom, R₂ is hydroxyl group, R₃ is 4-phenylbutyl group, R₈ is propionyl group, and R₉ is n-butyryl group)

The compound of Example 37 (13 mg) was added with and dissolved in tetrahydrofuran 0.5 ml and acetic acid 0.5 ml, and the solution was added with 1M tetrabutylammonium fluoride tetrahydrofuran solution 60 ml, and stirred at 60°C for 49 hours. The reaction mixture was slowly poured into saturated aqueous sodium hydrogencarbonate 10 ml, and extracted with ethyl acetate 25 ml. The organic layer was washed successively with water 15 ml, saturated aqueous sodium hydrogencarbonate 15 ml, and saturated brine 15 ml, dried over anhydrous sodium sulfate, and then filtered. The filtrate was concentrated under reduced pressure and the resulting residue was purified by preparative TLC (chloroform/methanol/aqueous ammonia (20:1:0.1)) to obtain the title compound 6.3 mg.
Physicochemical properties of the present compound:
(1) Molecular formula: C₅₀H₈₂N₂O₁₅
(2) Mass spectrum (FAB): m/z 951 (M+H)⁺
(3) Specific rotation: [ α ]_{D}²² -95°(c0.38, CHCl₃)
(4) ¹H NMR spectrum (300MHz, CDCl₃) δ (ppm): 0.91(d, 18-H), 0.98(t, 4"-OCO(CH₂)₂CH₃), 1.09(d, 6"-H), 1.13(t, 3"-OCOCH₂CH₃), 1.17(d, 6'-H), 1.26(d, 15-H), 1.42(s, 3"-CH₃), 1.46(m, Ph(CH₂)₄), 1.63(m, Ph(CH₂)₄), 1.69(sex, 4"-OCO(CH₂)₂CH₃), 2.38(t, 4"-OCO(CH₂)₂CH₃), 2.54(s, 3'-N(CH₃)₂), 2.62(t, Ph(CH₂)₄), 2.80(dd, 2-H), 3.02 (dd, 16-H), 3.19(m, 4'-H), 3.19(m, 5'-H), 3.23(d, 2"-Heq), 3.35(dd, 2'-H), 3.59(s, 4-OCH₃), 3.82(br d, 5-H), 4.28(br dd, 3-H), 4.34(d, 1'-H), 4.52(dq, 5"-H), 4.59(d, 4"-H), 4.85(d, 1"-H), 4.94(m, 14-H), 7.18(m, Ph), 7.28(m, Ph), 9.75(s, 17-H).

### Example 39

### Preparation method of 9,2'-di-O-acetyl-10,11,12,13-tetrahydro-10,11,12,13-tetrahydroxymidecamycin 18-dimethylacetal (in the formula (VI), the compound wherein R₁ is propionyl group, R₂ is acetoxy group, R₈ is hydrogen atom, R₉ is propionyl group, R₁₂ is acetyl group, and R₁₅ is methyl group)

In the same manner as the method of Example 1, from 9-O-acetylmidecamycin 18-dimethylacetal (International Publication WO 02/64607) 20.4 g, 9,2'-di-O-acetylmidecamycin 18-dimethylacetal (in the formula (V), the compound wherein R₁ is propionyl group, R₂ is acetoxy group, R₈ is hydrogen atom, R₉ is propionyl group, R₁₂ is acetyl group, and R₁₅ is methyl group) 21.3 g was obtained.

Further, the title compound 1.77 g was obtained from the above compound 4.00 g in the same manner as the method of Example 1.
Physicochemical properties of the present compound:
(1) Molecular formula: C₄₇H₈₁NO₂₁
(2) Mass spectrum (FAB): m/z 1012 (M+H)⁺
(3) Specific rotation: [α]_{D}²¹ -74°(c1.0, CHCl₃)
(4) ¹H NMR spectrum (300MHz, CDCl₃) δ (ppm): 0.96(d, 19-H), 1.12(s, 3"-CH₃), 1.13(t, 3-OCOCH₂CH₃), 1.14(d, 6"-H), 1.18(t, 4"-OCOCH₂CH₃), 1.27(d, 16-H), 1.27(d, 6'-H), 1.43(b r dd, 7-H), 1.53(b r dd, 14-H), 1.63(m, 6-H), 1.69(b r dd, 14-H), 1.72(m, 17-H), 1.85(dd, 2"-Hax), 2.02(d, 2"-Heq), 2.05(s, 9-OCOCH₃), 2.17(s, 2'-OCOCH₃), 2.35(m, 8-H), 2.42(s, 3'-N(CH₃)₂), 2.49(dd, 2-H), 2.73(dd, 2-H), 2.73(t, 3'-H), 3.18(s, 18-OCH₃), 3.23(s, 18-OCH₃), 3.34(t, 4'-H), 3.57(s, 4-OCH₃), 3.62(br d, 12-H), 3.86(br d, 5-H), 3.90(dd, 10-H), 4.10(m, 13-H), 4.38(dq, 5"-H),4.40(dd, 18-H), 4.63(d, 4"-H), 4.74(d, 1'-H), 5.01(dd, 2'-H), 5.02(br d, 9-H), 5.07(m, 15-H), 5.08(d, 1"-H), 5.35(br d, 3-H).

### Example 40

### Preparation method of (-)-(3R,4S,5S,6R,8R,9R,14R)-9-acetoxy-5-[2-O-acetyl-4-O-(2,6-dideoxy-3-C-methyl-4-O-propionyl- α-L-ribo-hexopyranosyl)-3,6-dideoxy-3-dimethylamino-β-D-glucopyranosyl]-6-(2,2-dimethoxyethyl)-4-methoxy-8-methyl-11-(4-phenylbutyl)-3-propionytoxy-11-aza-pentadecan-14-olide (in the formula (VIII), the compound wherein R₁ is propionyl group, R₂ is acetoxy group, R₃ is 4-phenylbutyl group, R₈ is hydrogen atom, R₉ is propionyl group, R₁₂ is acetyl group, and R₁₅ is methyl group)

In the same manner as the method of Example 2, (-)-(1R)-1-methyl-3-oxopropyl (3R,4S,5S,6R,8R,9R)-9-acetoxy-5-[2-O-acetyl-4-O-(2,6-dideoxy-3-C-methyl-4-O-propionyl- α-L-ribo-hexopyranosyl)-3,6-dideoxy-3-dimethylamino-β-D-glucopyranosyl]-6-(2,2-dimethoxyethyl)-4-methoxy-8-methyl-10-oxo-3-propionyloxydecanoate (in the formula (VII), the compound wherein R₁ is propionyl group, R₂ is acetoxy group, R₈ is hydrogen atom, R₉ is propionyl group, R₁₂ is acetyl group, and Ris is methyl group) 680 mg was obtained from the compound of Example 39 (714 mg) instead of the compound of Example 1.

From the above compound 680 mg, the title compound 63 mg was obtained in the same manner as the method of Example 3 by using 4-phenylbutylamine instead of methylamine hydrochloride.
Physicochemical properties of the present compound:
(1) Molecular formula: C₅₅H₉₀N₂O₁₈
(2) Mass spectrum (FAB): m/z 1067 (M+H)⁺
(3) Specific rotation: [ α ]_{D}²¹ -61°(c1.0, CHCl₃)
(4) ¹H NMR spectrum (300MHz, CDCl₃) δ(ppm): 0.92(d, 18-H), 1.13(s, 3"-CH₃), 1.14(d, 6"-H), 1.14(t, 3-OCOCH₂CH₃), 1.18(t, 4"-O COCH₂CH₃), 1.25(d, 15-H), 1.28(d, 6'-H), 1.40(br dd, 7-H), 1.44(quint, Ph(CH₂)₄), 1.61(m, Ph(CH₂)₄), 1.85(dd, 2"-Hax), 1.99(s, 9-OCOCH₃), 2.02 (d, 2"-Heq), 2.06(s, 2'-OCOCH₃), 2.42(s, 3'-N(CH₃)₂), 2.74 (t, 3'-H), 2.83(dd, 2-H), 3.14(s, 17-OCH₃), 3.26(s, 17-OCH₃), 3.34(m, 4'-H), 3.36(m, 5'-H), 3.47(br d, 4-H), 3.55(s, 4-OCH₃), 3.91(br d, 5-H), 4.39(dq, 5"-H), 4.54(dd, 17-H), 4.63(d, 4"-H), 4.72(d, 1'-H), 4.77(m, 9-H), 4.92(br q, 14-H), 5.03(dd, 2'-H), 5.08(d, 1"-H), 5.17(m, 3-H), 7.17(m, C₆H₅), 7.27(m, C₆H₅).

### Example 41

### Preparation method of (-)-(3R,4S,5S,6R,8R,9R,14R)-5-[4-O-(2,6-dideoxy-3-C-methyl-4-O-propionyl- α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-11-(4-phenylbutyl)-3-propionyloxy-11-aza-pentadecan-14-olide (in the formula (VIII), the compound wherein R₁ is propionyl group, R₂ is hydroxyl group, R₃ is 4-phenylbutyl group, R₈ is hydrogen atom, R₉ is propionyl group, R₁₂ is hydrogen atom, and R₁₅ is methyl group)

In the same manner as the method of Example 4, the title compound 42 mg was obtained from the compound of Example 40 (104 mg).
Physicochemical properties of the present compound:
(1) Molecular formula: C₅₁H₈₆N₂O₁₆
(2) Mass spectrum (FAB): m/z 983 (M+H)⁺
(3) Specific rotation: [ α ]_{D}²¹ -58°(c0.56, CHCl₃)
(4) ¹H NMR spectrum (300MHz, CDCl₃) δ(pp m): 0.97(d, 18-H), 1.06(br dd, 7-H), 1.13(t, 3-OCOCH₂CH₃), 1.13(s, 3"-CH₃), 1.14(d, 6"-H), 1.19(t, 4"-OCOCH₂CH₃), 1.26(d, 15-H), 1.28(d, 6'-H), 1.44(br dd, 7-H), 1.48(m, Ph(CH₂)₄), 1.64(m, Ph(CH₂)₄), 1.84(dd, 2"-Hax), 2.02(d, 2"-Heq), 2.51(s, 3'-N(CH₃)₂), 2.60(dd, 2-H), 2.63(t, Ph(CH₂)₄), 2.80(dd, 2-H), 3.16(s, 17-OCH₃), 3.28(s, 17-OCH₃), 3.54(br d, 4-H), 3.56(dd, 2'-H), 3.58(s, 4-OCH₃), 3.93(br d, 5-H), 4.45(d, 1'-H), 4.47(dq, 5"-H), 4.63(d, 4"-H), 4.85(m, 14-H), 5.09(d, 1"-H), 5.24(br dd, 3-H), 7.19(m, C₆H₅), 7.28(m, C₆H₅).

### Example 42

### Preparation method of (-)-(3R,4S,5S,6R,8R,9R,14R)-5-[4-O-(2,6-dideoxy-3-C-methyl-4-O-propionyl-α-L-ribo-hexopyranosyl)-3,6-dideoxy-3-dimethylamino-β-D-glucopyranosyl]-6-formylmethyl-9-hydroxy-4-methoxy-8-methyl-11-(4-phenylbutyl)-3-propionyloxy-11-aza-pentadecan-14-olide (in the formula (IX), the compound wherein R₁ is propionyl group, R₂ is hydroxyl group, R₃ is 4-phenylbutyl group, R₈ is hydrogen atom, and R₉ is propionyl group)

In the same manner as the method of Example 5, the title compound 15 mg was obtained from the compound of Example 41 (42 mg).
Physicochemical properties of the present compound:
(1) Molecular formula: C₄₉H₈₀N₂O₁₅
(2) Mass spectrum (FAB): m/z 937 (M+H)⁺
(3) Specific rotation: [ α ]_{D}²¹ -68°(c0.40, CHCl₃)
(4) ¹H NMR spectrum (300MHz, CDCl₃) δ(ppm): 1.00(d, 18-H), 1.13(s, 3"-CH₃), 1.16(d, 6"-H), 1.16(t, 3-OCOCH₂CH₃), 1.19(t, 4"-OCOCH₂CH₃), 1.21(d, 6'-H), 1.26(d, 15-H), 1.48(m, Ph(CH₂)₄), 1.64(quint, Ph(CH₂)₄), 1.79(m, 13-H), 1.84(dd, 2"-Hax), 2.02(d, 2"-Heq), 2.05(m, 6-H), 2.38(br d, 16-H), 2.52(s, 3'-N(CH₃)₂), 2.64(t, Ph(CH₂)₄), 2.79(dd, 2-H), 2.94(dd, 16-H), 3.28(m, 4'-H), 3.30(m, 5'-H), 3.54(dd, 2'-H), 3.57(s, 4-OCH₃), 3.64(dd, 4-H), 3.90(br d, 5-H), 4.42(d, 1'-H), 4.48(dq, 5"-H), 4.63(d, 4"-H), 4.80(m, 14-H), 5.08(d, 1"-H), 5.36(br dd, 3-H), 7.18(m, C₆H₅), 7.28(m, C₆H₅), 9.66(s, 17-H).

### Example 43

### Preparation method of (-)-(3R,4S,5S,6R,8R,9R,14R)-5-(3,6-dideoxy-3-dimethylamino- β-D-glucopyranosyl)-6-formylmethyl-9-hydroxy-4-methoxy-8-methyl-11-(4-phenylbutyl)-3-propionyloxy-11-aza-pentadecan-14-olide (in the formula (X), the compound wherein R₁ is propionyl group, R₂ is hydroxyl group, and R₃ is 4-phenylbutyl group)

In the same manner as the method of Example 5, the title compound 13 mg was obtained from the compound of Example 41 (42 mg).
Physicochemical properties of the present compound:
(1) Molecular formula: C₃₉H₆₄N₂O₁₁
(2) Mass spectrum (FAB): m/z 737 (M+H)⁺
(3) Specific rotation: [ α ]_{D}²³ -35°(c0.44, CHCIs)
(4) ¹H NMR spectrum (300MHz, CDCl₃) δ(ppm): 1.00(d, 18-H), 1.06(br t, 7-H), 1.17(t, 3-OCOCH₂CH₃), 1.25(d, 6'-H), 1.27(d, 15-H), 1.49(m, Ph(CH₂)₄), 1.64(quint, Ph(CH₂)₄), 1.68(m, 13-H), 1.79(m, 13-H), 2.05(br t, 6-H), 2.35(t, 3'-H), 2.51(s, 3'-N(CH₃)₂), 2.63(t, Ph(CH₂)₄), 2.80(dd, 2-H), 2.99(dd, 16-H), 3.04(t, 4'-H), 3.30(dq, 5'-H), 3.52(dd, 2'-H), 3.58(s, 4-OCH₃), 3.65(dd, 4-H), 3.92(br d, 5-H), 4.44(d, 1'-H), 4.81(m, 14-H), 5.37(br dd, 3-H), 7.19(m, C₆H₅), 7.29(m, C₆H₅), 9.68(s, 17-H).

### Example 44

### Preparation method of (-)-(3R,4S,5S,6R,8R,9R,15R)-9-acetoxy-5-[2-O-acetyl-4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8,11,12-trimethyl-3-propionyloxy-11,12-diaza-hexadecan-15-olide (in the formula (XI), the compound wherein R₁ is propionyl group, R₂ is acetoxy group, R₈ is acetyl group, R₉ is propionyl group, all of R₁₀, R₁₁ and R₁₅ are methyl groups, and R₁₂ is acetyl group)

In the same manner as the method of Example 3, the title compound 30 mg was obtained from the compound of Example 2 (218 mg) by using 1,2-dimethylhydrazine dihydrochloride instead of methylamine hydrochloride. Physicochemical properties of the present compound:
(1) Molecular formula: C₄₉H₈₅N₃O₁₉
(2) Mass spectrum (FAB): m/z 1019 (M)⁺
(3) Specific rotation: [ α ]_{D}²⁴ -71°(c0.76, CHCl₃)
(4) ¹H NMR spectrum (300MHz, CDCl₃) δ(ppm): 0.93(d, 19-H), 1.07(d, 6"-H), 1.15(t, 3-OCOCH₂CH₃), 1.19(t, 4"-OCOCH₂CH₃), 1.20(d, 6'-H), 1.26(d, 16-H), 1.41 (s, 3"-CH₃), 1.52(m, 17-H), 1.67(dd, 2"-Hax), 1.71(m, 8-H), 2.03(s, 9-OCOCH₃), 2.03(s, 3"-OCOCH₃), 2.05(s, 2'-OCOCH₃), 2.22(s, 11-NCH₃), 2.22(s, 12-NCH₃), 2.43(s, 3'-N(CH₃)₂), 2.61(t, 3'-H), 2.75(dd, 2-H), 3.14(t, 4'-H), 3.19(d, 2"-Heq), 3.20(s, 18-OCH₃), 3.25(s, 18-OCH₃), 3.43(br d, 4-H), 3.57(s, 4-OCH₃), 3.85(br d, 5-H), 4.45(t, 18-H), 4.48(dq, 5"-H), 4.57(d, 4"-H), 4.67(d, 1'-H), 4.81(d, 1"-H), 4.90(m, 15-H), 4.98(dd, 2'-H), 5.05(br dd, 9-H), 5.22(m, 3-H).

### Example 45

### Preparation method of (-)-(3R,4S,5S,6R,8R,9R,15R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8,11,12-trimethyl-3-propionyloxy-11,12-diaza-hexadecan-15-olide (in the formula (XI), the compound wherein R₁ is propionyl group, R₂ is acetoxy group, R₈ is acetyl group, R₉ is propionyl group, all of R₁₀, R₁₁, and R₁₅ are methyl groups, and R₁₂ is hydrogen atom)

In the same manner as the method of Example 7, the title compound 22 mg was obtained from the compound of Example 44 (29 mg).
Physicochemical properties of the present compound:
(1) Molecular formula: C₄₇H₈₃N₃O₁₈
(2) Mass spectrum (FAB): m/z 978 (M+H)⁺
(3) Specific rotation: [ α ]_{D}²² -52°(c0.77, CHCl₃)
(4) ¹H NMR spectrum (300MHz, CDCl₃) δ (ppm): 0.95(d, 19-H), 1.09(d, 6"-H), 1.15(t, 3-OCOCH₂CH₃), 1.19(t, 4"-OCOCH₂CH₃), 1.21(d, 6'-H), 1.26(d, 16-H), 1.42 (s, 3"-CH₃), 1.59(m, 17-H), 1.70(dd, 2"-Hax), 2.02(s, 9-OCOCH₃), 2.05(s, 3"-OCOCH₃), 2.37, 2.38(each q, 4"-OCOCH₂CH₃), 2.42(s, 11-NCH₃), 2.42(s, 12-NCH₃), 2.43(br q, 3-OCOCH₂CH₃), 2.54(s, 3'-N(CH₃)₂), 2.78(dd, 2-H), 3.22(s, 18-OCH₃), 3.24(d, 2"-Heq), 3.27(s, 18-OCH₃), 3.46(dd, 2'-H), 3.50(br d, 4-H), 3.63(s, 4-OCH₃), 3.88(br d, 5-H), 4.45(t, 18-H), 4.46(d, 1'-H), 4.56(m, 5"-H), 4.59(d, 4"-H), 4.85(d, 1"-H), 4.94(m, 15-H), 5.05(m, 9-H), 5.31(m, 3-H).

### Example 46

### Preparation method of (-)-(3R,4S,5S,6R,8R,9R,15R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-formylmethyl-4-methoxy-8,11,12-trimethyl-3-propionyloxy-11,12-diaza-hexadecan-15-olide (in the formula (XII), the compound wherein R₁ is propionyl group, R₂ is acetoxy group, R₈ is acetyl group, R₉ is propionyl group, and both of R₁₀ and R₁₁ are methyl groups)

In the same manner as the method of Example 5, the title compound 25 mg was obtained from the compound of Example 45 (28 mg).
Physicochemical properties of the present compound:
(1) Molecular formula: C₄₅H₇₇N₃O₁₇
(2) Mass spectrum (FAB): m/z 932 (M+H)⁺
(3) Specific rotation: [ α ]_{D}²² -74°(c0.42, CHCl₃)
(4) ¹H NMR spectrum (300MHz, CDCl₃) δ(ppm): 0.97(d, 19-H), 1.09(d, 6"-H), 1.15(d, 6'-H), 1.17(t, 3-OCOCH₂CH₃), 1.19(t, 4"-OCOCH₂CH₃), 1.26(d, 16-H), 1.42 (s, 3"-CH₃), 1.70(dd, 2"-Hax), 1.76(m, 8-H), 2.02(s, 9-OCOCH₃), 2.05(s, 3"-OCOCH₃), 2.22(s, 11-NCH₃), 2.25(s, 12-NCH₃), 2.56(s, 3'-N(CH₃)₂), 3.04(dd, 17-H), 3.23(m, 4'-H), 3.23(m, 5'-H), 3.23(d, 2"-Heq), 3.40(dd, 2'-H), 3.58(s, 4-OCH₃), 3.99(br d, 5-H), 4.42(d, 1'-H), 4.50(dq, 5"-H), 4.59(d, 4"-H), 4.86(d, 1"-H), 4.90(m, 15-H), 5.00(m, 9-H), 5.38(br dd, 3-H), 9.64(s, 18-H).

### Example 47

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-11-(trans-3-phenyl-2-propenyl)-3-propionyloxy-11-aza-pentadecan-14-olide (in the formula (VIII), the compound wherein R₁ is propionyl group, R₂ is hydroxyl group, R₃ is trans-3-phenyl-2-propenyl group, R₈ is acetyl group, R₉ is propionyl group, R₁₂ is hydrogen atom, and R₁₅ is methyl group)

The compound of Example 31 (30 mg) was added with and dissolved in methanol 0.6 ml, and the solution was added with acetic acid 9.6 µl and trans-cinnamaldehyde 6.3 µl under ice cooling and stirred for 30 minutes. The mixture was added with sodium cyanoborohydride 6.3 mg, and stirred for 12 hours with gradually warming to room temperature. The reaction mixture was added with ethyl acetate 3 ml and saturated aqueous sodium hydrogencarbonate 3 ml, and stirred at room temperature for 30 minutes, the organic layer was separated, and then the aqueous layer was twice extracted with ethyl acetate 5 ml. The organic layers were combined and washed successively with saturated aqueous sodium hydrogencarbonate 10 ml and saturated brine 10 ml, dried over anhydrous sodium sulfate, and then filtered. The filtrate was concentrated under reduced pressure, and the resulting residue was purified by preparative TLC (chloroform/methanol/aqueous ammonia (20:1:0.1)) to obtain the title compound 24 mg.
Physicochemical properties of the present compound:
(1) Molecular formula: C₅₂H₈₄N₂O₁₇
(2) Mass spectrum (FAB): m/z 1009 (M+H)⁺
(3) Specific rotation: [ α ]_{D}²¹ -66°(c0.50, CHCl₃)
(4) ¹H NMR spectrum (300MHz, CDCl₃) δ (ppm): 0.94(d, 18-H), 1.07(d, 6"-H), 1.12(t, 3-OCOCH₂CH₃), 1.18(t, 4"-OCOCH₂CH₃), 1.19(d, 6'-H), 1.25(d, 15-H), 1.40(s, 3"-CH₃), 1.44(br dd, 7-H), 1.56(m, 8-H), 1.68(dd, 2"-Hax), 2.00(s, 3"-OCOCH₃), 2.53(s, 3'-N(CH₃)₂), 2.81(dd, 2-H), 3.12(s, 17-OCH₃), 3.20(m, 4'-H), 3.20(m, 5'-H), 3.21(d, 2"-Heq), 3.25(s, 17-OCH₃), 3.40(dd, 2'-H), 3.59(s, 4-OCH₃), 3.60(br d, 4-H), 3.88(br d, 5-H), 4.47(d, 1'-H), 4.50(dq, 5"-H), 4.57(d, 4"-H), 4.82(ddq, 14-H), 4.84(d, 1"-H), 5.23(br dd, 3-H), 6.20(dt, PhCH=CH), 6.50(d, PhCH=CH), 7.26(m, C₆H₅).

### Example 48

### Preparation method of (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino-β-D-glucopyranosyl]-6-formylmethyl-9-hydroxy-4-methoxy-8-methyl-11-(trans-3-phenyl-2-propenyl)-3-propionyloxy-11-aza-pentadecan-14-olide (in the formula (IX), the compound wherein R₁ is propionyl group, R₂ is hydroxyl group, R₃ is trans-3-phenyl-2-propenyl group, R₈ is acetyl group, and R₉ is propionyl group)

In the same manner as the method of Example 5, the title compound 20 mg was obtained from the compound of Example 47 (24 mg).
Physicochemical properties of the present compound:
(1) Molecular formula: C₅₀H₇₈N₂O₁₆
(2) Mass spectrum (FAB): m/z 963 (M+H)⁺
(3) Specific rotation: [ α ]_{D}²⁰ -78°(c0.40, CHCl₃)
(4) ¹H NMR spectrum (300MHz, CDCl₃) δ(ppm): 0.96(d, 18-H), 1.08(d, 6"-H), 1.13(d, 6'-H), 1.15(t, 3-OCOCH₂CH₃), 1.18(t, 4"-OCOCH₂CH₃), 1.25(d, 15-H), 1.40(s, 3"-CH₃), 1.44(m, 8-H), 1.68(dd, 2"-Hax), 1.99(s, 3"-OCOCH₃), 2.54(s, 3'-N(CH₃)₂), 2.80(dd, 2-H), 2.95(dd, 16-H), 3.21(m, 4'-H), 3.21(m, 5'-H), 3.21(d, 2"-Heq), 3.30(dd, 2'-H), 3.52(br d, 9-H), 3.58(s , 4-OCH₃), 3.66(dd, 4-H), 3.87(br d, 5-H), 4.42(d, 1'-H), 4.49(dq, 5"-H), 4.57(d, 4"-H), 4.79(ddq, 14-H), 4.84(d, 1"-H), 5.36(br dd, 3-H), 6.19(dt, PhCH=CH), 6.50(d, PhCH=CH), 7.26(m, C₆H₅), 9.63(s, 17-H).

### Example 49

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β -D-glucopyranosyl]-6-(2,2-dimethoxyethyl)-9-hydroxy-4-methoxy-8-methyl-11-(3-phenyl-2-propynyl)-3-propionyloxy-11-aza-pentadecan-14-olide (in the formula (VIII), the compound wherein R₁ is propionyl group, R₂ is hydroxyl group, R₃ is 3-phenyl-2-propynyl group, R₈ is acetyl group, R₉ is propionyl group, R₁₂ is hydrogen atom, and R₁₅ is methyl group)

In the same manner as the method of Example 47, the title compound 20 mg was obtained from the compound of Example 31 (30 mg) by using phenylpropargyl aldehyde instead of trans-cinnamaldehyde.
Physicochemical properties of the present compound:
(1) Molecular formula: C₅₂H₈₂N₂O₁₇
(2) Mass spectrum (FAB): m/z 1007 (M+H)⁺
(3) Specific rotation: [ α ]_{D}²⁰ -67°(c0.40, CHCl₃)
(4) ¹H NMR spectrum (300MHz, CDCl₃) δ (ppm): 0.95(d, 18-H), 1.07(d, 6"-H), 1.13(t, 3-OCOCH₂CH₃), 1.18(t, 4"-OCOCH₂CH₃), 1.19(d, 6'-H), 1.27(d, 15-H), 1.40(s, 3"-CH₃), 1.47(br dd, 7-H), 1.57(m, 8-H), 1.57(m, 16-H), 1.68(dd, 2"-Hax), 1.88(m, 16-H), 2.00(s, 3"-OCOCH₃), 2.53(s, 3'-N(CH₃)₂), 2.79(dd, 2-H), 3.16(s, 17-OCH₃), 3.20(d, 2"-Heq), 3.22(m, 4'-H), 3.23(m, 5'-H), 3.25(s, 17-OCH₃), 3.42(dd, 2'-H), 3.59(s, 4-OCH₃), 3.62(br d, 4-H), 3.86(br d, 5-H), 4.44(d, 1'-H), 4.50(dq, 5"-H), 4.57(d, 4"-H), 4.83(d, 1"-H), 4.97(ddq, 14-H), 5.27(br dd, 3-H), 7.29(m, C₆H₅), 7.42(m, C₆H₅).

### Example 50

### Preparation method of (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino-β-D-glucopyranosyl]-6-fornnylmethyl-9-hydroxy-4-methoxy-8-methyl-11-(3-phenyl-2-propynyl)-3-propionyloxy-11-aza-pentadecan-14-olide (in the formula (IX), the compound wherein R₁ is propionyl group, R₂ is hydroxyl group, R₃ is 3-phenyl-2-propynyl group, R₈ is acetyl group, and R₉ is propionyl group)

In the same manner as the method of Example 5, the title compound 14 mg was obtained from the compound of Example 49 (20 mg).
Physicochemical properties of the present compound:
(1) Molecular formula: C₅₀H₇₆N₂O₁₆
(2) Mass spectrum (FAB): m/z 961 (M+H)⁺
(3) Specific rotation: [α]_{D}²³ -71°(c0.25, CHCl₃)
(4) ¹H NMR spectrum (300MHz, CDCl₃) δ (ppm): 0.98(d, 18-H), 1.08(d, 6"-H), 1.13(d, 6'-H), 1.15(t, 3-OCOCH₂CH₃), 1.18(t, 4"-OCOCH₂CH₃), 1.27(d, 15-H), 1.40(s, 3"-CH₃), 1.51(m, 8-H), 1.68(dd, 2"-Hax), 1.99(s, 3"-OCOCH₃), 2.54(s, 3'-N(CH₃)₂), 2.79(dd, 2-H), 2.94(dd, 16-H), 3.21(d, 2"-Heq), 3.21(m, 4'-H), 3.21(m, 5'-H), 3.35(dd, 2'-H), 3.58( s , 4-OCH₃), 3.61(br d, 4-H), 3.87(br d, 5-H), 4.41(d, 1'-H), 4.49(dq, 5"-H), 4.57(d, 4"-H), 4.84(d, 1"-H), 4.90(ddq, 14-H), 5.41(br dd, 3-H), 7.29(m, C₆H₅), 7.42(m, C₆H₅), 9.64(s, 17-H).

### Example 51

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-3-propionyloxy-11-(3-(pyridin-4-yl)propyl)-11-aza-pentadecan-14-olide (in the formula (VIII), the compound wherein R₁ is propionyl group, R₂ is hydroxyl group, R₃ is 3-(pyridin-4-yl)propyl group, R₈ is acetyl group, R₉ is propionyl group, R₁₂ is hydrogen atom, and R₁₅ is methyl group)

In the same manner as the method of Example 47, the title compound 30 mg was obtained from the compound of Example 31 (30 mg) by using 3-(pyridin-4-yl)propanal instead of trans-cinnamaldehyde.
Physicochemical properties of the present compound
(1) Molecular formula: C₅₁H₈₅N₃O₁₇
(2) Mass spectrum (FAB): m/z 1012 (M+H)⁺
(3) Specific rotation: [α]_{D}²² -70°(c0.50, CHCl₃)
(4) ¹H NMR spectrum (300MHz, CDCl₃) δ (ppm): 0.94(d, 18-H), 1.07(d, 6"-H), 1.11(t, 3-OCOCH₂CH₃), 1.17(t, 4"-OCOCH₂CH₃), 1.19(d, 6'-H), 1.24(d, 15-H), 1.40(s, 3"-CH₃), 1.58(m, 8-H), 1.58(m, 16-H), 1.68(dd, 2"-Hax), 2.00(s, 3"-OCOCH₃), 2.53(s, 3'-N(CH₃)₂), 2.81(dd, 2-H), 3.10(s, 17-OCH₃), 3.21(d, 2"-Heq), 3.23(m, 4'-H), 3.23(m, 5'-H), 3.24(s, 17-OCH₃), 3.38(dd, 2'-H), 3.56(br d, 4-H), 3.58(s, 4-OCH₃), 3.88(br d, 5-H), 4.47(d, 1'-H), 4.49(dq, 5"-H), 4.57(d, 4"-H), 4.81(ddq, 14-H), 4.84(d, 1"-H), 5.19(br dd, 3-H), 7.10(dd, pyridine), 8.48(dd, pyridine).

### Example 52

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-formylmethyl-9-hydroxy-4-methoxy-8-methyl-3-propionyloxy-11-(3-(pyridin-4-yl)propyl)-11-aza-pentadecan-14-olide (in the formula (IX), the compound wherein R₁ is propionyl group, R₂ is hydroxyl group, R₃ is 3-(pyridin-4-yl)propyl group, R₈ is acetyl group, and R₉ is propionyl group)

In the same manner as the method of Example 5, the title compound 24 mg was obtained from the compound of Example 51 (30 mg).
Physicochemical properties of the present compound:
(1) Molecular formula: C₄₉H₇₉N₃O₁₆
(2) Mass spectrum (FAB): m/z 966 (M+H)⁺
(3) Specific rotation: [ α ]_{D}²⁰ -84°(c0.50, CHCl₃)
(4) ¹H NMR spectrum (300MHz, CDCl₃) δ(ppm): 0.95(d, 18-H), 1.07(d, 6"-H), 1.13(d, 6'-H), 1.14(t, 3-OCOCH₂CH₃), 1.17(t, 4"-OCOCH₂CH₃), 1.24(d, 15-H), 1.40(s, 3"-CH₃), 1.43(m, 8-H), 1.68(dd, 2"-Hax), 1.98(s, 3"-OCOCH₃), 2.54(s, 3'-N(CH₃)₂), 2.79(dd, 2-H), 2.95(dd, 16-H), 3.21(d, 2"-Heq), 3.21(m, 4'-H), 3.21(m, 5'-H), 3.31(dd, 2'-H), 3.58( s , 4-OCH₃), 3.62(dd, 4-H), 3.87(br d, 5-H), 4.42(d, 1'-H), 4.48(dq, 5"-H), 4.57(d, 4"-H), 4.77(ddq, 14-H), 4.84(d, 1"-H), 5.31(br dd, 3-H), 7.10(dd, pyridine), 8.48(dd, pyridine), 9.63(s, 17-H).

### Example 53

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-11-(3-(2-benzyloxyphenyl)propyl)-6-(2,2-dimethoxyethyl)-9-hydroxy-4-methoxy-8-methyl-3-propionyloxy-11-aza-pentadecan-14-olide (in the formula (VIII), the compound wherein R₁ is propionyl group, R₂ is hydroxyl group, R₃ is 3-(2-benzyloxyphenyl)propyl group, R₈ is acetyl group, R₉ is propionyl group, R₁₂ is hydrogen atom, and R₁₅ is methyl group)

In the same manner as the method of Example 47, the title compound 30 mg was obtained from the compound of Example 31 (30 mg) by using 3-(2-benzyloxyphenyl)propanal instead of trans-cinnamaldehyde.
Physicochemical properties of the present compound:
(1) Molecular formula: C₅₉H₉₂N₂O₁₈
(2) Mass spectrum (FAB): m/z 1117 (M+H)⁺
(3) Specific rotation: [α]_{D}²² -57°(c0.50, CHCl₃)
(4) ¹H NMR spectrum (300MHz, CDCl₃) δ (ppm): 0.93(d, 18-H), 1.08(d, 6"-H), 1.11(t, 3-OCOCH₂CH₃), 1.18(t, 4"-OCOCH₂CH₃), 1.20(d, 6'-H), 1.20(d, 15-H), 1.41(s, 3"-CH₃), 1.64(m, 16-H), 1.68(dd, 2"-Hax), 1.86(m, C₆H₄(CH₂)₃), 1.90(m, 16-H), 2.00(s, 3"-OCOCH₃), 2.53(s, 3'-N(CH₃)₂), 2.81(dd, 2-H), 3.11(s, 17-OCH₃), 3.20(m, 4'-H), 3.20(m, 5'-H), 3.21(d, 2"-Heq), 3.25(s, 17-OCH₃), 3.40(dd, 2'-H), 3.56(br d, 4-H), 3.58(s, 4-OCH₃), 3.88(br d, 5-H), 4.47(d, 1'-H), 4.51(dq, 5"-H), 4.58(d, 4"-H), 4.75(ddq, 14-H), 4.84(d, 1"-H), 5.07(s, C₆H₅CH₂), 5.19(br dd, 3-H), 6.90(m, C₆H₄), 7.15(m, C₆H₄), 7.36(m, C₆H₅).

### Example 54

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-11-(3-(2-benzyloxyphenyl)propyl)-6-formylmethyl-9-hydroxy-4-methoxy-8-methyl-3-propionyloxy-11-azapentadecan-14-olide (in the formula (IX), the compound wherein R₁ is propionyl group, R₂ is hydroxyl group, R₃ is 3-(2-benzyloxyphenyl)propyl group, R₈ is acetyl group, and R₉ is propionyl group)

In the same manner as the method of Example 5, the title compound 22 mg was obtained from the compound of Example 53 (30 mg).
Physicochemical properties of the present compound:
(1) Molecular formula: C₅₇H₈₆N₂O₁₇
(2) Mass spectrum (FAB): m/z 1071 (M+H)⁺
(3) Specific rotation: [ α ]_{D}¹⁹ -73°(c0.50, CHCl₃)
(4) ¹H NMR spectrum (300MHz, CDCl₃) δ (ppm): 0.95(d, 18-H), 1.08(d, 6"-H), 1.13(d, 6'-H), 1.15(t, 3-OCOCH₂CH₃), 1.18(t, 4"-OCOCH₂CH₃), 1.20(d, 15-H), 1.41(s, 3"-CH₃), 1.46(m, 8-H), 1.69(dd, 2"-Hax), 1.77(m, C₆H₄(CH₂)₃), 1.99(s, 3"-OCOCH₃), 2.54(s, 3'-N(CH₃)₂), 2.66(m, C₆H₄(CH₂)₃), 2.79(dd, 2-H), 2.95(dd, 16-H), 3.21(m, 4'-H), 3.21(m, 5'-H), 3.21(d, 2"-Heq), 3.33(dd, 2'-H), 3.57( s , 4-OCH₃), 3.63(dd, 4-H), 3.87(br d, 5-H), 4.42(d, 1'-H), 4.48(dq, 5"-H), 4.58(d, 4"-H), 4.71(ddq, 14-H), 4.84(d, 1"-H), 5.07(s, C₆H₅CH₂), 5.31(br dd, 3-H), 6.88(m, C₆H₄), 7.15(m, C₆H₄), 7.36(m, C₆H₅), 9.63(s, 17-H).

### Example 55

### Preparation method of (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino- β-D-glucopyranosyl]-6-(2,2-dimethoxyethyl)-9-hydroxy-4-methoxy-11-(3-(6-methoxyquinolin-4-yl)propyl)-8-methyl-3-propionyloxy-11-aza-pentadecan-14-olide (in the formula (VIII), the compound wherein R₁ is propionyl group, R₂ is hydroxyl group, R₃ is 3-(6-methoxyquinolin-4-yl)propyl group, R₈ is acetyl group, R₉ is propionyl group, R₁₂ is hydrogen atom, and R₁₅ is methyl group)

In the same manner as the method of Example 47, the title compound 33 mg was obtained from the compound of Example 31 (30 mg) by using 3-(6-methoxyquinolin-4-yl)propanal instead of trans-cinnamaldehyde.
Physicochemical properties of the present compound:
(1) Molecular formula: C₅₆H₈₉N₃O₁₈
(2) Mass spectrum (FAB): m/z 1092 (M+H)⁺
(3) Specific rotation: [ α ]_{D}²¹ -60°(c0.50, CHCl₃)
(4) ¹H NMR spectrum (300MHz, CDCl₃) δ(ppm): 0.94(d, 18-H), 1.07(d, 6"-H), 1.11(t, 3-OCOCH₂CH₃), 1.18(t, 4"-OCOCH₂CH₃), 1.19(d, 6'-H), 1.23(d, 15-H), 1.40(s, 3"-CH₃), 1.42(br dd, 7-H), 1.59(m, 8-H), 1.68(dd, 2"-Hax), 1.94(m, quinoline-(CH₂)₃), 2.00(s, 3"-OCOCH₃), 2.53(s, 3'-N(CH₃)₂), 2.81(dd, 2-H), 3.04(m, quinoline-(CH₂)₃), 3.10(s, 17-OCH₃), 3.21(m, 4'-H), 3.21(m, 5'-H), 3.21(d, 2"-Heq), 3.24(s, 17-OCH₃), 3.38(dd, 2'-H), 3.57(br d, 4-H), 3.59(s, 4-OCHs), 3.89(br d, 5-H), 3.94(s, quinoline-OCH₃), 4.48(d, 1'-H), 4.50(dq, 5"-H), 4.57(d, 4"-H), 4.80(ddq, 14-H), 4.84(d, 1"-H), 5.19(br dd, 3-H), 7.18(d, quinoline), 7.21(d, quinoline), 7.35(dd, quinoline), 8.00(d, quinoline), 8.65(d, quinoline).

### Example 56

### Preparation method of (-)-(3R,4S,5S,6R,8R,9R,14R)-5-[4-O-(3-O-acetyl-2,6-dideoxy-3-C-methyl-4-O-propionyl- α-L-ribo-hexopyranosyl)-3,6-dideoxy-3-dimethylamino- β-D-glucopyranosyl]-6-formylmethyl-9-hydroxy-4-methoxy-11-(3-(6-methoxyquinolin-4-yl)propyl)-8-methyl-3-propionyloxy-11-azapentadecan-14-olide (in the formula (IX), the compound wherein R₁ is propionyl group, R₂ is hydroxyl group, R₃ is 3-(6-methoxyquinolin-4-yl)propyl group, R₈ is acetyl group, and R₉ is propionyl group)

In the same manner as the method of Example 5, the title compound 24 mg was obtained from the compound of Example 55 (33 mg).
Physicochemical properties of the present compound:
(1) Molecular formula: C₅₄H₈₃N₃O₁₇
(2) Mass spectrum (FAB): m/z 1046 (M+H)⁺
(3) Specific rotation: [ α ]_{D}¹⁸ -76°(c0.50, CHCl₃)
(4) ¹H NMR spectrum (300MHz, CDCl₃) δ(ppm): 0.95(d, 18-H), 1.08(d, 6"-H), 1.13(d, 6'-H), 1.15(t, 3-OCOCH₂CH₃), 1.18(t, 4"-OCOCH₂CH₃), 1.24(d, 15-H), 1.40(s, 3"-CH₃), 1.44(m, 8-H), 1.69(dd, 2"-Hax), 1.95(m, quinoline-(CH₂)₃), 1.99(s, 3"-OCOCH₃), 2.54(s, 3'-N(CH₃)₂), 2.80(dd, 2-H), 2.96(dd, 16-H), 3.03(m, quinoline-(CH₂)₃), 3.21(m, 4'-H), 3.21(m, 5'-H), 3.21(d, 2"-Heq), 3.31(dd, 2'-H), 3.51(br d, 9-H), 3.58( s , 4-OCH₃), 3.63(dd, 4-H), 3.87(br d, 5-H), 3.94(s, quinoline-OCH₃), 4.43(d, 1'-H), 4.48(dq, 5"-H), 4.57(d, 4"-H), 4.77(ddq, 14-H), 4.85(d, 1"-H), 5.31(br dd, 3-H), 7.18(d, quinoline), 7.21(d, quinoline), 7.35(dd, quinoline), 8.00(d, quinoline), 8.65(d, quinoline), 9.63(s, 17-H).

### Example 57

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-9-hydroxy-4-methoxy-8-methyl-11-(3-phenylpropionyl)-3-propionyloxy-11-aza-pentadecan-14-olide (in the formula (VIII), the compound wherein R₁ is propionyl group, R₂ is hydroxyl group, R₃ is 3-phenylpropionyl group, R₈ is acetyl group, R₉ is propionyl group, R₁₂ is hydrogen atom, and R₁₅ is methyl group)

The compound of Example 31 (30 mg) was added with dichloromethane 0.6 ml and dissolved, and the solution was added with triethylamine 17 µl and 3-phenylpropionyl chloride 6 µl under ice cooling, and stirred for 1 hour. The reaction mixture was added with chloroform 5 ml and water 5 ml, and the organic layer was separated. Then the aqueous layer was extracted twice with chloroform 10 ml. The organic layers were combined, washed successively with saturated aqueous sodium hydrogencarbonate 10 ml and saturated brine 10 ml, dried over anhydrous sodium sulfate, and then filtered. The filtrate was concentrated under reduced pressure, and the resulting residue was purified by preparative TLC (ethyl acetate/methanol/aqueous ammonia (30:1:0.1)), and then further purified by preparative TLC (chloroform/methanol/aqueous ammonia (20:1:0.1)) to obtain the title compound 20 mg.
Physicochemical properties of the present compound:
(1) Molecular formula: C₅₂H₈₄N₂O₁₈
(2) Mass spectrum (FAB): m/z 1025 (M+H)⁺
(3) Specific rotation: [ α ]_{D}²¹ -26°(c0.40, CHCl₃)
(4) ¹H NMR spectrum (300MHz, CDCl₃) δ (ppm): 0.99(d, 18-H), 1.08(d, 6"-H), 1.09(t, 3-OCOCH₂CH₃), 1.18(t, 4"-OCOCH₂CH₃), 1.19(d, 6'-H), 1.21(d, 15-H), 1.41(s, 3"-CH₃), 1.50(br dd, 7-H), 1.69(dd, 2"-Hax), 1.74(m, 8-H), 1.99(s, 3"-OCOCH₃), 2.54(s, 3'-N(CH₃)₂), 2.78(dd, 2-H), 3.07(s, 17-OCH₃), 3.21(s, 17-OCH₃), 3.22(d, 2"-Heq), 3.23(m, 4'-H), 3.24(m, 5'-H), 3.34(dd, 2'-H), 3.50(br d, 4-H), 3.61(s, 4-OCH₃), 3.78(br d, 9-H), 3.89(br d, 5-H), 4.51(dq, 5"-H), 4.54(ddq, 14-H), 4.55(d, 1'-H), 4.58(d, 4"-H), 4.85(d, 1"-H), 5.14(br dd, 3-H), 7.20(m, C₆H₅), 7.28(m, C₆H₅).

### Example 58

### Preparation method of (-)-(3R,4S,5S,6R,8R,9R,14R)-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-formylmethyl-9-hydroxy-4-methoxy-8-methyl-11-(3-phenylpropionyl)-3-propionyloxy-11-aza-pentadecan-14-olide (in the formula (IX), the compound wherein R₁ is propionyl group, R₂ is hydroxyl group, R₃ is 3-phenylpropionyl group, R₈ is acetyl group, and R₉ is propionyl group)

In the same manner as the method of Example 5, the title compound 15 mg was obtained from the compound of Example 57 (20 mg).
Physicochemical properties of the present compound:
(1) Molecular formula: C₅₀H₇₈N₂O₁₇
(2) Mass spectrum (FAB): m/z 979 (M+H)⁺
(3) Specific rotation: [ α ]_{D}²⁰ -37°(c0.40, CHCl₃)
(4) ¹H NMR spectrum (300MHz, CDCl₃) δ (ppm): 0.99(d, 18-H), 1.08(d, 6"-H), 1.11(d, 6'-H), 1.14(t, 3-OCOCH₂CH₃), 1.18(t, 4"-OCOCH₂CH₃), 1.22(d, 15-H), 1.40(s, 3"-CH₃), 1.53(m, 8-H), 1.69(dd, 2"-Hax), 1.98(s, 3"-OCOCH₃), 2.55(s, 3'-N(CH₃)₂), 2.76(dd, 2-H), 2.89(dd, 16-H), 3.21(d, 2"-Heq), 3.22(m, 4'-H), 3.22(m, 5'-H), 3.28(dd, 2'-H), 3.46(dd, 4-H), 3.61( s , 4-OCH₃), 3.73(br d, 9-H), 3.89(br d, 5-H), 4.44(d, 1'-H), 4.47(dq, 5"-H), 4.50(ddq, 14-H), 4.58(d, 4"-H), 4.85(d, 1"-H), 5.22(br dd, 3-H), 7.20(m, C₆H₅), 7.28(m, C₆H₅), 9.59(s, 17-H).

### Example 59

### Preparation method of (-)-(3R,4S,5S,6R,8R,9R,14R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino- β-D-glucopyranosyl]-6-(2,2-dimethoxyethyl)-4-methoxy-8-methyl-11-(4-phenylbutyl)-3-propionyloxy-11-aza-pentadecan-14-olide (in the formula (VIII), the compound wherein R₁ is propionyl group, R₂ is acetoxy group, R₃ is 4-phenylbutyl group, R₈ is acetyl group, R₉ is propionyl group, R₁₂ is hydrogen atom, and R₁₅ is methyl group)

In the same manner as the method of Example 4, title compound 41 mg was obtained from the compound of Example 17 (305 mg).
Physicochemical properties of the present compound:
(1) Molecular formula: C₅₅H₉₀N₂O₁₈
(2) Mass spectrum (FAB): m/z 1067 (M+H)⁺
(3) Specific rotation: [ α ]_{D}²⁷ -65°(c0.61, CHCl₃)
(4) ¹H NMR spectrum (300MHz, CDCl₃) δ (ppm): 0.90(d, 18-H), 1.07(d, 6"-H), 1.12(t, 3-OCOCH₂CH₃), 1.17(t, 4"-OCOCH₂CH₃), 1.19(d, 6'-H), 1.23(d, 15-H), 1.40(s, 3"-CH₃), 1.68(dd, 2"-Hax), 1.97(s, 9-OCOCH₃), 2.00(s, 3"-OCOCH₃), 2.53(s, 3'-N(CH₃)₂), 2.59(t, Ph(CH₂)₄), 2.83(dd, 2-H), 3.15(s, 17-OCH₃), 3.21(d, 2"-Heq), 3.25(s, 17-OCH₃), 3.41(dd, 2'-H), 3.51(br d, 4-H), 3.59(s, 4-OCH₃), 3.88(br d, 5-H), 4.47(d, 1'-H), 4.57(d, 4"-H), 4.77(m, 9-H), 4.84(d, 1"-H), 4.91(m, 14-H), 5.20(m, 3-H), 7.15(m, C₆H₅), 7.25(m, C₆H₅).

### Example 60

### Preparation method of (-)-(3R,4S,5S,6R,8R,9R,14R)-9-acetoxy-5-[4-O-(3-O-acetyl-2,6-dideoxy-3-C-methyl-4-O-propionyl- α -L-ribo-hexopyranosyl)-3,6-dideoxy-3-dimethylamino- β-D-glucopyranosyl]-6-formylmethyl-4-methoxy-8-methyl-11-(4-phenylbutyl)-3-propionyloxy-11-aza-pentadecan-14-olide (in the formula (IX), the compound wherein R₁ is propionyl group, R₂ is acetoxy group, R₃ is 4-phenylbutyl group, R₈ is acetyl group, and R₉ is propionyl group)

In the same manner as the method of Example 5, the title compound 29 mg was obtained from the compound of Example 59 (41 mg).
Physicochemical properties of the present compound:
(1) Molecular formula: C₅₃H₈₄N₂O₁₇
(2) Mass spectrum (FAB): m/z 1021 (M+H)⁺
(3) Specific rotation: [ α ]_{D}²⁶ -60°(c1.55, CHCl₃)
(4) ¹H NMR spectrum (300MHz, CDCl₃) **δ** (ppm): 0.91(d, 18-H), 1.08(d, 6"-H), 1.13(d, 6'-H), 1.16(t, 3-OCOCH₂CH₃), 1.18(t, 4"-OCOCH₂CH₃), 1.25(d, 15-H), 1.40(s, 3"-CH₃), 1.69(dd, 2"-Hax), 1.93(s, 9-OCOCH₃), 2.00(s, 3"-OCOCH₃), 2.16(m, 6-H), 2.54(s, 3'-N(CH₃)₂), 2.81(dd, 2-H), 2.93(dd, 16-H), 3.21(m, 4'-H), 3.21(m, 5'-H), 3.21(d, 2"-Heq), 3.34(dd, 2'-H), 3.58( s , 4-OCH₃), 3.91(br d, 5-H), 4.44(d, 1'-H), 4.49(dq, 5"-H), 4.57(d, 4"-H), 4.75(br dd, 9-H), 4.82(m, 14-H), 4.84(d, 1"-H), 5.34(m, 3-H), 7.16(m, C₆H₅), 7.25(m, C₆H₅), 9.62(s, 17-H).

### Example 61

### Preparation method of (3R,4S,5S,6R,8R,9R,15R)-9-acetoxy-5-[2-O-acetyl-4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8,11-dimethyl-12-(4-phenylbutyl)-3-propionyloxy-11,12-diaza-hexadecan-15-olide (in the formula (XI), the compound wherein R₁ is propionyl group, R₂ is acetoxy group, R₈ is acetyl group, R₉ is propionyl group, both of R₁₀ and R₁₅ are methyl groups, R₁₁ is 4-phenylbutyl group, and R₁₂ is acetyl group) and (3R,4S,5S,6R,8R,9R,15R)-9-acetoxy-5-(2-O-acetyl-4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8,12-dimethyl-11-(4-phenylbutyl)-3-propionyloxy-11,12-diaza-hexadecan-15-olide (in the formula (XI), the compound wherein R₁ is propionyl group, R₂ is acetoxy group, R₈ is acetyl group, R₉ is propionyl group, R₁₀ is 4-phenylbutyl group, both of R₁₁ and R₁₅ are methyl groups, and R₁₂ is acetyl group)

In the same manner as the method of Example 3, the title compounds 71 mg were obtained as a mixture in a ratio of about 1:1 (measured on the basis of intensities of specific signals) from the compound of Example 2 (788 mg) by using 1-methyl-2-(4-phenylbutyl)hydrazine dihydrochloride instead of methylamine hydrochloride.
Physicochemical properties of the present compound:
(1) Molecular formula: C₅₈H₉₅N₃O₁₉
(2) Mass spectrum (FAB): m/z 1138 (M+H)⁺
(3) ¹H NMR spectrum (300MHz, CDCl₃) δ(ppm): 0.93, 0.95(d, 19-H), 1.07(d, 6"-H), 1.42(s, 3"-CH₃), 1.68(dd, 2"-Hax), 2.02(s, 9-OCOCH₃), 2.02(s, 3"-OCOCH₃), 2.02(s, 2'-OCOCH₃), 2.22, 2.27(s, 11-NCH₃, 12-NCH₃), 2.44(s, 3'-N(CH₃)₂), 3.15(t, 4'-H), 3.17, 3.21(s, 18-OCH₃), 3.19(d, 2"-Heq), 3.24, 3.25(s, 18-OCH₃), 3.37(br d, 4-H), 3.58(s, 4-OCH₃), 3.84, 3.87(br d, 5-H), 4.57(d, 4"-H), 4.68, 4.71(d, 1'-H), 4.81(d, 1"-H), 4.86, 5.05(m, 15-H), 4.96, 5.00(dd, 2'-H), 5.25(m, 3-H), 7.19(m, C₆H₅), 7.27(m, C₆H₅).

### Example 62

### Preparation method of (3R,4S,5S,6R,8R,9R,15R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8,11-dimethyl-12-(4-phenylbutyl)-3-propionyloxy-11,12-diaza-hexadecan-15-olide (in the formula (XI), the compound wherein R₁ is propionyl group, R₂ is acetoxy group, R₈ is acetyl group, R₉ is propionyl group, both of R₁₀ and R₁₅ are methyl groups, R₁₁ is 4-phenylbutyl group, and R₁₂ is hydrogen atom) and (3R,4S,5S,6R,8R,9R,15R)-9-acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-(2,2-dimethoxyethyl)-4-methoxy-8,12-dimethyl-11-(4-phenylbutyl)-3-propionyloxy-11,12-diaza-hexadecan-15-olide (in the formula (XI), the compound wherein R₁ is propionyl group, R₂ is acetoxy group, R₈ is acetyl group, R₉ is propionyl group, R₁₀ is 4-phenylbutyl group, both of R₁₁ and R₁₅ are methyl groups, and R₁₂ is hydrogen atom)

In the same manner as the method of Example 7, the title compounds 51 mg were obtained as a mixture in a ratio of about 10:7.7 from the compound of Example 61 (70 mg).
Physicochemical properties of the present compound:
(1) Molecular formula: C₅₆H₉₃N₃O₁₈
(2) Mass spectrum (FAB): m/z 1096 (M+H)⁺
(3) ¹H NMR spectrum (300MHz, CDCl₃) δ (ppm): 0.94, 0.96(d, 19-H), 1.09(d, 6"-H), 1.14, 1.16(t, 3-OCOCH₂CH₃), 1.42 (s, 3"-CH₃), 1.69(dd, 2"-Hax), 2.23, 2.26(s, 11-NCH₃, 12-NCH₃), 2.54, 2.55(s, 3'-N(CH₃)₂), 3.21, 3.25(s, 18-OCH₃), 3.26(s, 18-OCH₃), 3.33, 3.42(br d, 4-H), 3.62(s, 4-OCH₃), 3.86, 3.89(br d, 5-H), 4.43, 4.49(d, 1'-H), 4.59(d, 4"-H), 4.84, 4.85(d, 1"-H), 4.87, 5.01(m, 15-H), 5.01, 5.07(m, 9-H), 5.31(m, 3-H), 7.18(m, C₆H₅), 7.26(m, C₆H₅).

### Example 63

### Preparation method of (3R,4S,5S,6R,8R,9R,15R)-9-Acetoxy-5-[4-O-(3-O-acetyl-2,6-dideoxy-3-C-methyl-4-O-propionyl-α-L-ribo-hexopyranosyl)-3,6-dideoxy-3-dimethylamino-β-D-glucopyranosyl]-6-formylmethyl-4-methoxy-8,11-dimethyl-12-(4-phenylbutyl)-3-propionyloxy-11,12-diaza-hexadecan-15-olide (in the formula (XII), the compound wherein R₁ is propionyl group, R₂ is acetoxy group, R₈ is acetyl group, R₉ is propionyl group, R₁₀ is methyl group, and R₁₁ is 4-phenylbutyl group) and (3R,4S,5S,6R,8R,9R,15R)-9-acetoxy-5-[4-O-(3-O-acetyl-2,6-dideoxy-3-C-methyl-4-O-propionyl- α -L-ribo-hexopyranosyl)-3,6-dideoxy-3-dimethylamino-β-D-glucopyranosyl]-6-formylmethyl-4-methoxy-8,12-dimethyl-11-(4-phenylbutyl)-3-propionyloxy-11,12-diaza-hexadecan-15-olide (in the formula (XII), the compound wherein R₁ is propionyl group, R₂ is acetoxy group, R₈ is acetyl group, R₉ is propionyl group, R₁₀ is 4-phenylbutyl group, and R₁₁ is methyl group)

In the same manner as the method of Example 5, the title compounds 48 mg were obtained as a mixture in a ratio of about 10:8.7 from the compound of Example 62 (50 mg).
Physicochemical properties of the present compound:
(1) Molecular formula: C₅₄H₈₇N₃O₁₇
(2) Mass spectrum (FAB): m/z 1050 (M+H)⁺
(3) ¹H NMR spectrum (300MHz, CDCl₃) δ (ppm): 0.95, 0.97(d, 19-H), 1.08, 1.09(d, 6"-H), 1.42 (s, 3"-CH₃), 1.70(dd, 2"-Hax), 2.18, 2.26(s, 11-NCH₃, 12-NCH₃), 2.54, 2.56(s, 3'-N(CH₃)₂), 3.17(t, 4'-H), 3.56, 3.60(s, 4-OCH₃), 3.94(br d, 5-H), 4.40, 4.44(d, 1'-H), 4.51(dq, 5"-H), 4.59(d, 4"-H), 4.84, 4.86(d, 1"-H), 4.82, 4.98(m, 15-H), 4.85, 5.10(m, 9-H), 5.38, 5.44(m, 3-H), 7.18(m, C₆H₅), 7.27(m, C₆H₅), 9.63, 9.65(s, 18-H).

### Example 64

### Preparation method of (-)-(1R)-1-methyl-3-hydroxypropyl (3R,4S,5S,6R,8R,9R)-9-acetoxy-5-[2-O-acetyl-4-O-(3-O-acetyl-2,6-dideoxy-3-C-methyl-4-O-propionyl- α -L-ribo-hexopyranosyl)-3,6-dideoxy-3-dimethylamino-β-D-glucopyranosyl]-6-(2,2-dimethoxyethyl)-10-hydroxy-4-methoxy-8-methyl-3-propionyloxydecanoate (in the formula (III), the compound wherein R₁ is propionyl group, R₂ is acetoxy group, R₈ is acetyl group, R₉ is propionyl group, R₁₂ is acetyl group, R₁₃ is dimethoxymethyl group, and X is hydroxymethyl group)

The compound of Example 2 (300 mg) was dissolved in ethanol 6 ml, and added with sodium borohydride 12 mg, and the mixture was stirred under ice cooling for 1 hour. The reaction mixture was added with ethyl acetate 30 ml and 10% aqueous ammonium chloride 15 ml, and the organic layer was separated. The organic layer was washed successively with saturated aqueous sodium hydrogencarbonate 15 ml and saturated brine 15 ml, dried over anhydrous sodium sulfate, and then filtered. The filtrate was concentrated under reduced pressure, and the resulting residue was purified by silica gel column chromatography (chloroform/methanol (80:1-60:1)) to obtain the title compound 25 mg.
Physicochemical properties of the present compound:
(1) Molecular formula: C₄₇H₈₁NO₂₁
(2) Mass spectrum (FAB): m/z 996 (M+H)⁺
(3) Specific rotation: [ α ]_{D}²⁵ -91°(c1.05, CHCl₃)
(4) ¹H NMR spectrum (300MHz, CDCl₃) δ(ppm): 0.92(d, 8- CH₃), 0.99(m, 7- H), 1.07(d, 6"-H), 1.13(t, 3-OCOCH₂CH₃), 1.20(t, 4"-OCOCH₂CH₃), 1.21(d, 6'-H), 1.28(d, OCH(CH₃)CH₂CH₂OH), 1.42(s, 3"-CH₃), 1.55(m, 17-H), 1.58(m, 8-H), 1.68(dd, 2"-Hax), 2.03(s, 3"-OCOCH₃), 2.05(s, 2'-OCOCH₃), 2.10(s, 9-OCOCH₃), 2.43(s, 3'-N(CH₃)₂), 2.62(t, 3'-H), 2.63(dd, 2-H), 2.73(dd, 2-H), 3.15(t, 4'-H), 3.15(s, CH(OCH₃)₂), 3.20(d, 2"-Heq), 3.26(s, CH(OCH₃)₂), 3.49(dd, 4-H), 3.52(s, 4-OCH₃), 3.68(d, CH(CH₃)CH₂CH₂OH), 3.83(br d, 5-H), 3.99(dd, 10-H), 4.27(dd, 10-OH), 4.49(dq, 5"-H), 4.57(d, 4"-H), 4.65(d, 1'-H), 4.81(d, 1"-H), 4.97(dd, 2'-H), 5.07(ddq, OCH(CH₃) CH₂CH₂OH), 5.33(br dd, 3-H).

Chemical formulas of the compounds obtained in the above examples are shown below.

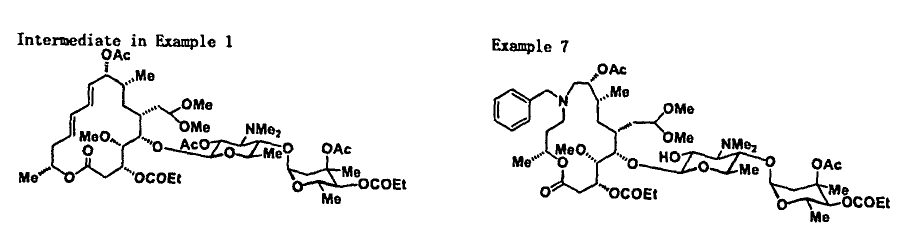

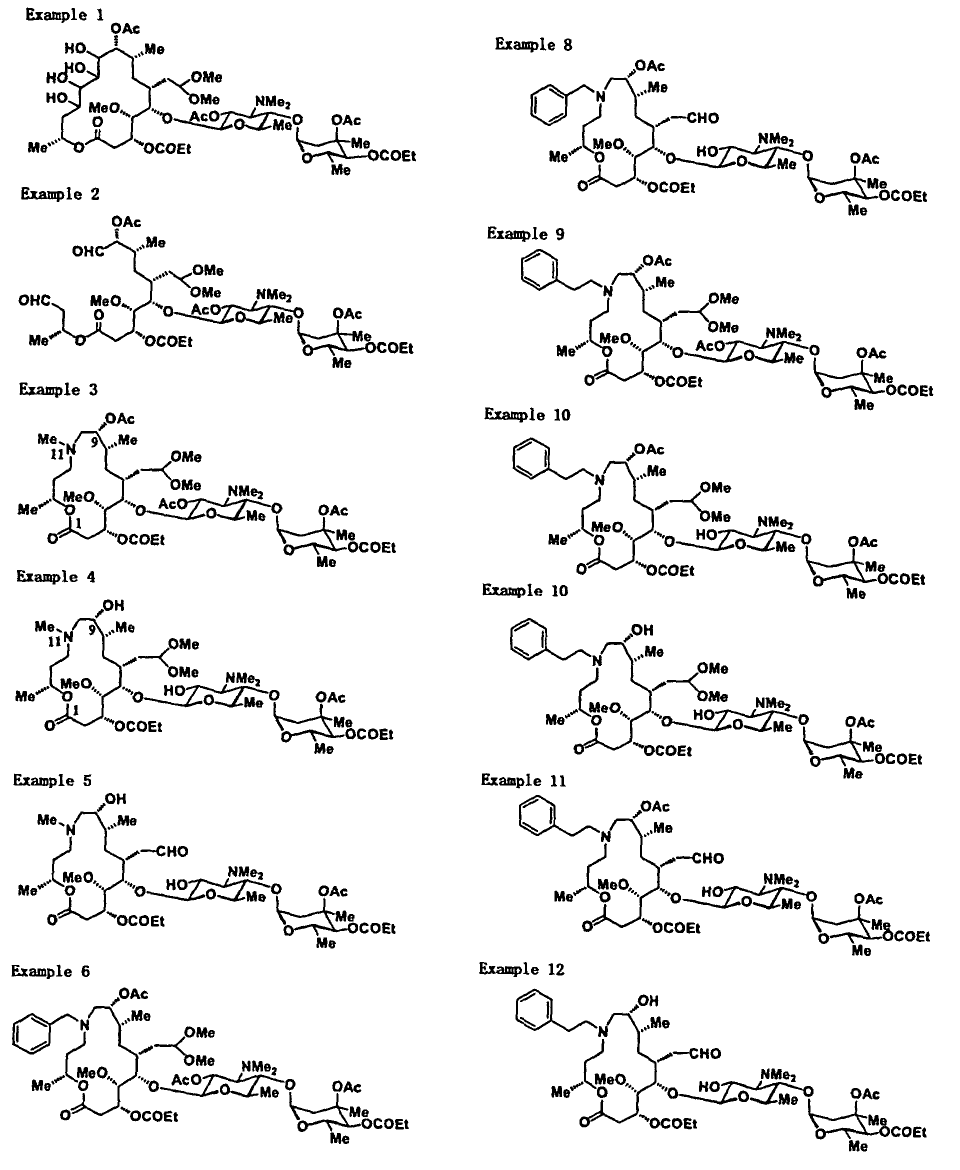

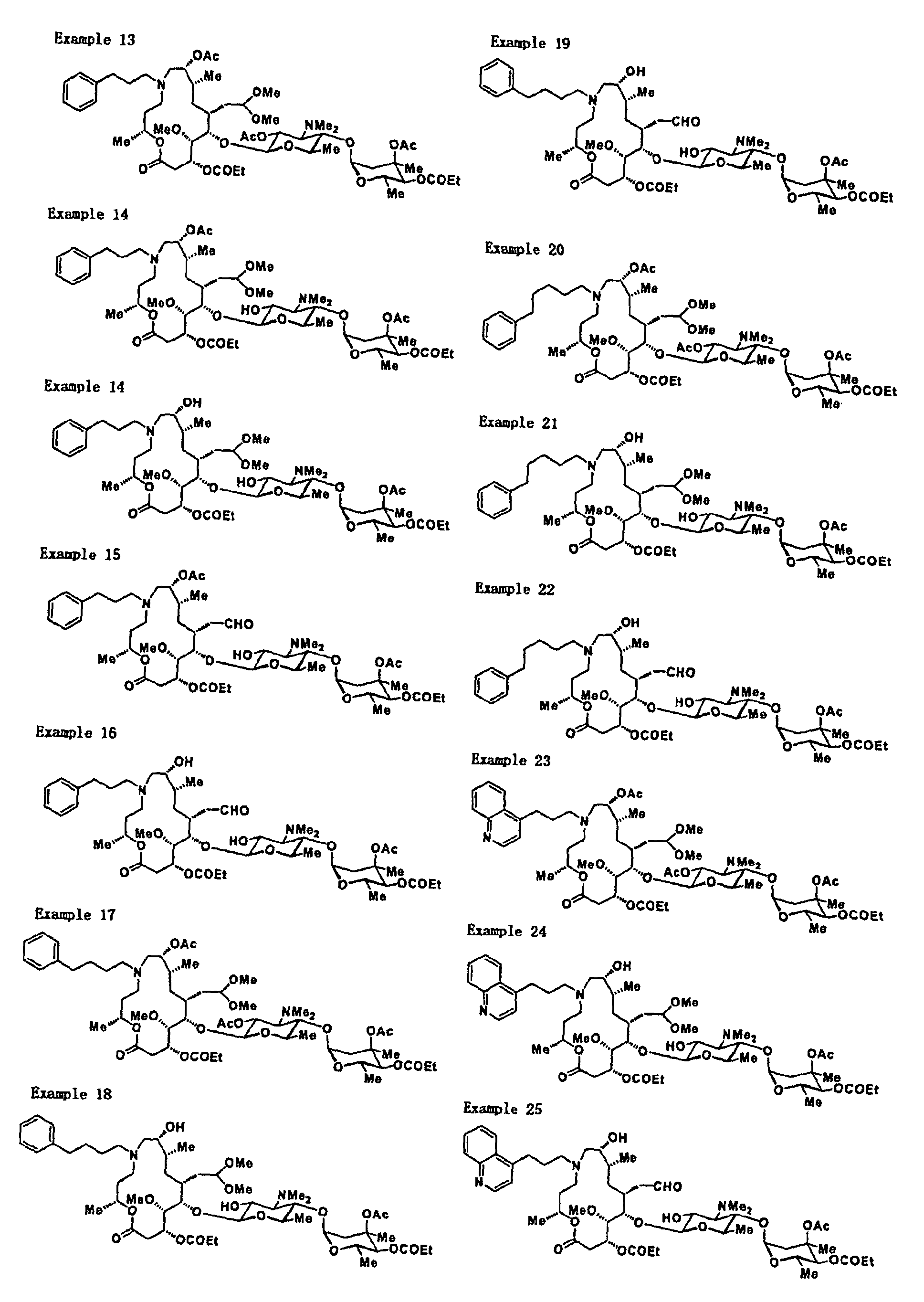

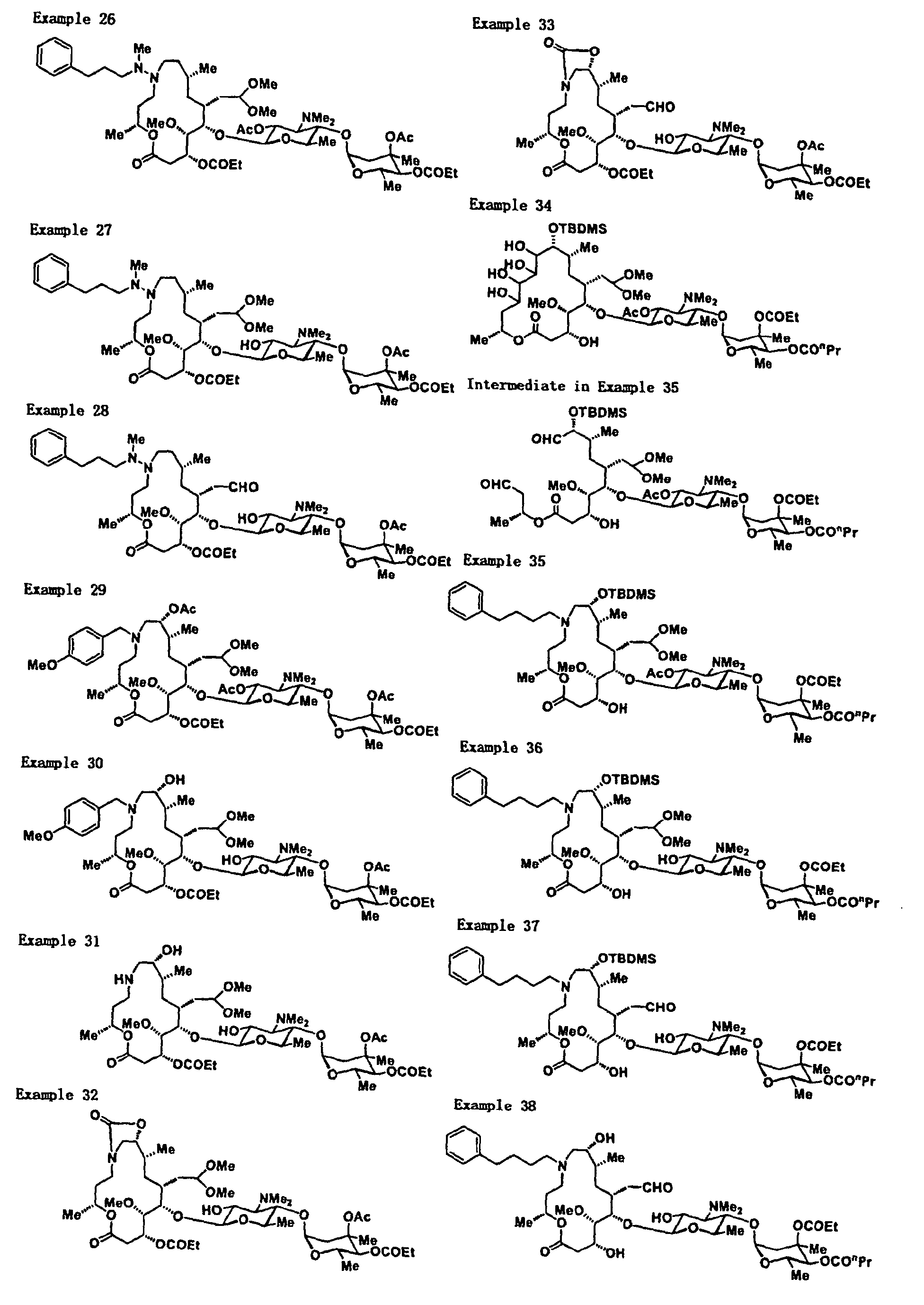

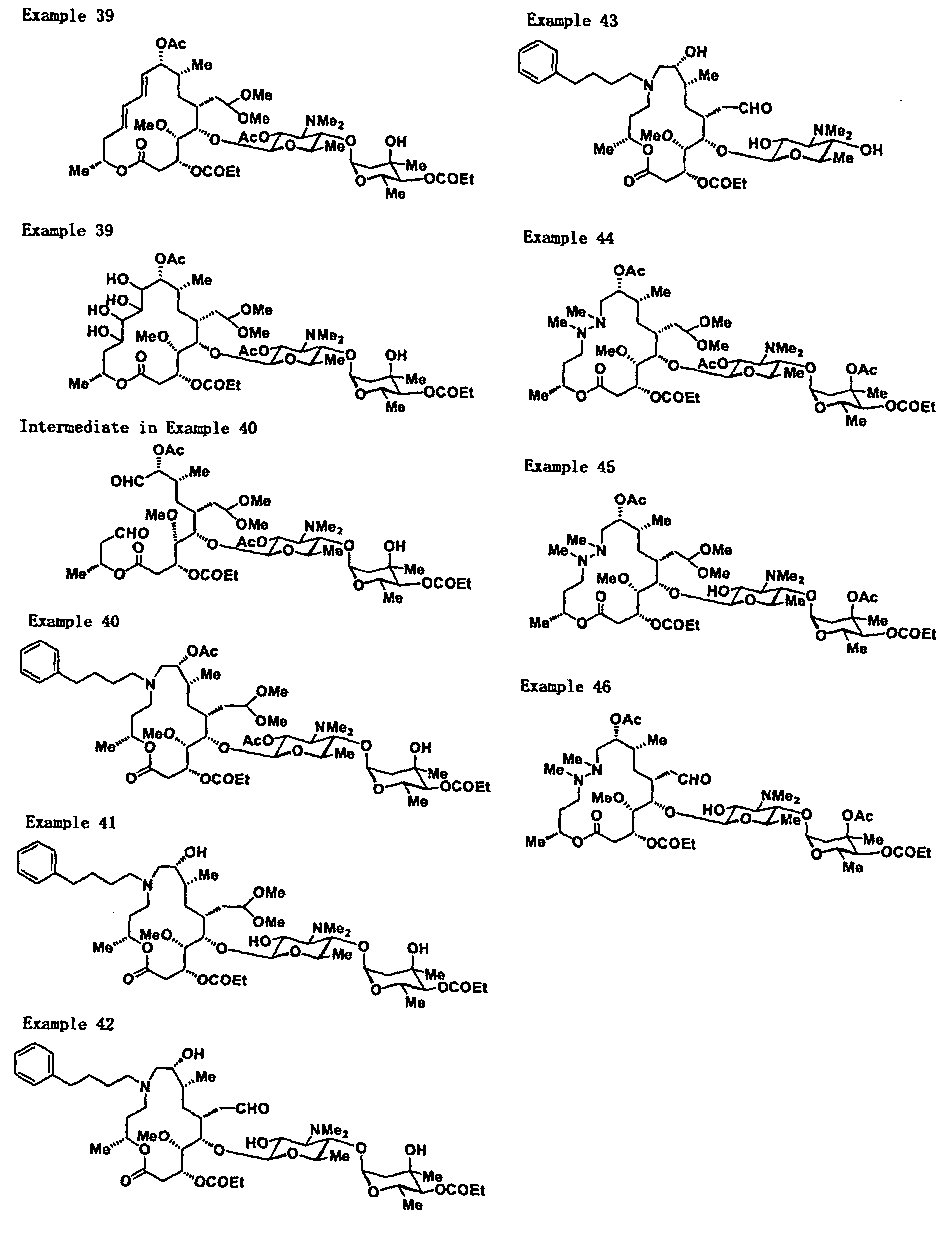

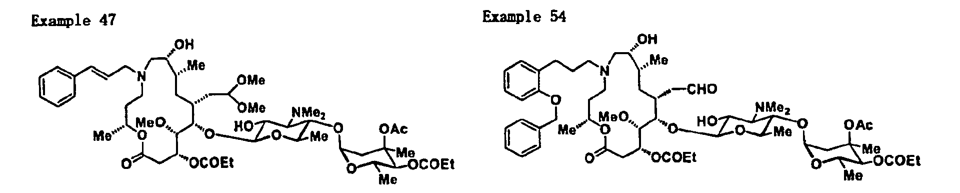

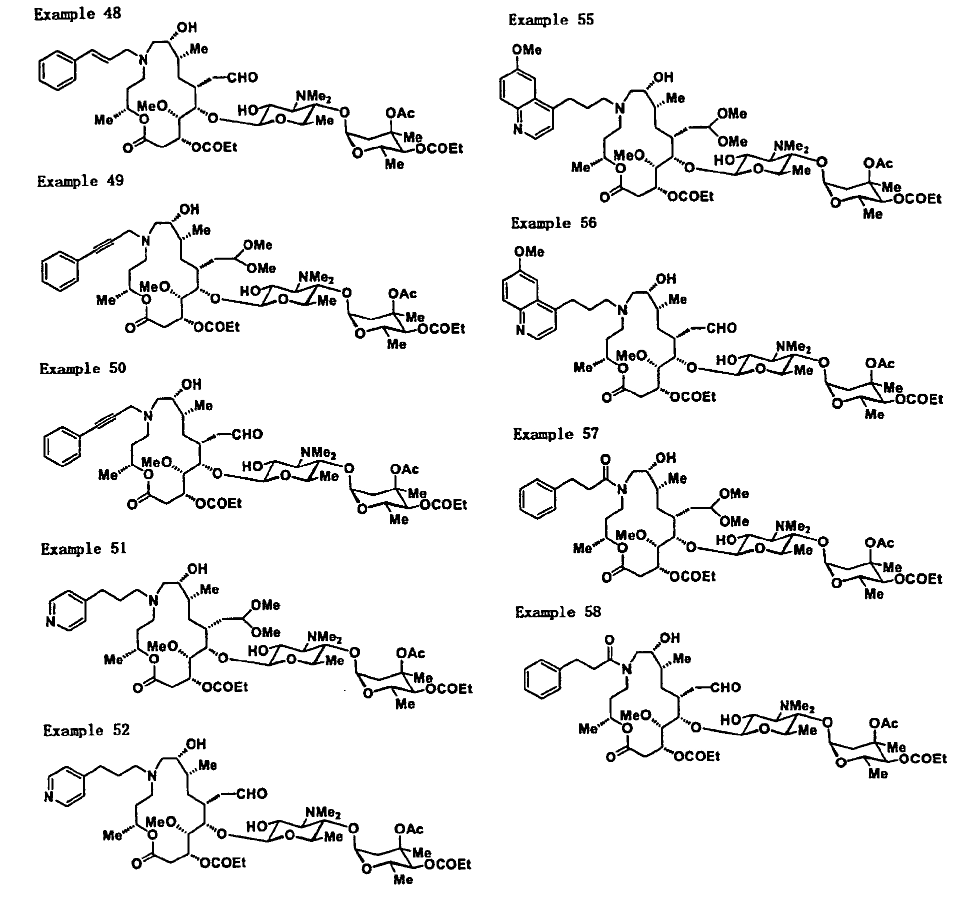

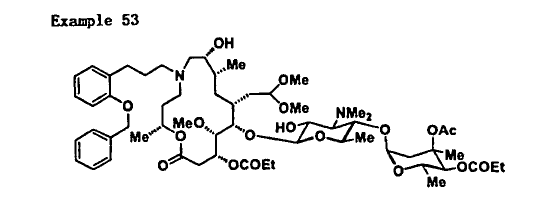

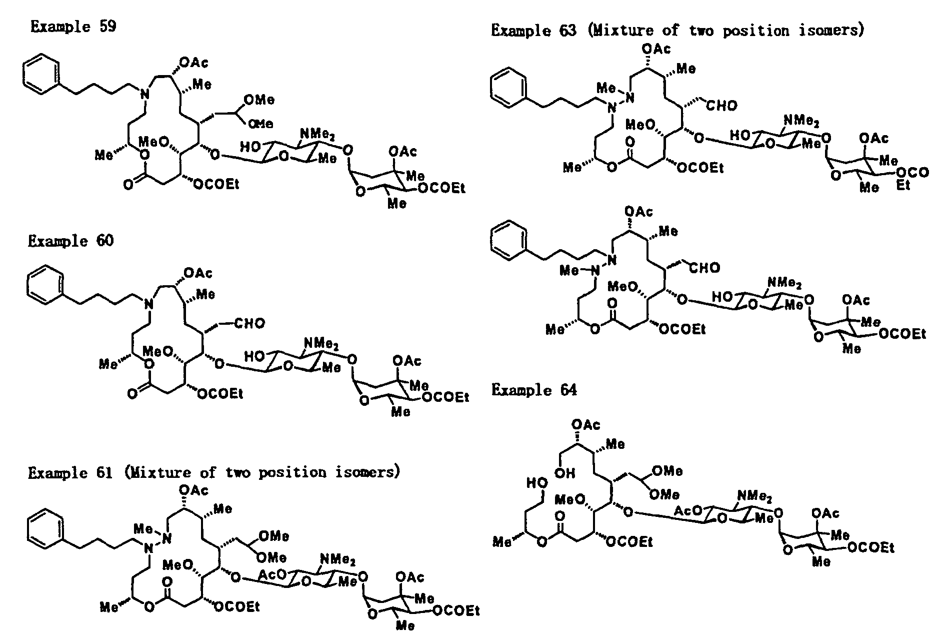

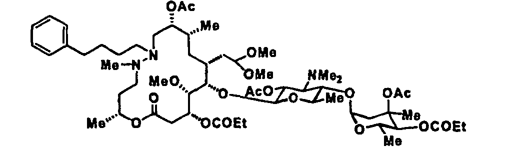

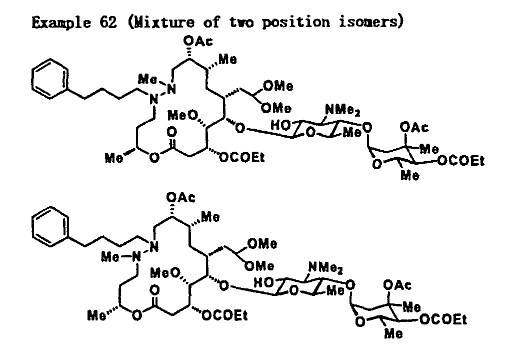

### Test Example 1: Antibacterial activity test

Antibacterial activities in vitro of the compounds of Examples 19 and 25 were measured as follows by referring to the standard method of Japanese Society of Chemotherapy (Chemotherapy, Vol. 29, p. 76-79, 1981). Antibacterial activities of miokamycin (MOM) was measured in the same manner for comparison.

For each of the solutions of the test drugs, which were dissolved in methanol so as to be 6,400 µg/ml, serial two-fold diluents with methanol were prepared. Each 200 µl of the test drug solutions prepared was placed in a laboratory dish, and the agar medium for sensitivity measurement, which was added with 5% equine sterile defibrinated blood, 15 µg/ml β-Nicotinamide-adenine dinucleotide and 2.5 µg/ml hemin, was dispensed in 10 ml aliquots into the dish and then mixed with the solution for dilution to prepare the agar plate containing the test drug. A given amount of the liquid medium for the sensitivity measurement, which was prepared so as to contain a given amount of test bacterium was inoculated on the agar plate containing the test drug by a microplanter (Sakuma Seisakusyo, Co. Ltd.), and after the inoculation, the plate was incubated at 37°C for about 20 hours. After the incubation, the existence of growth of the test bacterium was visually observed, and a minimum concentration at which no growth was observed was defined as a minimum inhibitory concentration of a test drug (MIC) against each of test bacteria. The results are shown in Table 1.

| Minimum inhibitory concentration (MIC, µg/ml) | | | |
|---|---|---|---|
| Test bacterium | Compound of Example 19 | Compound of Example 25 | MOM |
| *Staphylococcus aureus* Smith | 0.5 | 0.5 | 1 |
| *Micrococcus Iuteus* ATCC9341 | 0.03 | 0.03 | 0.13 |
| *Streptococcus pneumoniae* DP1 | 0.06 | 0.06 | 0.25 |
| TypeI | | | |
| *Streptococcus pneumoniae* IP692 | 0.13 | 0.06 | 0.25 |
| *Moraxella catarrhalis* W-0506 | 0.25 | 0.25 | 1 |
| *Haemophilus influenzae* 9334 | 2 | 1 | 4 |
| (Note) Medium used: Blood agar medium using Medium-N for sensitivity disc "Nissui" as the base medium | | | |

The compounds of Examples 19 and 25 according to the present invention gave higher antibacterial activities than MOM. Further, the present inventors also verified that the 16-membered diazalide in which leucomycin 16-membered macrolide was chemically modified gave excellent antibacterial activities, although the results are not particularly demonstrated.

### Test Example 2: Antibacterial activity after treatment with liver homogenate

Antibacterial activities of the compound of Example 19 after treatment with mouse liver homogenate were examined as follows. Antibacterial activities of MOM were examined in the same manner for comparison. The liver was collected from a mouse, and added with a phosphate buffer to prepare 5% homogenate. A 50 µl of the test drug solution, dissolved in methanol so as to be 150 µg/ml, was added to 1.45 ml of the mouse liver homogenate, and the mixture was allowed to react at 37°C. Each portion of the reaction mixture was collected at the beginning of the reaction, and after 10, 20 and 40 minutes, and added with a given volume of an acetonitrile solution to prepare each bioassay sample for the measurement of a concentration of the test drug. A paper disc in 8 mm diameter was impregnated with a given amount of the sample for bioassay, and then the disc was placed on the agar plate prepared by suspending Micrococcus luteus ATCC9341 so as to contain a given amount of the bacterium, and the plate was incubated at 37°C for about 20 hours. After the incubation, a diameter of growth inhibitory circle of Micrococcus luteus ATCC9341 observed around the paper disc was measured with a digital slide calipers. Standard solutions of the test drug at known concentrations, diluted and prepared with the mouse liver homogenate, were also treated in the same manner. On the basis of a calibration curve which was drawn from the concentrations and the diameters of growth inhibitory circles of the standard solutions, residual activities of the samples at the above sampling times were calculated, and compared with antibacterial activities at the beginning of the reaction.

As a result, apparent decreases in antibacterial activities of MOM were observed at 10, 20, and 40 minutes after the start of the reaction compared with the results at the start of the reaction. Whilst no decrease in antibacterial activities of the compound of Example 19 was observed even at 40 minutes after the start of the reaction.

### Industrial Applicability

The novel 15-membered azalide and 16-membered diazalide derivative provided by the present invention have excellent antibacterial activities, and therefore, they are useful as active ingredients of medicaments for therapeutic and/or preventive treatment of various kinds of microorganism infectious diseases.

In addition, according to the present invention, the 15- to 21-membered macrolactone such as the novel 15-membered azalide represented by the general formula (I) and the novel 16-membered diazalide derivative represented by the general formula (II) can be economically prepared in high yields via the compounds represented by the general formula (III).

## Claims

1. A compound represented by the following general formula (I) or a pharmaceutically acceptable salt thereof: 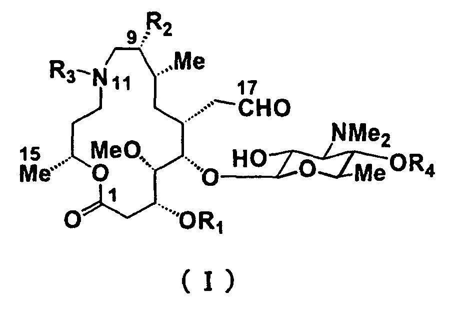 [wherein
R₁ represents hydrogen atom or a C2-C4 linear alkylcarbonyl group,
R₂ represents hydrogen atom, oxygen atom, hydroxyl group, or a C2-C5 alkylcarbonyloxy group,
wherein, when R₂ represents hydrogen atom,
R₃ represents the following group (a): 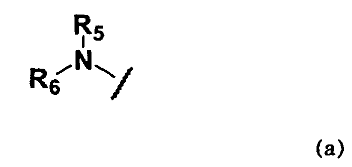 (wherein R₅ and R₆ may be the same or different and each represents hydrogen atom, or a C1-C10 alkyl group which may be substituted with Ar group (Ar represents an aryl group or a heterocyclic group)),
when R₂ represents either hydroxyl group or a C2-C5 alkylcarbonyloxy group,
R₃ represents hydrogen atom, a C1-C10 alkyl group, a C2-C10 alkylcarbonyl group, a C2-C10 alkenyl group, a C3-C10 alkenylcarbonyl group, a C2-C10 alkynyl group, Ar-A- group (wherein Ar has the same meaning as that mentioned above, and A represents a C1-C10 alkyl group, a C2-C10 alkylcarbonyl group, a C2-C10 alkenyl group, a C3-C10 alkenylcarbonyl group, or a C2-C10 alkynyl group), or Ar-(CH₂)ₚ-B-(CH₂)_{q}- group (wherein Ar has the same meaning as that mentioned above, p represents an integer of from 0 to 7, q represents an integer of from 2 to 9, and p+q represents an integer of from 2 to 9, B is a group selected from oxygen atom, sulfur atom or -NR₇-, wherein R₇ represents hydrogen atom, or a C1-C8 alkyl group which may be substituted with Ar group), or
when R₂ represents oxygen atom,
R₃ is W and combines together with R₂ to represent the following group (b): 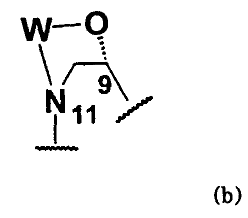 (wherein W represents carbonyl group or thiocarbonyl group), and
R₄ represents hydrogen atom or the following group (c): 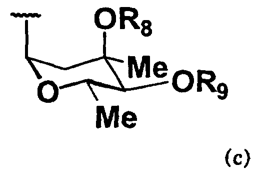 (wherein R₈ and R₉ may be the same or different and each represents hydrogen atom, or a C2-C5 linear or branched alkylcarbonyl group)].

2. The compound according to claim 1 or a pharmaceutically acceptable salt thereof, wherein
R₁ is hydrogen atom or a C2-C4 linear alkylcarbonyl group, R₂ is hydrogen atom, oxygen atom, hydroxyl group, or a C2-C5 alkylcarbonyloxy group,
R₂ is hydrogen atom, and
R₃ is the following group (a-1): 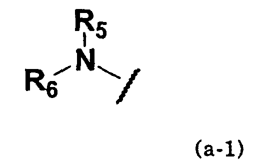 (wherein R₅ is a C1-C10 alkyl group, and R₆ represents a C1-C10 alkyl group which may be substituted with Ar group (Ar has the same meaning as that defined above)), or R₂ is either hydroxyl group or a C2-C5 alkylcarbonyloxy group, and
Rs is hydrogen atom, a C1-C10 alkyl group, a C2-C10 alkylcarbonyl group, or Ar-A- group (wherein Ar has the same meaning as that defined above, and A represents a C1-C10 alkyl group, a C2-C10 alkylcarbonyl group, a C2-C10 alkenyl group, or a C2-C10 alkynyl group), or
R₂ is oxygen atom, and
R₃ combines with R₂ to represent the following group (b-1): 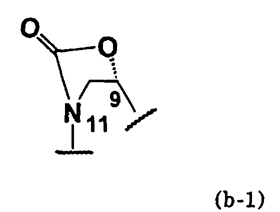 and R₄ is hydrogen atom or the following group (c-1): 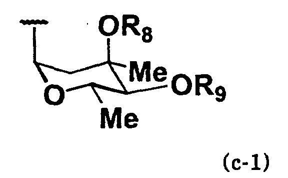 (wherein R₈ is hydrogen atom, or a C2-C5 linear or branched alkylcarbonyl group, and R₉ represents a C2-C5 linear or branched alkylcarbonyl group).

3. The compound according to claim 1 or a pharmaceutically acceptable salt thereof, wherein
R₁ is a C2-C4 linear alkylcarbonyl group,
R₂ is hydroxyl group or a C2-C5 alkylcarbonyloxy group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and
R₄ is the group (c) wherein R₈ and R₉ may be the same or different and each represents a C2-C5 linear or branched alkylcarbonyl group.

4. The compound according to claim 1 or a pharmaceutically acceptable salt thereof, wherein
R₁ is a C2-C4 linear alkylcarbonyl group,
R₂ is hydroxyl group or a C2-C5 alkylcarbonyloxy group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and R₄ is the group (c) wherein R₈ is hydrogen atom, and R₉ is a C2-C5 linear or branched alkylcarbonyl group.

5. The compound according to claim 1 or a pharmaceutically acceptable salt thereof, wherein
R₁ is hydrogen atom,
R₂ is hydroxyl group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and R₄ is the group (c) wherein R₈ and R₉ may be the same or different and each represents a C2-C5 linear or branched alkylcarbonyl group.

6. The compound according to claim 1 or a pharmaceutically acceptable salt thereof, wherein
R₁ is a C2-C4 linear alkylcarbonyl group,
R₂ is hydroxyl group or a C2-C5 alkylcarbonyloxy group,
R₃ is a C1-C10 alkyl group which may be substituted with Ar group, a C2-C10 alkylcarbonyl group which may be substituted with Ar group, a C2-C10 alkenyl group which may be substituted with Ar group, or a C2-C10 alkynyl group which may be substituted with Ar group (wherein Ar has the same meaning as that defined above), and R₄ is hydrogen atom.

7. The compound according to claim 1 or 2 or a pharmaceutically acceptable salt thereof, wherein
R₃ is methyl group, benzyl group, 2-phenylethyl group, 3-phenylpropyl group, 4-phenylbutyl group, 5-phenylpentyl group, 3-phenylpropionyl group, 3-phenyl-2-propenyl group, 3-phenyl-2-propynyl group, 3-(2-benzyloxyphenyl)propyl group, 3-(pyridin-4-yl)propyl group, N-methyl-N-(3-phenylpropyl)amino group, 3-(quinolin-4-yl)propyl group, or 3-(6-methoxyquinolin-4-yl)propyl group.

8. A compound represented by the following general formula (II) or a pharmaceutically acceptable salt thereof: 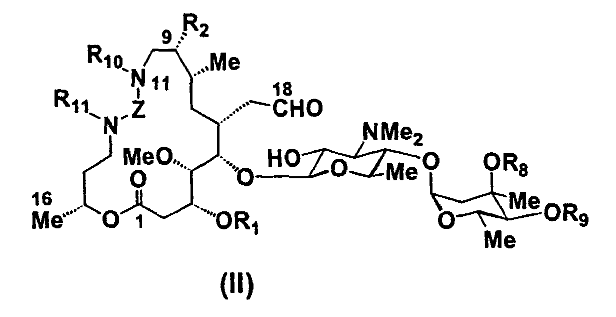 [wherein
R₁ represents hydrogen atom or a C2-C4 linear alkylcarbonyl group,
R₂ represents hydroxyl group or a C2-C5 alkylcarbonyloxy group,
R₈ and R₉ may be the same or different and each represents hydrogen atom, or a C2-C5 linear or branched alkylcarbonyl group,
R₁₀ and R₁₁ may be the same or different and each represents hydrogen atom, a C1-C10 alkyl group, a C2-C 10 alkenyl group, a C2-C 10 alkynyl group, or Ar-A- group (Ar has the same meaning as that mentioned above, and A represents a C1-C10 alkyl group, a C2-C10 alkenyl group or a C2-C10 alkynyl group), or R₁₀ and R₁₁ may optionally form a 6 to 7-membered aliphatic hetero ring together with the nitrogen atom to which they bind and with Z (wherein the aliphatic hetero ring may have a double bond), and Z is a single bond, -(CH₂)ₘ-C₆H₄-(CH₂)ₘ- group (wherein m represents an integer of 0 or 1, and a substituting position on the aromatic ring is not particularly limited) or a - (CH₂)ₙ- group (n represents an integer of from 1 to 3)].

9. The compound according to claim 8 or a pharmaceutically acceptable salt thereof, wherein
R₁₀ and R₁₁ may be the same or different and each represents hydrogen atom, a C1-C10 alkyl group, or Ar-A- group (Ar has the same meaning as that defined above, and A represents a C1-C10 alkyl group, a C2-C10 alkenyl group, or a C2-C10 alkynyl group), and Z is a single bond.

10. The compound according to either of claim 8 or 9 or a pharmaceutically acceptable salt thereof, wherein
both of R₁₀ and R₁₁ are methyl groups.

11. The compound according to either of claim 8 or 9 or a pharmaceutically acceptable salt thereof, wherein
R₁₀ is methyl group and R₁₁ is 4-phenylbutyl group, or R₁₀ is 4-phenylbutyl group and R₁₁ is methyl group.

12. A compound represented by the following general formula (III) or a pharmaceutically acceptable salt thereof: 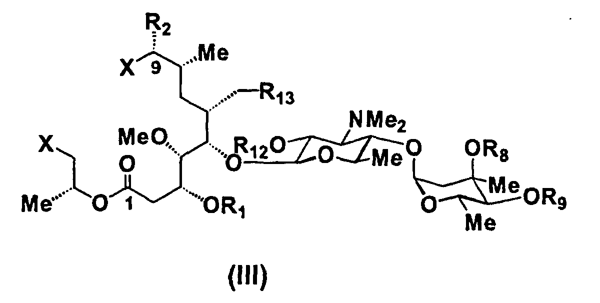 [wherein
R₁ represents hydrogen atom or a C2-C4 linear alkylcarbonyl group,
R₂ represents hydroxyl group, a C2-C5 alkylcarbonyloxy group, or the following group (d): 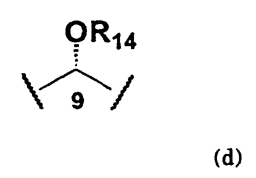 (wherein R₁₄ represents a silyl-type protecting group or an acetal-type protecting group),
R₈ and R₉ may be the same or different and each represents hydrogen atom, or a C2-C5 linear or branched alkylcarbonyl group,
R₁₂ represents hydrogen atom, a C2-C5 linear alkylcarbonyl group, ethoxyethyl group, a silyl-type protecting group, benzyloxycarbonyl group, 4-methoxybenzyloxycarbonyl group, or 4-nitrobenzyloxycarbonyl group,
R₁₃ represents -CH(YR₁₅)₂ group (wherein Y is oxygen atom or sulfur atom, and R₁₅ represents a C1-C5 alkyl group) or the following group (e): 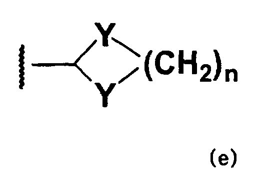 (wherein Y has the same meaning as that mentioned above, and n represents an integer of 2 or 3), and
X represents formyl group, hydroxymethyl group, R₁₆SO₂OCH₂- group (R₁₆ represents methyl group, trifluoromethyl group, or phenyl group, benzyl group, or naphthyl group in which one of the hydrogen atoms on the aromatic ring may be substituted with methyl group, nitro group or azide group), halomethyl group, or R₁₃ (R₁₃ has the same meaning as that mentioned above)].

13. The compound according to claim 12 or a pharmaceutically acceptable salt thereof, wherein X is formyl group.

14. The compound according to claim 12 or a pharmaceutically acceptable salt thereof, wherein X is hydroxymethyl group.

15. A method for preparing a 15 to 21-membered macrolactone, which comprises the step of cyclization of the compound according to any one of claims 12 to 14.

16. The method according to claim 15, which is used for preparing the compound according to any one of claims 1 to 11 or a pharmaceutically acceptable salt thereof.

17. A medicament which comprises the compound according to any one of claims 1 to 11 or a pharmaceutically acceptable salt thereof as an active ingredient.

18. The medicament according to claim 17, which is an antibacterial agent.

19. A method for therapeutic treatment of an infectious disease, which comprises the step of administering an effective amount of the compound according to any one of claims 1 to 11 or a pharmaceutically acceptable salt thereof to an animal including a human.

20. A use of the compound according to any one of claims 1 to 11 or a pharmaceutically acceptable salt thereof for the manufacture of the medicament according to claim 17 or 18.
